# EUROPEAN PATENT APPLICATION

(11) **EP 3 323 826 A1**
(43) Date of publication of application: **23.05.2018**
(21) Application number: 16199726.7
(22) Date of filing: 21.11.2016
(51) Int. Cl.: C07K 14/47, C12N 15/85

(54) **NATIVE CHINESE HAMSTER OVARY CELL SECRETION SIGNAL PEPTIDES FOR PRODUCTION OF RECOMBINANT POLYPEPTIDES**

(71) Applicant: Danmarks Tekniske Universitet, 2800 Kgs. Lyngby (DK)
(72) Inventor: ANDERSEN, Mikael Rørdam, 3520 Farum (DK); BORODINA, Irina, 2990 Nivå (DK); DO, Duy Duc, 2700 Brønshøj (DK); LUND, Anne Mathilde, 2400 Copenhagen NV (DK)
(74) Representative: Guardian IP Consulting I/S

(57) **Abstract**

The invention provides a genetically modified Chinese Hamster Ovary (CHO) cell for production of a recombinant polypeptide, comprising a transgene encoding a CHO-derived signal peptide fused to a recombinant polypeptide, wherein said signal peptide is capable of directing co-translational transport of said polypeptide in the cell resulting in enhanced levels of polypeptide secretion. The invention further provides a method for producing a recombinant polypeptide by using the genetically modified CHO cell. The invention further provides for the use of a CHO-derived signal peptide and a transgene encoding said signal peptide to enhance production of extracellular recombinant polypeptide by a genetically modified CHO cell.

## Description

### Field of the invention

The invention provides a genetically modified Chinese Hamster Ovary (CHO) cell for production of a recombinant polypeptide, comprising a transgene encoding a CHO-derived signal peptide fused to a recombinant polypeptide, wherein said signal peptide is capable of directing co-translational transport of said polypeptide in the cell resulting in enhanced levels of polypeptide secretion. The invention further provides a method for producing a recombinant polypeptide by using the genetically modified CHO cell. The invention further provides for the use of a CHO-derived signal peptide and a recombinant gene encoding said signal peptide to enhance production of extracellular recombinant polypeptide by a genetically modified CHO cell.

### Background of the invention

Chinese hamster ovary (CHO) cells are currently the expression system of choice for production of therapeutic proteins, mainly because they are able to produce proteins that require complex folding, post-translational modifications such as glycoforms that are compatible with and bioactive in humans [Walsh G, 2014].

Accordingly, there exists an increasing interest in construction and development of mammalian cell factories for production of therapeutic proteins. This interest has been further stimulated by the recent publication of the first genome drafts of CHO cell lines [Xu X et al., 2011].

Construction of a system for secretory expression of recombinant proteins in CHO cells having high levels of production requires a balance between gene expression, the protein transport process, cell viability and the composition of the medium. For the process of protein transport specifically, the translocation of secretory proteins into the lumen of the endoplasmic reticulum (ER) may be one of the limiting steps within the classical secretory pathway. In addition to the components of the secretion apparatus, the signal peptides that channel the export proteins into the secretion machinery play a key role in the translocation across the membrane.

Secreted proteins comprise an N-terminal signal peptide of 15-30 amino acids that is cleaved off while the protein is translocated through the membrane of the ER [von Heijne G, 1985]. The structure of signal peptides from various proteins is described to share common features consisting of three domains: an amino terminal positively charged n-region, followed by a central hydrophobic h-region, and a neutral but polar c-region [von Heijne G, 1984, 1985]. Usually, the cleavage sites have small and natural residues at position -3 and -1 from the cleavage site in order for cleavage to occur. Beyond these overall patterns, no precise sequence conservation has been found or higher order structures [von Heijne G, 1984].

Signal peptides are heterogeneous and may, to some degree, be functionally interchangeable between different species [von Heijne G, 1985]. Although heterologous signal peptides from human protein interleukin-2 and interferon-gamma growth factor-β 1 and 2, interferon (IFN-γ), human albumin, and immunoglobulin G have been used for expression of therapeutic proteins in CHO cells, the limited yield of these proteins has been attributed to insufficient secretion efficiency. Accordingly, there exists a need for signal peptides that can enhance the secretion efficiency and yield of recombinant proteins expressed in CHO cells.

### Summary of the invention

According to a first embodiment, the invention provides a recombinant gene for expression of a precursor polypeptide, the gene comprising an in-frame fusion of:
a) a first nucleic acid molecule encoding a signal peptide, and
b) a second nucleic acid molecule encoding a recombinant polypeptide,
wherein the fused first and second nucleic acid molecule encode the precursor polypeptide, and wherein the signal peptide is between 15 and 25 amino acid residues in length and comprises an amino acid sequence selected from the group consisting of: SP1 [SEQ ID No.:2], SP2 [SEQ ID No.:4], SP3 [SEQ ID No.:6], SP4 [SEQ ID Neo.:8], SP5 [SEQ ID No.:10], SP7 [SEQ ID No.:14] and SP11 [SEQ ID No.:22].

The recombinant polypeptide encoded by the second nucleic acid molecule in the recombinant gene may be selected from the group consisting of a hormone, enzyme, immunoglobulin heavy chain, immunoglobulin light chain, whole immunoglobulin, fragment antigen-binding (Fab) fragment, Fc fragment, single-chain variable fragment (scFv), cytokine, interleukin, complement factor, coagulation factor, an extracellular matrix protein, and a soluble receptor.

According to a second embodiment, the invention provides a vector comprising the recombinant gene for expression of a precursor polypeptide in a CHO cell.

According to a third embodiment, the invention provides a genetically modified CHO cell transformed with a recombinant gene for expression of a precursor polypeptide according to the invention.

The genetically modified CHO cell transformed with the recombinant gene according to the invention may be derived from a CHO cell line selected from the group consisting of: CHO-K1, CHO-S or GS-CHO, CHO-Lec1, CHO-Lec10, CHO-Lec13, CHO Pro-5, CHO DX B11 and CHO DG44 cells.

According to a fourth embodiment, the invention provides a method for producing a recombinant protein *in vivo* comprising:
a) providing one or more genetically modified CHO cells transformed with a recombinant gene according to the invention;
b) introducing said one or more genetically modified CHO cells into a culture medium to produce a culture;
c) incubating the culture;
d) recovering the produced recombinant polypeptide from the culture and optionally
e) purifying the recombinant polypeptide recovered.

According to fifth embodiment, the invention provides for the use of a signal peptide to enhance the production of a recombinant polypeptide in a genetically modified CHO cell, wherein the signal peptide is fused to the recombinant protein, and wherein the signal peptide is between 15 and 25 amino acid residues in length and comprises an amino acid sequence selected from the group consisting of: SP1 [SEQ ID No.:2], SP2 [SEQ ID No.:4], SP3 [SEQ ID No.:6], SP4 [SEQ ID No.:8], SP5 [SEQ ID No.: 10], SP7 [SEQ ID No.:14] and SP11 [SEQ ID No.:22].

The recombinant polypeptide fused to the signal peptide may be selected from the group consisting of a hormone, enzyme, immunoglobulin heavy chain, immunoglobulin light chain, whole immunoglobulin, fragment antigen-binding (Fab) fragment, Fc fragment, single-chain variable fragment (scFv), cytokine, interleukin, complement factor, coagulation factor, an extracellular matrix protein, and a soluble receptor.

### Description of the invention

### Description of the figures:

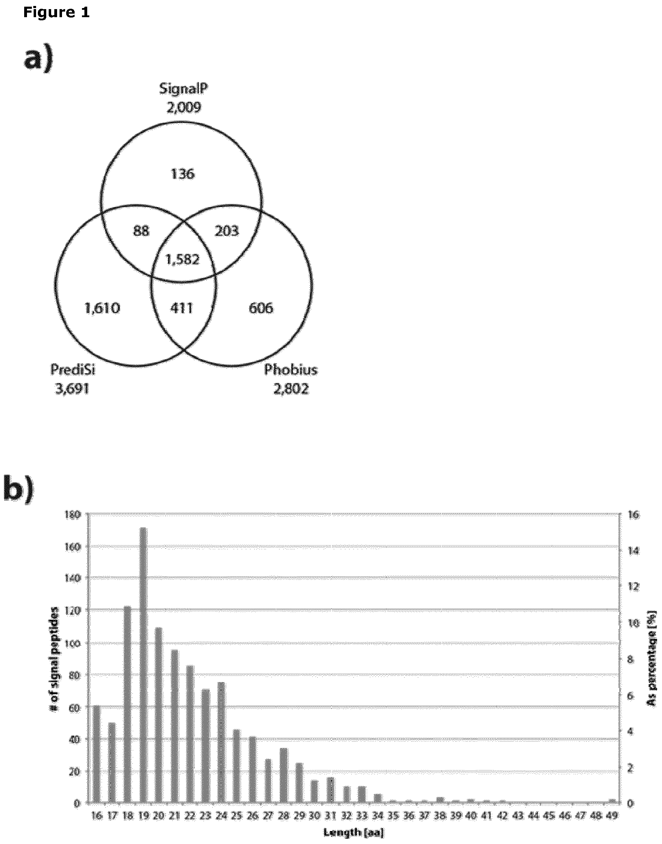

**Figure 1****.** a) Venn diagrams of predicted proteins with signal peptide cleavage site by the three computational tools: SignaIP, Phobius and PrediSi. The diagram represents the number of predicted signal peptides constituting the secretome. b) Signal peptide library length distribution represented in quantities and as percentage of the total amount of signal peptides. The 999 signal peptides represent the signal peptide library refined by predicted length of the signal peptides were consistently within the secretome.

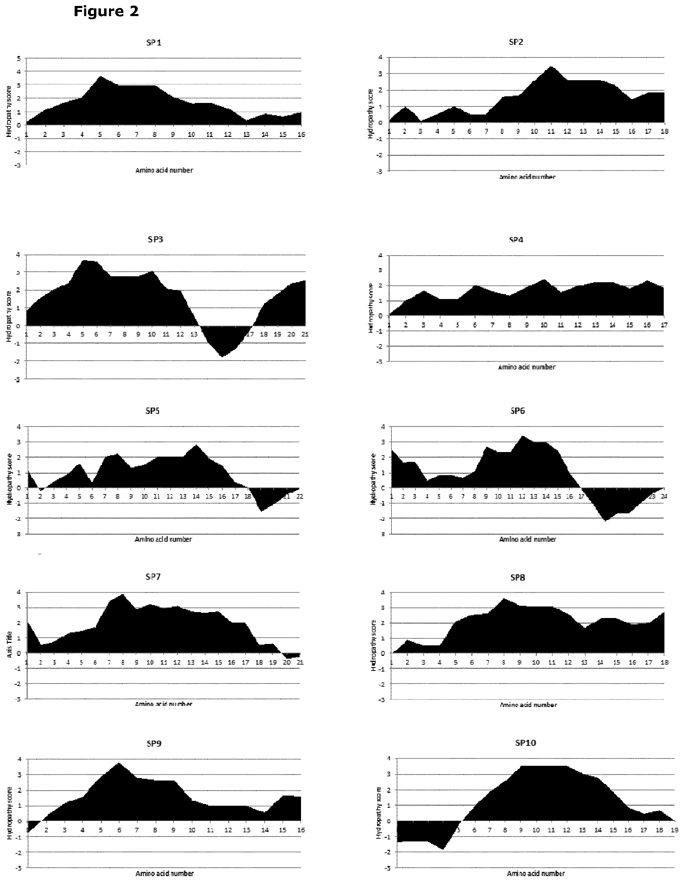

**Figure 2** Hydropathy plots of the selected 13 native CHO cell signal peptide denoted SP1-SP13.

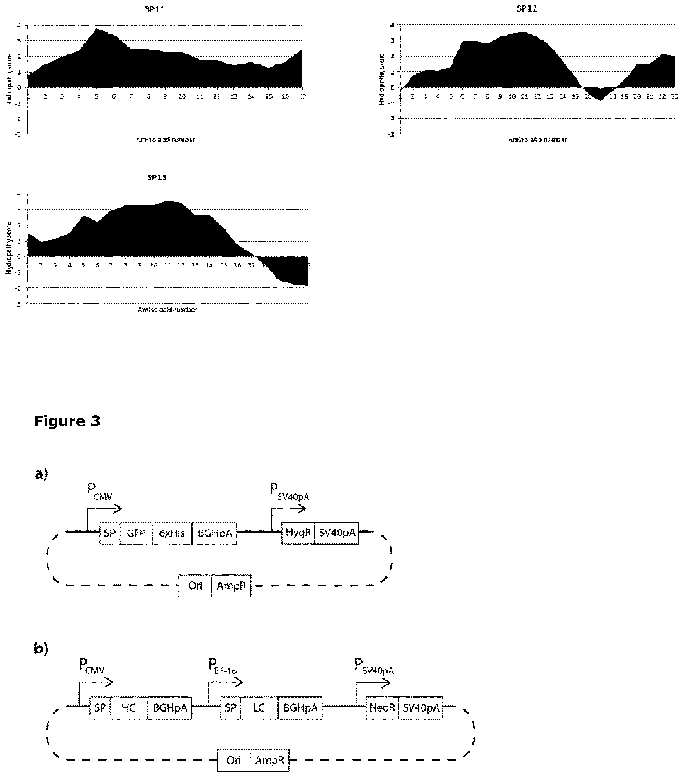

**Figure 3** Cartoon showing the gene structure of expression vectors for expression of Rituximab antibody therapeutic model glycoprotein.

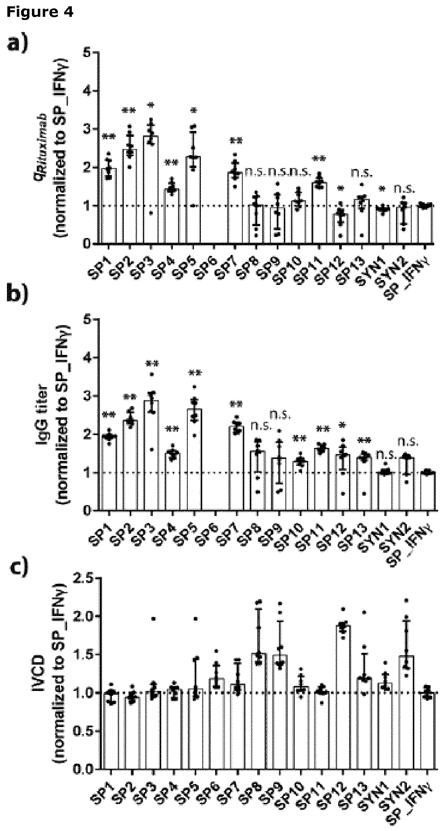

**Figure 4** Bar graph showing the integral of viable cells and cell-specific productivity, and titer of Rituximab by CHO cells comprising vectors having a gene encoding a heterologous fusion of a CHO-derived signal peptide to the heavy and light chains of Rituximab. Control vector constructs expressed in CHO cells, used as comparators, comprised a gene encoding a heterologous fusion of a control signal peptide to the heavy and light chains of Rituximab. Vectors encoding Rituximab with the different signal peptides were transfected into CHO-S cells in 96-half-deepwell microplates. Rituximab titer was determined in supernatants obtained three days after transfection. a) Cell specific productivity (qRituximab) normalized to SP_IFNγ, b) IgG titer normalized to SP_IFNγ and c) IVCD normalized to SP_IFNγ are shown. Eight (14 for SP_IgG) separate transfections were performed in each experiment and experiments were performed twice (n=2; 8 wells [n=2; 28 wells for SP_IgG]). Median and interquartile range are depicted. Statistically significant differences (p<0.01) compared to SP_IFNγ are indicated in panels a and b (* = p<0.01; ** = p<0.001; n.s. = not significant).

### Abbreviations and terms:

**gi number:** (genInfo identifier) is a unique integer which identifies a particular sequence, independent of the database source, which is assigned by NCBI to all sequences processed into Entrez, including nucleotide sequences from DDBJ/EMBL/GenBank, protein sequences from SWISS-PROT, PIR and many others.

**Amino acid sequence identity:** The term "sequence identity" as used herein, indicates a quantitative measure of the degree of homology between two amino acid sequences of substantially equal length. The two sequences to be compared must be globally aligned to give a best possible fit, by means of the insertion of gaps, where the aligned sequences can end in gaps. The sequence identity can be calculated as ((Nref-Ndif)100)/(Nref), wherein Ndif is the total number of non-identical residues in the two sequences when aligned and wherein Nref is the number of residues in one of the sequences. Sequence identity can be calculated using the BLAST program e.g. the BLASTP program (Pearson W.R and D.J. Lipman (1988)) (www.ncbi.nlm.nih.gov/cgi-bin/BLAST). Alignment of multiple sequences may be performed with the global sequence alignment method ClustalW with default parameters as described by Thompson J., et al 1994, available at http://www2.ebi.ac.uk/clustalw/.

Preferably, the numbers of substitutions, insertions, additions or deletions of one or more amino acid residues in the polypeptide as compared to its comparator polypeptide is limited, i.e. no more than 1, 2, 3, 4, 5, 6, 7, 8, 9, or 10 substitutions, no more than 1, 2, 3, 4, 5, 6, 7, 8, 9, or 10 insertions, no more than 1, 2, 3, 4, 5, 6, 7, 8, 9, or 10 additions, and no more than 1, 2, 3, 4, 5, 6, 7, 8, 9, or 10 deletions. Preferably the substitutions are conservative amino acid substitutions: limited to exchanges within members of group 1: Glycine, Alanine, Valine, Leucine, Isoleucine; group 2: Serine, Cysteine, Selenocysteine, Threonine, Methionine; group 3: Proline; group 4: Phenylalanine, Tyrosine, Tryptophan; Group 5: Aspartate, Glutamate, Asparagine, Glutamine.

**Cell specific productivity of the recombinant polypeptide:** the amount of recombinant polypeptide produced, normalised to the number of cells and per day of cultivation.

**Cognate native signal peptide** is defined as a signal peptide that is found in nature, as a translational fusion, at the amino-terminus of a polypeptide. A polypeptide can be expressed recombinantly *in vivo,* as a translational fusion of the polypeptide with its cognate native signal peptide.

**In frame translational fusion:** the in-frame joining of two or more nucleic acid molecules, that each originally coded for separate peptides and/or protein proteins, is used to artificially create a recombinant gene DNA, and such that the reading frame of the joined nucleic acid molecules is preserved, and the translation product of the recombinant gene is a fusion polypeptide

**Native gene:** endogenous gene in a microbial cell genome, homologous to host micro-organism.

**Non-CHO-derived signal peptide:** is a signal peptide that is not native to a CHO cell, and is not encoded as an N-terminal signal peptide sequence by the native CHO genome.

**Operably linked:** a gene (nucleic acid molecule comprising a coding sequence) is operably linked to a promoter when its transcription is under the control of the promoter and where transcription results in a transcript whose subsequent translation yields the product encoded by the gene.

**Recombinant gene:** Recombinant gene is composed of nucleic acid molecules formed by laboratory methods of genetic recombination (such as molecular cloning) to bring together genetic material from 2 or more sources, creating sequences that would not otherwise be found in the genome.

**Transgene:** in the context of the invention is a gene or genetic material that has been transferred by any of a number of genetic engineering techniques from one organism to another.

**Titer of the recombinant polypeptide:** concentration of the recombinant polypeptide in the culture medium.

**Vector:** The term "vector" refers to viral or non-viral, prokaryotic or eukaryotic, deoxyribonucleic acid, ribonucleic acid or a nucleic acid analog, that is capable of carrying another nucleic acid. A vector may either carry a nucleic acid into a cell, referred to as "host cell," so that all or a part of the nucleic acid is transcribed or expressed. Vectors are frequently assembled as composites of elements derived from different viral, bacterial, or mammalian genes. Vectors contain various coding and non-coding sequences including sequences coding for selectable markers (e.g., an antibiotic resistance gene), sequences that facilitate their propagation in bacteria, or one or more transcription units that are expressed only in certain cell types. For example, mammalian expression vectors often contain both prokaryotic sequences that facilitate the propagation of the vector in bacteria and one or more eukaryotic transcription units that are expressed only in eukaryotic cells. It will be appreciated by those skilled in the art that the design of the expression vector can depend on such factors as the choice of the host cell to be transformed, the level of expression of protein desired, etc.

### Detailed description of the invention

The present invention is based on the first genome-wide analysis of the secretome to identify native secretion signal peptides of CHO cells. The invention is further based on evidence that native CHO cell secretion signal peptides can outperform the standard signal peptides currently used in biopharmaceutical production by enhancing the secretion efficiency and yield of recombinant proteins expressed in CHO cells. In order to investigate native CHO cell secretion signal peptides, *in silico* tools for prediction of the CHO-K1 secretome (Genbank assembly GCF_000223135.1) were used to identify potential secretion signal sequences. The identification was based on the premise that signal peptides must consistently rank as a potential secretion signal across a range of prediction tools, in order to efficiently narrow down the search space and increase the possibility of detecting an efficient signal peptide. To analyze the functionality and demonstrate secretion efficiency of native CHO cell secretion signal peptides, 13 predicted signal peptides were tested *in vivo* for polypeptide production and secretion as compared to signal peptides derived from human interferon-gamma and interleukin-2, that are widely used in biopharmaceutical production, and two synthetic signal peptides.

### I In silico identification of the CHO-K1 secretome

A combination of three signal peptide prediction tools facilitated a comprehensive bioinformatical analysis of the CHO-K1 proteome, which resulted in generating a secretome of CHO cells, constituting 1,582 proteins; and generating a signal peptide library constituting of 999 proteins (see Example 1). By additional use of predictors for transmembrane helices (TMHs), subcellular localisation and protein function combined with references to the primary and secondary literature, signal peptides of 13 proteins having potentially high secretory efficacy were selected (see Example 2).

### II CHO-derived signal peptides for enhanced secretion of a recombinant polypeptide expressed in CHO cells

The present invention provides for the use of a CHO-derived signal peptide for enhancing the secretion of a recombinant polypeptide *in vivo* in CHO cells, where the recombinant polypeptide is expressed as a precursor polypeptide comprising the signal peptide N-terminally fused to the recombinant polypeptide. The signal peptide is selected from the group consisting of signal peptides SP1, SP2, SP3, SP4, SP5, SP7 and SP11. Each of these signal peptides preferably has a length of between 15 and 25 amino acid residues. The amino acid sequence of each signal peptide (see Table 2) comprises (or consists of) the following sequence: SP1 [SEQ ID No.:2], SP2 [SEQ ID No.:4], SP3 [SEQ ID No.:6], SP4 [SEQ ID No.:8], SP5 [SEQ ID No..:10], SP7 [SEQ ID No.:14] and SP11 [SEQ ID No.:22].

From amongst the 13 selected signal peptides, the CHO-derived signal peptides SP1, SP2, SP3, SP4, SP5, SP7 and SP11 (see Table 2) were all demonstrated to enhance the secretion of a recombinantly expressed polypeptide *in vivo* in CHO cells as compared to control. This was demonstrated for the biopharmaceutical model antibody glycoprotein (Rituximab) and for secreted alkaline phosphatase (SEAP). These 7 high-performing CHO-derived signal peptides also improved the cell-specific productivity and titer of Rituximab when compared to the widely used signal peptides derived from human interferon-gamma, IFNγ (SP_IFNγ). Additionally the cell-specific productivity and titer of Rituximab when using any of the 7 high-performing CHO-derived signal peptides was also enhanced when compared to two computer-generated synthetic signal peptide sequences that have shown promising secretion efficiency of SEAP in HEK293 cells [Barash S et al., 2002].

The recombinant polypeptide, whose expression and secretion in a CHO cell is enhanced by use of a CHO-derived signal peptide, may be any polypeptide that is secreted by a CHO cell. The recombinant polypeptide may be chosen from a hormone, an enzyme, an immunoglobulin heavy chain or light chain, a whole immunoglobulin or any fragment derived from an immunoglobulin, a protein involved in the immune response such as cytokines, interleukins, complement factors, a chimeric protein, or another therapeutic protein such as a coagulation factor, an extracellular matrix protein, or a soluble receptor. Preferably the recombinant polypeptide is an immunoglobulin heavy chain and/or a light chain, a whole antibody or a fragment thereof (e.g. fragment antigen-binding (Fab) fragment, Fc fragment, single-chain variable fragment (scFv)).

### III A recombinant gene for enhanced expression of a polypeptide in vivo in CHO cells

The invention further provides a first nucleic acid molecule encoding a CHO-derived signal peptide for enhancing the secretion of a recombinantly-expressed polypeptide *in vivo* in CHO cells, where the signal peptide is selected from the group consisting of signal peptides SP1, SP2, SP3, SP4, SP5, SP7 and SP11. Each of these signal peptides preferably has a length of between 15 and 25 amino acid residues (see Table 2). The amino acid sequence of each signal peptide (see Table 2) encoded by the nucleic acid molecule comprises (or consists of) the following sequence: SP1 [SEQ ID No.:2], SP2 [SEQ ID No.:4], SP3 [SEQ ID No.:6], SP4 [SEQ ID No.:8], SP5 [SEQ ID No.:10], SP7 [SEQ ID No.:14] and SP11 [SEQ ID No.:22].

When using the nucleic acid molecule, encoding the CHO-derived signal peptide, for the purpose of enhancing the secretion of a recombinantly expressed polypeptide *in vivo* in CHO cells, the signal peptide coding sequence of the nucleic acid molecule is fused in frame to a nucleic acid molecule encoding the recombinant polypeptide to be expressed in a CHO cell. A recombinant gene comprises the in-frame fusion of the nucleic acid molecule encoding the signal peptide and the nucleic acid molecule encoding the recombinant polypeptide. For the purpose of enhancing the secretion of a recombinantly expressed polypeptide *in vivo* in CHO cells, the recombinant gene may be operably linked to a nucleic acid molecule comprising a promoter and additionally may be operably linked to a nucleic acid molecule functioning as a terminator. The recombinant gene, comprising nucleic acid sequences functioning as a promoter and/or terminator, may then be introduced into a CHO cell, where subsequent incubation of the cell under suitable cultivation conditions facilitates expression and secretion of the recombinant polypeptide.

The recombinant gene of the invention encodes a fusion polypeptide, comprising a CHO-derived signal peptide (as defined above) fused to a polypeptide, where the polypeptide is preferably heterologous with respect to the signal peptide; and where the amino acid sequence of the encoded fusion is not therefore to be found in nature. For expression of the fusion polypeptide, the recombinant gene is transferred into a host CHO cell where it may be expressed as a transient or a stably integrated transgene. The fusion polypeptide encoded by the transgene is a precursor polypeptide, where the signal peptide at its amino-terminal end causes the precursor polypeptide to pass into the rough endoplasmic reticulum (RER) where it will be matured, and where the signal peptide is normally cleaved off by a peptidase at the signal peptide cleavage site.

The recombinant polypeptide encoded by the recombinant gene may be chosen from a hormone, an enzyme, an immunoglobulin heavy chain or light chain, a whole immunoglobulin or any fragment derived from an immunoglobulin, a protein involved in the immune response such as cytokines, interleukins, complement factors, a chimeric protein, or another therapeutic protein such as a coagulation factor, an extracellular matrix protein, or a soluble receptor. Preferably the recombinant polypeptide is an immunoglobulin heavy chain and/or a light chain, a whole antibody or a fragment thereof (e.g. fragment antigen-binding (Fab) fragment, Fc fragment, single-chain variable fragment (scFv)).

According to one embodiment the recombinant polypeptide is an immunoglobulin composed of a heavy chain and/or a light chain or comprises a heavy chain and/or a light chain variable domain, where the immunoglobulin heavy and light chain (or heavy chain and/or a light chain variable domain) is selected from the group of: rituximab (having heavy chain [SEQ ID No.:28] and the light chain [SEQ ID No.:30]; Abciximab; Adalimumab (heavy chain [SEQ ID No.: 98] and light chain [SEQ ID No.: 99] variable domains); Alemtuzumab (complete light chain [SEQ ID No.: 100] and complete heavy chain [SEQ ID No.:101]; Altumomab pentetate; Arcitumomab; Atlizumab (= tocilizumab); Basiliximab (heavy chain [SEQ ID No.: 102] and light chain [SEQ ID No.: 103] variable domains); Bectumomab; Belimumab (heavy chain [SEQ ID No.:104] and light chain [SEQ ID No.:105] variable domains); Besilesomab; Bevacizumab (complete heavy chain [SEQ ID No.:106] and complete light chain [SEQ ID No.: 107]; Biciromab; Canakinumab (heavy chain [SEQ ID No.:108] and light chain [SEQ ID No.: 109] variable domains); Capromab pendetide; Catumaxomab; Certolizumab pegol; Cetuximab (light chain [SEQ ID No.:110] and heavy chain [SEQ ID No.:111] variable domains); Clivatuzumab (complete light chain [SEQ ID No.: 112] and complete heavy chain [SEQ ID No.:113]); Daclizumab (complete light chain [SEQ ID No.:114] and complete heavy chain [SEQ ID No.:115]); Denosumab (heavy chain [SEQ ID No.:116] and light chain [SEQ ID No.:117] variable domains)]; Eculizumab (complete heavy chain [SEQ ID No.:118] and complete light chain [SEQ ID No.:119]); Edrecolomab [SEQ ID No.:]; Efalizumab (light chain [SEQ ID No.:120] and heavy chain [SEQ ID No.: 121] variable domains); Efungumab; Ertumaxomab; Etaracizumab (complete heavy chain [SEQ ID No.: 122] and complete light chain [SEQ ID No.: 123]); Fanolesomab; Fontolizumab (complete light chain [SEQ ID No.: 124] and complete heavy chain [SEQ ID No.: 125]); Gemtuzumab ozogamicin; Girentuximab (complete light chain [SEQ ID No.: 126] and complete heavy chain [SEQ ID No.:127]); Golimumab (heavy chain [SEQ ID No.:128] and light chain [SEQ ID No.: 129] variable domains)]; Ibritumomab tiuxetan (heavy chain [SEQ ID No.:130] and light chain [SEQ ID No.:131] variable domains); Igovomab; Imciromab; Infliximab (heavy chain [SEQ ID No.: 132] and light chain [SEQ ID No.:133] variable domains); Ipilimumab (heavy chain [SEQ ID No.:134] and light chain [SEQ ID No.: 135] variable domains); Labetuzumab (complete heavy chain [SEQ ID No.:136] and complete light chain [SEQ ID No.:137]); Mepolizumab (heavy chain [SEQ ID No.: 138] and light chain [SEQ ID No.: 139] variable domains); Motavizumab (complete light chain [SEQ ID No.: 140] and complete heavy chain [SEQ ID No.:141]); Muromonab-CD3; Natalizumab (light chain [SEQ ID No.: 142] and heavy chain [SEQ ID No.: 143] variable domains); Nimotuzumab (light chain [SEQ ID No.: 144] and heavy chain [SEQ ID No.: 145] variable domains)]; Nivolumab (complete heavy chain [SEQ ID No.: 146] and complete light chain [SEQ ID No.: 147]); Nofetumomab merpentan; Obinutuzumab (complete heavy chain [SEQ ID No.: 148] and complete light chain [SEQ ID No.: 149]); Ofatumumab (heavy chain [SEQ ID No.: 149] and light chain [SEQ ID No.: 150] variable domains); Omalizumab (complete heavy chain [SEQ ID No.: 152] and complete light chain [SEQ ID No.: 153]); Oregovomab; Palivizumab (complete heavy chain [SEQ ID No.: 154] and complete light chain [SEQ ID No.: 155]); Panitumumab (complete heavy chain [SEQ ID No.: 156] and complete light chain [SEQ ID No.: 157]); Pemtumomab; Pertuzumab (heavy chain [SEQ ID No.: 158] and light chain [SEQ ID No.: 159] variable domains); Ramucirumab (complete heavy chain [SEQ ID No.:160] and complete light chain [SEQ ID No.:161]); Ranibizumab (heavy chain [SEQ ID No.:162] and light chain [SEQ ID No.:163] variable domains); Rovelizumab; Ruplizumab (heavy chain [SEQ ID No.: 164] and light chain [SEQ ID No.:165] variable domains); Sulesomab; Tacatuzumab tetraxetan; Tefibazumab; Tositumomab; Trastuzumab (complete heavy chain [SEQ ID No.:166] and complete light chain [SEQ ID No.:167]); Trastuzumab emtansine (complete heavy chain [SEQ ID No.:168] and complete light chain [SEQ ID No.:169]); Ustekinumab (complete light chain [SEQ ID No.: 170] and complete heavy chain [SEQ ID No.:171]); Visilizumab (complete light chain [SEQ ID No.:172] and complete heavy chain [SEQ ID No.: 173]) Votumumab; Zalutumumab (light chain [SEQ ID No.: 174] and heavy chain [SEQ ID No.: 175] variable domains); and Zanolimumab (light chain [SEQ ID No.:176] and heavy chain [SEQ ID No.:177] variable domains).

A DNA molecule capable of functioning as a promoter in a CHO cell that may be operably linked to the recombinant gene, may be selected from among a number of constitutive or inducible promoters known in the art. As an example, a suitable promoter for directing constitutive or inducible expression of the recombinant gene in a CHO cell may be selected from the cytomegalovirus (CMV) promoter [SEQ ID No.: 31]; SV40 early promoter (SV40pA) GenBank: E00567.1 [SEQ ID No.: 32].

A DNA molecule capable of functioning as a terminator of a recombinant gene for expression of a polypeptide in a CHO cell, that may be operably linked to the recombinant gene, may be selected from the bovine growth hormone polyadenylation (bgh-PolyA) signal [SEQ ID No.:32] (nucleotides 1943-2167 in GenBank E00847.1); SV40 poly adenylation signal [SEQ ID No.:34] (nucleotides 5929-6181 in GenBank: A70359.1)

### IV: A genetically modified CHO cell capable of enhanced secretion of a recombinant polypeptide

The invention further provides a genetically modified CHO cell comprising a transgene. The transgene, introduced into the CHO cell, is a recombinant gene comprising a coding sequence for a CHO-derived signal peptide for enhancing the secretion of a recombinantly expressed polypeptide *in vivo* (as described in Section III). The signal peptide is selected from the group consisting of signal peptides SP1, SP2, SP3, SP4, SP5, SP7 and SP11. Each of these signal peptides preferably has a length of between 15 and 25 amino acid residues (see Table 2). The amino acid sequence of each signal peptide (see Table 2) encoded by the nucleic acid molecule comprises (or consists of) the following sequence: SP1 [SEQ ID No.:2], SP2 [SEQ ID No.:4], SP3 [SEQ ID No.:6], SP4 [SEQ ID No.:8], SP5 [SEQ ID No.:10], SP7 [SEQ ID No.: 14] and SP11 [SEQ ID No.:22].

The coding sequence of the transgene encodes a fusion polypeptide, comprising a CHO-derived signal peptide (as defined above) fused in-frame to a recombinant polypeptide. The transgene may be localized on a transiently expressed vector in the genetically modified CHO cell or the transgene (transformed into the CHO cell) may be stably integrated into the genome of the CHO cell. Suitable vectors for transient expression of a transgene in the genetically modified CHO cell of the invention are well known in the art; for example pcDNA3.1 from Thermo Fischer, or CHEF1 vectors from CMC Biologics.

The CHO cell may be obtained from a number of biological resource centers such as the European Collection of Cell Cultures (ECACC). A number of variants of the first isolated CHO-K1 cell line are available. A suitable CHO cell line for expression of a secreted recombinant protein according to the invention includes CHO-K1, CHO-S or GS-CHO, CHO-Lec1, CHO-Lec10, CHO-Lec13, CHO Pro-5, CHO DX B11 and CHO DG44 cells, in particular CHO-S or GS-CHO.

### V: A method for producing a recombinant polypeptide in a genetically modified CHO using a signal peptide of the invention

According to a further embodiment, the invention provides a method for enhancing expression of a recombinant protein in a CHO cell, comprising:
a. providing a CHO cell comprising a transgene, wherein said transgene comprises:
   i. a first nucleic acid molecule encoding a CHO-derived signal peptide, and
   ii. a second nucleic acid molecule encoding a recombinant polypeptide;
   wherein said first nucleic acid molecule is translationally fused in-frame to said second nucleic acid molecule and wherein the amino acid sequence of said CHO-derived signal peptide is selected from the group consisting of signal peptides SP1, SP2, SP3, SP4, SP5, SP7 and SP11, and
b. culturing said CHO cell in a culture medium; and
c. recovering said recombinant protein from the culture.

The transgene, comprising the recombinant gene described in Section III, may be localized on a transiently expressed vector in the genetically modified CHO cell. Alternatively, the transgene transformed into the CHO cell, may be stably integrated into the genome of the CHO cell. The recombinant gene may be introduced into the host CHO cell by transfection. For example, vector DNA comprising the recombinant gene can be complexed with lipid reagents to mediate efficient delivery into the cell's nucleus. For example, cationic lipid-based reagents spontaneously form condensed nucleic acid-cationic lipid reagent complexes via electrostatic interactions between the negatively charged nucleic acid and the positively charged head group of the synthetic lipid reagent. These complexes are believed to be taken up by the cell through endocytosis and then released in the cytoplasm. Once in the cell, transfected DNA is translocated to the nucleus to be expressed.

Approximately one in 10⁴ transfected cells will stably integrate the transgene DNA, although the efficiency varies with cell type; where integration is most efficient when linear DNA is used. The selection of genetically modified CHO cells that stably express transfected DNA can be achieved by including a selectable marker on the DNA vector used for transfection, and then applying the corresponding selective pressure to the cells after a short recovery period.

The genetically modified CHO cell comprising the transgene of the invention is cultured in a growth medium and under conditions that support protein synthesis and production of the recombinant protein encoded by the transgene. Suitable cultivation conditions for production of a recombinant protein by a genetically modified CHO cell are described in Animal Cell Technology: From Biopharmaceuticals to Gene Therapy (2008) by editors Leda R. Castilho et al.

The polypeptide encoded by the transgene is a precursor polypeptide comprising the CHO-derived signal peptide fused to the recombinant protein. The signal peptide of the precursor polypeptide is co-translationally cleaved off the precursor polypeptide, such that the amino acid sequence of the recombinant protein secreted by the CHO cell is that of the mature recombinant protein.

The method of the invention, using CHO cells transfected with a transgene of the invention (encoding a precursor polypeptide comprising in-frame translational fusion of the CHO-derived signal peptide to the recombinant protein), is characterized by an enhanced cell-specific productivity and/or titer of the recombinant polypeptide, as compared to a transgene encoding the recombinant protein translationally fused to its native homologous signal peptide. Preferably, the titer and cell-specific productivity of the recombinant polypeptide by the transfected CHO cells of the invention is enhanced as compared to a transgene encoding the recombinant protein that is in-frame translationally fused to a signal peptide derived from human interferon-gamma or interleukin-2, or a cognate native signal peptide of the recombinant protein, or a non-CHO derived signal peptide.

### VI Methods for producing a transgene of the invention

Integration and transient expression vectors suitable for cloning and introducing a transgene encoding a translational fusion of the CHO-derived signal peptide of the invention to a recombinant polypeptide are commercially available and known to those skilled in the art (e.g. Animal Cell Technology: From Biopharmaceuticals to Gene Therapy (2008) by editors Leda R. Castilho et al.). Suitable methods for transfection to integrate the transgene into a CHO cell are given in Section V. Examples of recombinant expression of transgenes encoding a translational fusion of the CHO-derived signal peptide of the invention to a recombinant polypeptide in a CHO cell of the invention is demonstrated in Example 3.

### VI Evaluation of the Level of Recombinant Polypeptide Secreted

Methods for detecting and quantifying extracellular recombinant polypeptide produced by a CHO cell of the invention, depends on the polypeptide expressed by the transgene. For example, when the recombinant polypeptide is an IgG, suitable methods include the Fast ELYSA kit (RD-biotech) according to the supplier's instructions. The optical density is read with a spectrophotometer at 450 nm. The antibody concentration is determined in comparison with a standard range contained in the kit.

### Examples

### Example 1 in silico prediction of the CHO-K1 secretome

### 1.1 In silico analysis:

The proteome (Genbank assembly GCF_000223135.1) of the CHO-K1 cell line (Baycib-Hizal et al., 2012) was used for the *in silico* construction of a CHO cell signal peptide library. The three computational programs SignalP 4.1 [Petersen TN et al, 2011], Phobius [Käll L, et al., 2004], and PrediSi [Hiller K et al., 2004] were applied to predict signal peptide cleavage sites in order to generate a CHO-K1 secretome and create signal peptide library. Phobius also comprises a TMH predictor in order to discriminate between signal peptides and helices and thereby increase the signal peptide prediction accuracy by [Käll L, et al., 2004].

Transmembrane predictions of the signal sequences were performed using the TMHMM server [Krogh A et al, 2001]. Subcellular localisation of the secretome was analysed with Yloc+ [Briesemeister S., et al, 2010] and SubCellProt [Garg P. et al, 2009]. Finally, protein function was determined by comparing functional protein domains with gene annotation predicted by HmmScan [Finn RD et al, 2011] and Genbank, respectively. The hydrophobicity of selected signal peptides was estimated using the ExPASy program [Gasteiger E. et al., 2003] and the Kyte & Doolittle scale [Kyte J, et al., 1982] using a sliding window of five residues. OligoAnalyser 3.1 [Owczarzy R, et al., 2008] was used for analysing potential primer secondary structures. All the servers for the different bioinformatic tools used in this study are listed in Table 1.

**Table 1. In silico tools used for prediction of secretome and signal peptide library construction.**

| **Prediction tool** | **Prediction** | **URL** | **Reference** |
|---|---|---|---|
| **SignalP 4.1** | Signal peptide | http://www.cbs.dtu.dk/services/Signal P/ | |
| **Phobius** | Signal peptide | http://phobius.binf.ku.dk | |
| **PrediSi** | Signal peptide | http://www.predisi.de | |
| **TMHMM** | Transmembrane helix | http://www.cbs.dtu.dk/services/TMHM M | |
| **HMMScan** | Protein domain | http://hmmer.janelia.org/search/hmms can | |
| **Genbank** | Protein annotation | http://www.ncbi.nlm;nih.gov/genbank | |
| **SubCellProt** | Subcellular localisation | http://www.databases.niper.ac.in/SubC ellProt | |
| **Yloc** | Subcellular localisation | http://abi.inf.unituebinaen.de/Services/YLoc/webloc.cgi | |
| **OligoAnalys er 3.1** | Primer secondary structures | http://eu.idtdna.com/analyser/Applicat ions/OligoAnalyser/ | |

### 1.2 Results

Of the 24,238 (GenBank: AFTD00000000.1) putative CHO-K1 protein sequences, a total of 8.502 proteins were predicted to have a signal peptide. SignalP predicted 2,009 (8.3% of total number of protein sequences) proteins to encode a signal peptide. In comparison Phobius and PredSi predicted 2,802 (11.6 %) and 3,691 (15.2 %) proteins with signal peptide, respectively (see Figure 1 a). 1,582 (6.5 %) proteins were predicted by all three methods to encode a signal peptide. Further, to increase the likelihood of precise prediction of the signal peptides, the predicted length of the signal peptides were integrated. Thereby, out of the 1,582 predicted signal peptides, 999 (4.1% of total number of protein sequences or 63 % of predicted secreted protein) were predicted to have the same length by all three methods. 2,352 proteins (28% of predicted secreted protein) were predicted by only one model.

These 999 predicted signal peptides constituted the signal peptide library of CHO-K1. The length of the signal peptide library varies from 16 to 49 residues, with a mean length of 21.9 residues (see Figure 1b). 64.8% of the signal peptides have a length between 18 and 24 residues, with 19 residues as the most common length, constituting 15.8 % of the library. Only 53 (5.3 %) signal peptides were predicted to be longer than 30 residues.

### Example 2 In silico selection of a small subset of diverse and potentially potent signal peptides

### 2.1 In silico selection methods

A small subset of diverse and potentially potent native CHO cell signal peptides signal peptides was first screened for *in silico* for subsequent in vivo testing. The selection was performed in several steps using bioinformatics prediction tool for analyzing secondary structures, functional domains, cellular location of proteins, and natural function. Firstly, as proteins destined to be membrane proteins can also encode a SP that resembles those of secreted proteins, potential membrane signal peptide were excluded from the library of 999 signal peptides. Exclusion was based on the presence of transmembrane helices (TMHs) structures, indicative of membrane location [48]. Using the TMHMM server [Krogh A et al., 2001] which is designed for predicting TMHs, a total of 337 proteins (33.7 %) were then discarded as signal peptides. Secondly, the remaining 662 signal peptides were analysed for the presence of functional domains in the protein by the HMMScan server [Finn RD et al., 2011] and compared to Genbank annotations, which further reduced the number of potential signal peptides to 50. These 50 signal peptides were then subjected to: 1) Predicting the proteins subcellular location using both Yloc+ [Briesemeister S, et al., 2010] and SubCellProt [Garg P et al., 2009] for evidence of secretion and natural function; 2) hydrophobicity patterns were predicted by use of the CLC Main Workbench (CLC bio, Denmark) with Kyte & Doolittle scale [Kyte J,et al., 1982] for determination of secretion efficiency; 3) potential hairpin structures within the signal peptide were assessed by OligoAnalyzer 3.1.

### 2.2 Results:

A total of 13 signal peptides were selected by this procedure, the peptides having different lengths, hydrophobicity and a variety of origins e.g. hormone and enzyme selected (Table 2 and Figure 2).

**Table 2. Characteristics of the selected CHO signal peptides and comparator signal peptides**

| **Name** | **Accession number** | **Amino acid sequence** | **Length [aa]** | **D score^{a}** | **Sub-cellular Localizati on^{b}** | **Hydrop hobicity ^{c}[%]** | **SEQ ID No.:** |
|---|---|---|---|---|---|---|---|
| SP1 | EGW05316 | MKFLLLVTLLTAG ATA | 16 | 0.804 | E/EE | 75 | 2 |
| SP2 | EGW11684 | MQMLQLWTLAV LLGAVAG | 18 | 0.913 | E/EE | 83 | 4 |
| SP3 | EGW05785 | MKILLLCVGLLLT WDNGMVLG | 21 | 0.769 | E/EE | 76 | 6 |
| SP4 | EGW08182 | MWTLVSWLAFM AGLVAG | 17 | 0.931 | E/EE | 88 | 8 |
| SP5 | EGW00421 | MSCRFVFQLLGL WLLLSQPCRA | 22 | 0.881 | E/EE | 63 | 10 |
| SP6 | EGW08477 | MTSQIRQNYSTEV EAVVTHLVNLH | 24 | 0.871 | E/EE | 83 | 12 |
| SP7 | EGW07653 | MAFRAICVLVGVF ICSISVRG | 21 | 0.736 | P/EE | 76 | 14 |
| SP8 | EGW05330 | MKALWAVLVVTL LAGCLA | 18 | 0.913 | E/EE | 83 | 16 |
| SP9 | EGW00575 | MKGLILFLSLLKM SLA | 16 | 0.890 | E/EE | 75 | 18 |
| SP10 | EGV97682 | MRPWPSLVLALL VLGCSSA | 19 | 0.899 | E/EE | 74 | 20 |
| SP11 | EGW15258 | MNLLLLLATLCLG TALA | 17 | 0.890 | LE/LL | 76 | 22 |
| SP12 | EGV95205 | MARLCAFLLALV VMSYWSTCCLG | 23 | 0.868 | E/EE | 65 | 24 |
| SP13 | EGV92826 | MPVSALLCCLLLL AGVGPSRG | 21 | 0.891 | E/EE | 71 | 26 |

| | | | | | | | |
|---|---|---|---|---|---|---|---|
| Abbreviations: E=Extracellular space, L=Lysozome and P=Plasma membrane. SubCellProt uses two models for the prediction hence the double abbreviation. **a** D score calculated by SignalP 3.0 (http://www.cbs.dtu.dk/services/SignalP/). **b** Location predicted by Yloc+ / SubCellProt. **c** Percentage of hydrophobic amino acids in each signal sequence, calculated with amino acids G, A, V, L, I, M, F, W, and P defined as hydrophobic and any other amino acid defined as hydrophilic. | | | | | | | |

### Example 3 In vivo testing of native CHO signal peptides secretion rate of Rituximab

Expression vectors were constructed comprising a nucleotide coding sequence for each of the 13 selected native CHO signal peptides fused to the coding sequence for the therapeutic model glycoprotein: Rituximab IgG antibody (heavy chain [SEQ ID No. 27] and light chain [SEQ ID No. 29]), in substitution for the nucleotide sequences encoding their respective native signal peptides. For comparison, expression plasmids comprising the coding sequences for two synthetic signal peptides (SYN1 [SEQ ID No. 36] and SYN2 [SEQ ID No. 38) and the signal peptide from human interferon-gamma, IFNγ (SP_IFNγ) [SEQ ID No. 40] were each similarly constructed for expression of the Rituximab IgG antibody (Table 3). These reference signal peptides were chosen as comparators since they have been used (or are commonly used) in both commercial protein production and gene therapy research. The rate of protein secretion facilitated by the signal sequences was analyzed by use of a high-throughput screening platform of transient expression in CHO-S cells.

**Table 3. Signal peptides used for comparison with native CHO signal peptides**

| **Name** | **Amino acid sequence** | **Length [aa]** | **Reference** | **SEQ ID No.** |
|---|---|---|---|---|
| SYN1 | MWWRLWWLLLLLLLLWPMVWA | 21 | [1] | 36 |
| SYN2 | MRPTWAWWLFLVLLLALWAPARG | 23 | [1] | 38 |
| IFNγ | MKYTSYILAFQLCIVLGSAG | 20 | [2] | 40 |

| | | | | |
|---|---|---|---|---|
| [1]: Barash S et al., 2002 [2]: UniProtKB - P01579 | | | | |

### 3.1 Vector design and construction

The genes encoding the different signal peptides, N-terminally fused to the Rituximab light and heavy chains, were located on the same vector, the two genes being expressed under the control of the human elongation factor 1α promoter (EF-1α) and cytomegalovirus (CMV) promoter, respectively (Figure 3). The vectors were constructed with uracil-specific excision reagent (USER) cloning method according to previously described [Lund AM et al., 2014]. The nucleotide components of the vector were amplified with uracil-containing primers (DNA Technologies, Leuven, Belgium) and proofreading polymerase PfuX7 [Nørholm MH, 2010]. The CMV promoter, Neomycin resistance gene (NeoR) expression cassette (pSV40-NeoR-SV40 pA), and bovine growth hormone polyadenylation signal (BGHpA) coding sequence were each amplified directly from commercial expression vectors. The promoter of the Human elongation factor gene (EF-1α) promoter, and the coding sequence for the model antibody Rituximab heavy (HC) and light (LC) chains were synthesized. Primer sequences and all vectors used as PCR templates are listed in Table 4 and 5, respectively. Following purification of PCR products by agarose gel separation and illustra GFX Gel Band Purification Kit (GE Healthcare), the purified PCR fragments were assembled with USER enzyme (New England Biolabs, Ipswich, MA) and transformed into *E. coli* Mach1 competent cells (Life Technologies, Leuven, Belgium). Vectors were purified from transformants selected on LB agar plates with ampicillin. All constructs were verified with sequencing and purified using NucleoBond Xtra Midi EF (Macherey-Nagel, Düren, Germany) or Qiagen Plasmid Plus Midi kit (Qiagen, CA, USA) according to manufacturer's instructions.

**Table 4. Primer sequences**

| **Name** | **Purpose** | **Sequence (5'→ 3')** | **SEQ ID No.** |
|---|---|---|---|
| CMV_FW | CMV | **ACGTCGCU**CGATGTACGGGCCAGATATAC | 41 |
| CMV_RV | CMV | **ATCGCACU**ATTTCGATAAGCCAGTAAGCAGT | 42 |
| BGH pA_FW | BGHpA | **ACACAGTCU**CTGTGCCTTCTAGTTGCCAGC | 43 |
| BGH pA_RV | BGHpA | **ACGCAAGU**CCATAGAGCCCACCGCATC | 44 |
| BGHpA_HC-Ritux_FW | BGHpA (HC-Rituximab) | **ATTAAGACU**CTGTGCCTTCTAGTTGCCAGC | 45 |
| BGHpA_HC-Ritux_RV | BGHpA (HC-Rituximab) | **AGGCATAGU**CCATAGAGCCCACCGCATC | 46 |
| EF1alpa_FW | EF1alpa | **ACTATGCCU**GTGAGGCTCCGGTGCCCG | 47 |
| EF1alpa_RV | EF1alpa | **ACCAGCGCU**TCACGACACCTGAAATGGAAGAA | 48 |
| NaSP-HC-Rituxi_FW | Native SP_HC-Rituximab | **AGTGCGAU**ATGGGCTGGTCCTGCATCA | 49 |
| HC-Rituxi_RV | Native SP_HC-Rituximab | **AGTCTTAAU**TCACTTGCCGGGGCTCAG | 50 |
| HC-Rituxi-FW | SPX_HC-Rituximab | **AGCAGCCU**GGCGCCGAG | 51 |
| SP1-HC-Ritux-RV | CMV_SP1 | AGGCTGCUGCAGCTGCACTGCAGTAGCACCTGC TGTGAG | 52 |
| SP2-HC-Ritux-RV | CMV_SP2 | AGGCTGCUGCAGCTGCACTCCAGCTACTGCTCCC AGCA | 53 |
| SP3-HC-Ritux-RV | CMV_SP3 | AGGCTGCU GCAGCTGCACTCCCAGGACCATGCC ATT | 54 |
| SP4-HC-Ritux-RV | CMV_SP4 | AGGCTGCUGCAGCTGCACTCCAGCCACCAGGCCT G | 55 |
| SP5-HC-Ritux-RV | CMV_SP5 | AGGCTGCUGCAGCTGCACCGCCCTGCAAGGCTG GC | 56 |
| SP6-HC-Ritux-RV | CMV_SP6 | AGGCTGCUGCAGCTGCACGTGCAGGTTGACTAG GTGGGTC | 57 |
| SP7-HC-Ritux-RV | CMV_SP7 | AGGCTGCUGCAGCTGCACTCCTCTGACAGAAAT GGAACAAA | 58 |
| SP8-HC-Ritux-RV | CMV_SP8 | AGGCTGCUGCAGCTGCACGGCCAGGCATCCTGC CA | 59 |
| SP9-HC-Ritux-RV | CMV_SP9 | AGGCTGCUGCAGCTGCACTGCCAGGCTCATTTT CAATAGA | 60 |
| SP10-HC-Ritux-RV | CMV_SP10 | AGGCTGCUGCAGCTGCACAGCAGAGCTGCAGCC TAGAAC | 61 |
| SP11-HC-Ritux-RV | CMV_SP11 | AGGCTGCUGCAGCTGCACGGCCAAGGCTGTTCC CAA | 62 |
| SP12-HC-Ritux-RV | CMV_SP12 | AGGCTGCUGCAGCTGCACTCCCAGACAGCAGGT AGACCA | 63 |
| SP13-HC-Ritux-RV | CMV_SP13 | AGGCTGCUGCAGCTGCACTGAGAGCGCTGGATC ACAGA | 64 |
| SYN1-HC-Ritux-RV | CMV_SYN1 | AGGCTGCU GCAGCTGCACGGCCCACACCATGGG CC | 65 |
| SYN2-HC-Ritux-RV | CMV_SYN2 | AGGCTGCUGCAGCTGCACGCCCCTGGCGGGGC CC | 66 |
| SP-IFNy-HC-Ritux-RV | CMV_SPIFNy | AGGCTGCUGCAGCTGCACGCCAAGAGAACCCAA AACGA | 67 |
| SP1-LC-Ritux_FW | SP1_LC-Rituximab | AGCGCTGGUATGAAATTCCTTCTGCTGGTCACT CTGCTCACAGCAGGTGCTACTGCAATTGTGCTG TCTCAGTCCCCC | 68 |
| SP2-LC-Ritux_FW | SP2_LC-Rituximab | AGCGCTGGUATGCAGATGCTGCAGCTGTGGACA CTCGCAGTGCTGCTGGGAGCAGTAGCTGGAATT GTGCTGTCTCAGTCCCCC | 69 |
| SP3-LC-Ritux_FW | SP3_LC-Rituximab | AGCGCTGGUATGAAGATTCTCCTGTTGTGCGTG GGGCTGCTGCTGACCTGGGACAATGGCATGGTC CTGGGAATTGTGCTGTCTCAGTCCCCC | 70 |
| SP4-LC-Ritux_FW | SP4_LC-Rituximab | AGCGCTGGUATGTGGACCCTGGTAAGCTGGCTG GCCTTCATGGCAGGCCTGGTGGCTGGAATTGTG CTGTCTCAGTCCCCC | 71 |
| SP5-LC-Ritux_FW | SP5_LC-Rituximab | AGCGCTGGUATGTCCTGCAGATTCGTGTTCCAG CTCCTGGGGCTCTGGCTGCTGCTGAGCCAGCCTT GCAGGGCGATTGTGCTGTCTCAGTCCCCC | 72 |
| SP6-LC-Ritux_FW | SP6_LC-Rituximab | AGCGCTGGUATGACCTCCCAGATTCGCCAGAAT TATTCCACCGAAGTGGAAGCTGTGGTGACCCAC CTAGTCAACCTGCACATTGTGCTGTCTCAGTCC CCC | 73 |
| SP7-LC-Ritux_FW | SP7_LC-Rituximab | AGCGCTGGUATGGCATTCCGTGCAATTTGTGTA TTGGTTGGAGTATTTATTTGTTCCATTTCTGTC AGAGGAATTGTGCTGTCTCAGTCCCCC | 74 |
| SP8-LC-Ritux_FW | SP8_LC-Rituximab | AGCGCTGGUATGAAGGCTCTGTGGGCCGTGCTG GTGGTCACATTGCTGGCAGGATGCCTGGCCATT GTGCTGTCTCAGTCCCCC | 75 |
| SP9-LC-Ritux_FW | SP9_LC-Rituximab | AGCGCTGGUATGAAGGGCCTGATCCTGTTCCTG TCTCTATTGAAAATGAGCCTGGCAATTGTGCTG TCTCAGTCCCCC | 76 |
| SP10-LC-Ritux_FW | SP10_LC-Rituximab | AGCGCTGGUATGAGACCCTGGCCGTCCCTGGTC CTGGCACTCCTGGTTCTAGGCTGCAGCTCTGCT ATTGTGCTGTCTCAGTCCCCC | 77 |
| SP11-LC-Ritux_FW | SP11_LC-Rituximab | AGCGCTGGUATGAATCTTTTACTCCTCCTGGCC ACCCTGTGCTTGGGAACAGCCTTGGCCATTGTG CTGTCTCAGTCCCCC | 78 |
| SP12-LC-Ritux_FW | SP12_LC-Rituximab | AGCGCTGGUATGGCAAGGCTCTGTGCTTTCCTG TTGGCCCTGGTGGTGATGAGCTACTGGTCTACC TGCTGTCTGGGAATTGTGCTGTCTCAGTCCCCC | 79 |
| SP13-LC-Ritux_FW | SP13_LC-Rituximab | AGCGCTGGUATGAAGCTCTGTGTGTCTGCCCTC TCTCTCCTCCTGTTCGTGGCTGCCTTCTGTGATC CAGCGCTCTCAATTGTGCTGTCTCAGTCCCCC | 80 |
| SYN1-LC-Ritux_FW | SYN1_LC-Rituximab | AGCGCTGGUATGTGGTGGAGGCTGTGGTGGCTG CTGCTGCTGCTGCTGCTGCTGTGGCCCATGGTG TGGGCCATTGTGCTGTCTCAGTCCCCC | 81 |
| SYN2-LC-Ritux_FW | SYN2_LC-Rituximab | AGCGCTGGUATGAGGCCCACCTGGGCCTGGTGG CTGTTCCTGGTGCTGCTGCTGGCCCTGTGGGCCC CCGCCAGGGGCATTGTGCTGTCTCAGTCCCCC | 82 |
| IFNy-LC-Ritux_FW | SPIFNy_LC-Rituximab | AGCGCTGGUATGAAATATACAAGTTATATCTTG GCTTTTCAGCTCTGCATCGTTTTGGGTTCTCTT GGCATTGTGCTGTCTCAGTCCCCC | 83 |
| NaSP-LC-Ritux FW | Native SP_LC-Rituximab | AGCGCTGGUATGGGCTGGTCCTGCATC | 84 |
| LC-Ritux RV | SPX_LC-Rituximab | AGACTGTGUTCAGCACTCGCCCCGGTTGAAA | 85 |
| Vector_Mam-RV | Backbone | AGCGACGUGAGTCGAATAAGGGCGACACC | 86 |
| Vector_Mam-FW | Backbone | ATTAAGCUAGTGAGTCGAATAAGGGCGACA | 97 |

**Table 5. Vectors used as PCR templates**

| **Vector name** | **Elements** | **Source** |
|---|---|---|
| pBudCE4.1 | EF-1a, light chain Rituximab, heavy chain Rituximab | Life Technologies |
| pcDNA3.1(+) | pCMV, BGH pA, pSV40-NeoR-SV40 pA, pSV40-HygR-SV40 pA | Life Technologies |
| pU0002 | AmpR, pUC19 replication origin | Lund et al., 2014 |

### 3.2 Cell cultivation

CHO-S suspension cells obtained from Life Technologies were maintained in CD-CHO medium (Gibco, Life Technologies) supplemented with 8 mM L-Glutamine (Gibco, Life Technologies) and 1:500 Anti-Clumping Agent (Gibco, Life Technologies). CHO-S cells were expanded in Erlenmeyer cell culture flasks (Corning, Sigma-Aldrich) in a humidified incubator at 37°C with 5 % CO₂.

### 3.3 Transfection and cell counting: Signal peptide- Rituximab constructs

Experiments were performed in pre-sterilized polystyrene 96-square System Duetz half-deep well-microplates (CR1496c, Enzyscreen, Haarlem, Netherlands) capped with autoclaved low-evaporation sandwich Duetz covers (CR1296a, Enzyscreen). Plates were incubated in a humidified S41i incubator shaker (New Brunswick, Eppendorf, Hamburg, Germany) at the following conditions: 37°C, 5% CO₂, 325 rpm, 25 mm orbit. 250 µL 1x10⁶ cells/mL CHO-S cells were transfected using FreeStyleTM MAX reagent as previously described [Hansen HG et al., 2015]. The day after transfection, anti-clumping agent was added to all wells reaching a final concentration of 2 µL/mL. VCD and viability were measured every day on the Celigo Imaging Cell Cytometer using Hoechst-33342 and propidium iodide as previously described [42]. Transfection efficiency was based on green fluorescence of cells transfected with pmaxGFP (Lonza Group AG, Basel, Switzerland). Transfection efficiency was determined at previously described [Hansen HG et al., 2015]. Supernatant samples were obtained three days post-transfection by centrifugation (500g, RT, 5 min) and supernatants were stored at -80°C.

### 3.4 Determination of Rituximab titer

Rituximab titer was determined in 200 µL three-fold diluted supernatants using an Octet RED96 (ForteBio, Pall, Menlo Park, CA, USA) essentially as previously described [Grav LM et al., 2015], but with an increased shaking speed of 1000 rpm.

### 3.5 Calculation of productivity parameters

Integral of viable cell density (IVCD) and daily specific production rate were calculated as described elsewhere [45]. In short, the IVCD was calculated as the sum of the area under the curve for viable cell density. The area was calculated - for each interval between samples - by multiplication of the elapsed time with the average cell density in the interval. The daily specific production rate was calculated as the increase in product concentration divided by the IVCD since last measurement.

### 3.6 Statistical analysis

Data (fold-changes) were tested for normality by D'Augustino-Pearson omnibus test using GraphPad Prism software (version 6.05 for Windows, GraphPad Software, La Jolla, CA, USA). Data (non-transformed and log2-transformed) in some groups did not pass the normality test, so statistical significance (p≤0.05) was assessed by performing the Mann-Whitney nonparametric test (two-tailed) on non-transformed fold-changes using GraphPad Prism software. Outliers were assessed by performing Grubb's test (p≤0.05) using GraphPad Prism software.

### 3.7 Results:

Higher specific and volumetric productivity was observed for CHO cells expressing Rituximab IgG antibody heavy chain (HL) and light chain (LC) having the signal peptides SP1-SP5, SP7, and SP11, as compared to CHO cells compared to cells expressing Rituximab IgG antibody (heavy and light chains) having the signal peptide SP_IFNγ or either of the synthetic signal peptides SYN1 and SYN2 (Figure 3a-b). In respect of CHO cells expressing Rituximab IgG antibody (heavy and light chains) having the signal peptides SP6, the IgG titer was below the detection limit for SP6, despite having the correct vector sequence.

IVCD was increased for SP8, SP9, SP12 and SYN2 compared to SP_IFNγ (Figure 4c). For these four signal peptide constructs we observed a correlation between IVCD and titer in contrast to no correlation for signal peptide constructs with no increase in IVCD (SP1, SP2, SP4 and SP_IFNγ) (Figure 4c). Since low transfection efficiency gives rise to faster growth (our unpublished observations), we speculate that the faster growth for the clones comprising the SP8, SP9, SP12 and SYN2 signal peptides was due to experimental variations leading to a relatively low transfection efficiency.

### Example 4 In silico re-evaluation of signal peptides fused to Rituximab IgG antibody

All signal peptides fused to Rituximab HC and LC were re-evaluated to examine if prediction could be achieved.

According to both *in silico* programs SignalP and PredSi, the tested peptide SP6 did not contain a signal peptide; which was consistent with the failure of CHO cells comprising the vector encoding the SP6- Rituximab HC and LC fusion to express detectable levels of Rituximab IgG.

Re-evaluation of all other tested signal peptides by SignalP confirmed the original prediction that they were signal peptides.

### Example 5 In vivo testing of native CHO signal peptides secretion rate of secreted alkaline phosphatase (SEAP)

In this example, the signal peptides are tested with a commonly used mammalian secretion reporter protein, secreted alkaline phosphatase (SEAP).

### 5.1 Vector design and construction

Expression vectors were constructed, as described in Example 3, comprising a nucleotide coding sequence for each of the 13 selected native CHO signal peptides fused to the coding sequence for mature SEAP polypeptide (SEQ ID No.:88), whereby the native SEAP signal peptide (present in the SEAP precursor polypeptide [SEQ ID No.:90] was substituted with each of the 13 selected native CHO signal peptides. For comparison, expression plasmids comprising the coding sequences for two synthetic signal peptides (SYN1 and SYN2) and the signal peptide from human interferon-gamma, IFNγ (SP_IFNγ) were each similarly constructed for expression of SEAP (Table 3). These reference signal peptides were chosen as comparators since they have been used (or are commonly used) in both commercial protein production and gene therapy research. Expression vectors comprising each of the constructs were individually transformed into CHO-S cells to produce transformed CHO-S cell lines. The rate of protein secretion facilitated by the signal sequences was analyzed by use of a high-throughput screening platform of transient expression in CHO-S cells of each transformed cell line.

### 5.2 Determination of SEAP titer

The concentration of secreted SEAP in the supernatant from each transformed cell line culture was determined by a standard colormetric assay [Durocher et al, 2000]. Samples were taken and heat-treated at 65 deg C for 30 min. 50 µl of used culture medium was transferred to a new 96-well plate and mixed with 50 µl SEAP assay solution (20 nM para-nitrophenyl phosphate (pNPP), 1 mM MgCl2, 1 M diethanolamine, pH adjusted to 9.8). Samples are measured as dilution rows 1, 1:2, 1:4, 1:8, 1:16 to ensure getting enough measurements and avoiding overload. Absorbance is measured at 410 nm at 1 min intervals to determine pNPP hydrolysis rates. EDTA is added to a final concentration of 10 mM to stop the reaction.

### Example 6 In vivo testing of native CHO signal peptides secretion rate of erythropoetin (EPO)

In this example, the signal peptides are tested with a commercial pharmaceutical protein of the hormone type erythropoetin (EPO).

### 6.1 Vector design and construction

Expression vectors were constructed, as described in Example 3, comprising a nucleotide coding sequence for each of the 13 selected native CHO signal peptides fused to the coding sequence for mature EPO (SEQ ID NO:92)whereby the native EPO signal peptide was substituted with each of the 13 selected native CHO signal peptides. For comparison, expression plasmids comprising the coding sequences for two synthetic signal peptides (SYN1 and SYN2) and the signal peptide from human interferon-gamma, IFNγ (SP_IFNγ) were each similarly constructed for expression of SEAP (Table 3). These reference signal peptides were chosen as comparators since they have been used (or are commonly used) in both commercial protein production and gene therapy research. Expression vectors comprising each of the constructs were individually transformed into CHO-S cells to produce transformed CHO-S cell lines. The rate of protein secretion facilitated by the signal sequences was analyzed by use of a high-throughput screening platform of transient expression in CHO-S cells of each transformed cell line.

### 6.2 Determination of EPO titer

EPO titer was determined in 200 µL three-fold diluted supernatants derived from each transformed cell line culture using an Octet RED96 (ForteBio, Pall, Menlo Park, CA, USA) essentially as previously described [Grav LM et al., 2015], but with an increased shaking speed of 1000 rpm.

### Example 7 In vivo testing of native CHO signal peptides secretion rate of the receptor-antibody fusion protein Enbrel/Etanercept/Etacept/Benepali/ Brenzys

In this example, the signal peptides are tested with a pharmaceutical tumor necrosis factor (TNF) inhibitor fusion protein of the TNF and the constant region of human IgG1 (Enbrel).

### 7.1 Vector design and construction

Expression vectors were constructed, as described in Example 3, 5 and 6, comprising a nucleotide coding sequence for each of the 13 selected native CHO signal peptides fused to the coding sequence for mature Enbrel (SEQ ID No.:94) whereby the native Enbrel signal peptide (present in the Enbrel precursor polypeptide [SEQ ID No.:96]was substituted with each of the 13 selected native CHO signal peptides. For comparison, expression plasmids comprising the coding sequences for two synthetic signal peptides (SYN1 and SYN2) and the signal peptide from human interferon-gamma, IFNγ (SP_IFNγ) were each similarly constructed for expression of SEAP (Table 3). These reference signal peptides were chosen as comparators since they have been used (or are commonly used) in both commercial protein production and gene therapy research. Expression vectors comprising each of the constructs were individually transformed into CHO-S cells to produce transformed CHO-S cell lines.The rate of protein secretion facilitated by the signal sequences was analyzed by use of a high-throughput screening platform of transient expression in CHO-S cells of each transformed cell line.

### 7.2 Determination of Enbrel titer

Enbrel titer was determined in 200 µL three-fold diluted supernatants derived from each transformed cell line culture using an Octet RED96 (ForteBio, Pall, Menlo Park, CA, USA) essentially as previously described [Grav LM et al., 2015], but with an increased shaking speed of 1000 rpm.

### References

Barash S, Wang W, Shi Y: Human secretory signal peptide description by hidden Markov model and generation of a strong artificial signal peptide for secreted protein expression. Biochem Biophys Res Commun 2002, 294:835-842.
Briesemeister S, Rahnenführer J, Kohlbacher O: YLoc-an interpretable web server for predicting subcellular localization. Nucleic Acids Res 2010, 38(suppl 2):W497-W502.
Durocher et al., 2000, Analytical Chemistry 284, 316-326.
Finn RD, Clements J, Eddy SR: HMMER web server: interactive sequence similarity searching. Nucleic Acids Res 2011, 39(suppl 2):W29-W37.
Garg P, Sharma V, Chaudhari P, Roy N: SubCellProt: Predicting Protein Subcellular Localization Using Machine Learning Approaches. In Silico Biol 2009, 9:35-44. Gasteiger E, Gattiker A, Hoogland C, Ivanyi I, Appel RD, Bairoch A: ExPASy: The proteomics server for in-depth protein knowledge and analysis. Nucleic Acids Res 2003, 31:3784-3788.
Grav LM, Lee JS, Gerling S, Kallehauge TB, Hansen AH, Kol S, Lee GM, Pedersen LE, Kildegaard HF: One-step generation of triple knockout CHO cell lines using CRISPR/Cas9 and fluorescent enrichment. Biotechnol J 2015, 10:1446-1456.
Hansen HG, Nilsson CN, Lund AM, Kol S, Grav LM, Lundqvist M, Rockberg J, Lee GM, Andersen MR, Kildegaard HF: Versatile microscale screening platform for improving recombinant protein productivity in Chinese hamster ovary cells. Sci Rep 2015, 5:18016.
von Heijne G: Signal sequences. The limits of variation. J Mol Biol 1985, 184:99-105.
von Heijne G: How signal sequences maintain cleavage specificity. J Mol Biol 1984, 173:243-251.
Hiller K, Grote A, Scheer M, Munch R, Jahn D: PrediSi: prediction of signal peptides and their cleavage positions. Nucleic Acids Res 2004, 32(suppl 2):W375-W379.
Käll L, Krogh A, Sonnhammer ELL: A Combined Transmembrane Topology and Signal Peptide Prediction Method. J Mol Biol 2004, 338:1027-1036.
Krogh A, Larsson B, von Heijne G, Sonnhammer ELL: Predicting transmembrane protein topology with a hidden markov model: application to complete genomes. J Mol Biol 2001, 305:567-580.
Kyte J, Doolittle RF: A simple method for displaying the hydropathic character of a protein. J Mol Biol 1982, 157:105-132.
Lund AM, Kildegaard HF, Petersen MBK, Rank J, Hansen BG, Andersen MR, Mortensen UH: A Versatile System for USER Cloning-Based Assembly of Expression Vectors for Mammalian Cell Engineering. PLoS ONE 2014, 9:e96693.
Nørholm MH: A mutant Pfu DNA polymerase designed for advanced uracil-excision DNA engineering. BMC Biotechnol 2010, 10:21.
Owczarzy R, Tataurov AV, Wu Y, Manthey JA, McQuisten KA, Almabrazi HG, Pedersen KF, Lin Y, Garretson J, McEntaggart NO, Sailor CA, Dawson RB, Peek AS: IDT SciTools: a suite for analysis and design of nucleic acid oligomers. Nucleic Acids Res 2008, 36(suppl 2):W163-W169.
Petersen TN, Brunak S, von Heijne G, Nielsen H: SignalP 4.0: discriminating signal peptides from transmembrane regions. Nat Methods 2011, 8:785-786.
Walsh G: Biopharmaceutical benchmarks 2014. Nat Biotechnol 2014, 32:992-1000. Xu X, Nagarajan H, Lewis NE, Pan S, Cai Z, Liu X, Chen W, Xie M, Wang W, Hammond S, Andersen MR, Neff N, Passarelli B, Koh W, Fan HC, Wang J, Gui Y, Lee KH, Betenbaugh MJ, Quake SR, Famili I, Palsson BO, Wang J: The genomic sequence of the Chinese hamster ovary (CHO)-K1 cell line. Nat Biotechnol 2011, 29:735-741.

## Claims

1. A recombinant gene for expression of a precursor polypeptide, the gene comprising an in-frame fusion of:
a. a first nucleic acid molecule encoding a signal peptide, and
b. a second nucleic acid molecule encoding a recombinant polypeptide, wherein the fused first and second nucleic acid molecules encode the precursor polypeptide, and wherein the signal peptide is between 15 and 25 amino acid residues in length and comprises an amino acid sequence selected from the group consisting of: SP1 [SEQ ID No.:2], SP2 [SEQ ID No.:4], SP3 [SEQ ID No.:6], SP4 [SEQ ID No.:8], SP5 [SEQ ID No.: 10], SP7 [SEQ ID No.:14] and SP11 [SEQ ID No.:22].

2. The recombinant gene for expression of a precursor polypeptide according to claim 1, wherein the recombinant polypeptide is selected from the group consisting of a hormone, enzyme, immunoglobulin heavy chain, immunoglobulin light chain, whole immunoglobulin, fragment antigen-binding (Fab) fragment, Fc fragment, single-chain variable fragment (scFv), cytokine, interleukin, complement factor, coagulation factor, an extracellular matrix protein, and a soluble receptor.

3. The recombinant gene for expression of a precursor polypeptide according to claim 1 or 2, wherein the gene is operably linked to a promoter DNA molecule.

4. The recombinant gene for expression of a precursor polypeptide according to any one of claims 1 to 3, wherein the gene is operably linked to a terminator DNA molecule.

5. A vector comprising the recombinant gene for expression of a precursor polypeptide according to any one of claims 1 to 4.

6. A genetically modified CHO cell transformed with the recombinant gene for expression of a precursor polypeptide according to any one of claims 1 to 4.

7. The genetically modified CHO cell according to claim 6, wherein said cell is derived from a CHO cell line selected from the group consisting of: CHO-K1, CHO-S or GS-CHO, CHO-Lec1, CHO-Lec10, CHO-Lec13, CHO Pro-5, CHO DX B11 and CHO DG44 cells.

8. A method for producing a recombinant protein *in vivo* comprising:
a. providing one or more genetically modified CHO cells transformed with a recombinant gene according to claim 6 or 7;
b. introducing said one or more genetically modified CHO cells into a culture medium to produce a culture;
c. incubating the culture;
d. recovering the produced recombinant polypeptide from the culture, and optionally
e. purifying the recombinant polypeptide recovered.

9. A method for producing a recombinant polypeptide *in vivo* according to claim 8, wherein the cell-specific productivity of recombinant polypeptide by the CHO cell is enhanced in comparison to a CHO cell wherein the encoded signal peptide is a cognate native signal peptide of the recombinant polypeptide or a non-CHO-derived signal peptide.

10. Use of a signal peptide to enhance the production of a recombinant polypeptide in a genetically modified CHO cell, wherein the signal peptide is fused to the recombinant polypeptide, and wherein the signal peptide is between 15 and 25 amino acid residues in length and comprises an amino acid sequence selected from the group consisting of: SP1 [SEQ ID No.:2], SP2 [SEQ ID No.:4], SP3 [SEQ ID No.:6], SP4 [SEQ ID No.:8], SP5 [SEQ ID No..:10], SP7 [SEQ ID No.:14] and SP11 [SEQ ID No.:22].

11. Use of a signal peptide to enhance the production of a recombinant polypeptide in a genetically modified CHO cell according to claim 10, wherein the recombinant polypeptide is selected from the group consisting of a hormone, enzyme, immunoglobulin heavy chain, immunoglobulin light chain, whole immunoglobulin, fragment antigen-binding (Fab) fragment, Fc fragment, single-chain variable fragment (scFv), cytokine, interleukin, complement factor, coagulation factor, an extracellular matrix protein, and a soluble receptor.


SEQUENCE LISTING

<110>  Danmarks Tekniske Universitet

<120>  Native Chinese Hamster Ovary cell secretion signal peptides for
       production of recombinant polypeptides

<130>  P1982EP00

<160>  177

<170>  PatentIn version 3.5

<210>  1
<211>  48
<212>  DNA
<213>  Chinese hamster

<220>
<221>  CDS
<222>  (1)..(48)
<223>  CHO Signal peptide 1 coding sequence

<400>  1
atg aaa ttc ctt ctg ctg gtc act ctg ctc aca gca ggt gct act gca          48
Met Lys Phe Leu Leu Leu Val Thr Leu Leu Thr Ala Gly Ala Thr Ala
1               5                   10                  15

<210>  2
<211>  16
<212>  PRT
<213>  Chinese hamster

<400>  2

Met Lys Phe Leu Leu Leu Val Thr Leu Leu Thr Ala Gly Ala Thr Ala
1               5                   10                  15

<210>  3
<211>  54
<212>  DNA
<213>  Chinese hamster

<220>
<221>  CDS
<222>  (1)..(54)
<223>  CHO Signal Peptide 2 coding sequence

<400>  3
atg cag atg ctg cag ctg tgg aca ctc gca gtg ctg ctg gga gca gta          48
Met Gln Met Leu Gln Leu Trp Thr Leu Ala Val Leu Leu Gly Ala Val
1               5                   10                  15

gct gga                                                                  54
Ala Gly

<210>  4

<211>  18
<212>  PRT
<213>  Chinese hamster

<400>  4

Met Gln Met Leu Gln Leu Trp Thr Leu Ala Val Leu Leu Gly Ala Val
1               5                   10                  15

Ala Gly

<210>  5
<211>  63
<212>  DNA
<213>  Chinese hamster

<220>
<221>  CDS
<222>  (1)..(63)
<223>  CHO Signal Peptide 3 coding sequence

<400>  5
atg aag att ctc ctg ttg tgc gtg ggg ctg ctg ctg acc tgg gac aat        48
Met Lys Ile Leu Leu Leu Cys Val Gly Leu Leu Leu Thr Trp Asp Asn
1               5                   10                  15

ggc atg gtc ctg gga                                                    63
Gly Met Val Leu Gly
            20

<210>  6
<211>  21
<212>  PRT
<213>  Chinese hamster

<400>  6

Met Lys Ile Leu Leu Leu Cys Val Gly Leu Leu Leu Thr Trp Asp Asn
1               5                   10                  15

Gly Met Val Leu Gly
            20

<210>  7
<211>  51
<212>  DNA
<213>  Chinese hamster

<220>
<221>  CDS
<222>  (1)..(51)
<223>  CHO Signal Peptide 4 coding sequence

<400>  7
atg tgg acc ctg gta agc tgg ctg gcc ttc atg gca ggc ctg gtg gct        48

Met Trp Thr Leu Val Ser Trp Leu Ala Phe Met Ala Gly Leu Val Ala
1               5                   10                  15

gga                                                                   51
Gly

<210>  8
<211>  17
<212>  PRT
<213>  Chinese hamster

<400>  8

Met Trp Thr Leu Val Ser Trp Leu Ala Phe Met Ala Gly Leu Val Ala
1               5                   10                  15

Gly

<210>  9
<211>  66
<212>  DNA
<213>  Chinese hamster

<220>
<221>  CDS
<222>  (1)..(66)
<223>  CHO Signal Peptide 5 coding sequence

<400>  9
atg tcc tgc aga ttc gtg ttc cag ctc ctg ggg ctc tgg ctg ctg ctg       48
Met Ser Cys Arg Phe Val Phe Gln Leu Leu Gly Leu Trp Leu Leu Leu
1               5                   10                  15

agc cag cct tgc agg gcg                                               66
Ser Gln Pro Cys Arg Ala
            20

<210>  10
<211>  22
<212>  PRT
<213>  Chinese hamster

<400>  10

Met Ser Cys Arg Phe Val Phe Gln Leu Leu Gly Leu Trp Leu Leu Leu
1               5                   10                  15

Ser Gln Pro Cys Arg Ala
            20

<210>  11
<211>  72
<212>  DNA
<213>  Chinese hamster

<220>
<221>  CDS
<222>  (1)..(72)
<223>  CHO Signal Peptide 6 coding sequence

<400>  11
atg acc tcc cag att cgc cag aat tat tcc acc gaa gtg gaa gct gtg        48
Met Thr Ser Gln Ile Arg Gln Asn Tyr Ser Thr Glu Val Glu Ala Val
1               5                   10                  15

gtg acc cac cta gtc aac ctg cac                                        72
Val Thr His Leu Val Asn Leu His
            20


<210>  12
<211>  24
<212>  PRT
<213>  Chinese hamster

<400>  12

Met Thr Ser Gln Ile Arg Gln Asn Tyr Ser Thr Glu Val Glu Ala Val
1               5                   10                  15


Val Thr His Leu Val Asn Leu His
            20


<210>  13
<211>  63
<212>  DNA
<213>  Chinese hamster


<220>
<221>  CDS
<222>  (1)..(63)
<223>  CHO Signal Peptide 7 coding sequence

<400>  13
atg gca ttc cgt gca att tgt gta ttg gtt gga gta ttt att tgt tcc        48
Met Ala Phe Arg Ala Ile Cys Val Leu Val Gly Val Phe Ile Cys Ser
1               5                   10                  15

att tct gtc aga gga                                                    63
Ile Ser Val Arg Gly
            20


<210>  14
<211>  21
<212>  PRT
<213>  Chinese hamster

<400>  14

Met Ala Phe Arg Ala Ile Cys Val Leu Val Gly Val Phe Ile Cys Ser
1               5                   10                  15

Ile Ser Val Arg Gly
            20

<210>  15
<211>  54
<212>  DNA
<213>  Chinese hamster

<220>
<221>  CDS
<222>  (1)..(54)
<223>  CHO Signal Peptide 8 coding sequence

<400>  15
atg aag gct ctg tgg gcc gtg ctg gtg gtc aca ttg ctg gca gga tgc      48
Met Lys Ala Leu Trp Ala Val Leu Val Val Thr Leu Leu Ala Gly Cys
1               5                   10                  15

ctg gcc                                                              54
Leu Ala

<210>  16
<211>  18
<212>  PRT
<213>  Chinese hamster

<400>  16

Met Lys Ala Leu Trp Ala Val Leu Val Val Thr Leu Leu Ala Gly Cys
1               5                   10                  15

Leu Ala

<210>  17
<211>  48
<212>  DNA
<213>  Chinese hamster

<220>
<221>  CDS
<222>  (1)..(48)
<223>  CHO Signal Peptide 9 coding sequence

<400>  17
atg aag ggc ctg atc ctg ttc ctg tct cta ttg aaa atg agc ctg gca      48
Met Lys Gly Leu Ile Leu Phe Leu Ser Leu Leu Lys Met Ser Leu Ala
1               5                   10                  15

<210>  18
<211>  16
<212>  PRT
<213>  Chinese hamster

<400>  18

Met Lys Gly Leu Ile Leu Phe Leu Ser Leu Leu Lys Met Ser Leu Ala
1               5                   10                  15

<210>  19
<211>  57
<212>  DNA
<213>  Chinese hamster

<220>
<221>  CDS
<222>  (1)..(57)
<223>  CHO Signal Peptide 10 coding sequence

<400>  19
atg aga ccc tgg ccg tcc ctg gtc ctg gca ctc ctg gtt cta ggc tgc      48
Met Arg Pro Trp Pro Ser Leu Val Leu Ala Leu Leu Val Leu Gly Cys
1               5                   10                  15

agc tct gct                                                          57
Ser Ser Ala

<210>  20
<211>  19
<212>  PRT
<213>  Chinese hamster

<400>  20

Met Arg Pro Trp Pro Ser Leu Val Leu Ala Leu Leu Val Leu Gly Cys
1               5                   10                  15

Ser Ser Ala

<210>  21
<211>  51
<212>  DNA
<213>  Chinese hamster

<220>
<221>  CDS
<222>  (1)..(51)
<223>  CHO Signal Peptide 11 coding sequence

<400>  21
atg aat ctt tta ctc ctc ctg gcc acc ctg tgc ttg gga aca gcc ttg      48
Met Asn Leu Leu Leu Leu Leu Ala Thr Leu Cys Leu Gly Thr Ala Leu
1               5                   10                  15

gcc                                                                  51
Ala

<210>  22
<211>  17
<212>  PRT
<213>  Chinese hamster

<400>  22

Met Asn Leu Leu Leu Leu Leu Ala Thr Leu Cys Leu Gly Thr Ala Leu
1               5                   10                  15

Ala

<210>  23
<211>  69
<212>  DNA
<213>  Chinese hamster

<220>
<221>  CDS
<222>  (1)..(69)
<223>  CHO Signal Peptide 12 coding sequence

<400>  23
atg gca agg ctc tgt gct ttc ctg ttg gcc ctg gtg gtg atg agc tac        48
Met Ala Arg Leu Cys Ala Phe Leu Leu Ala Leu Val Val Met Ser Tyr
1               5                   10                  15

tgg tct acc tgc tgt ctg gga                                            69
Trp Ser Thr Cys Cys Leu Gly
            20

<210>  24
<211>  23
<212>  PRT
<213>  Chinese hamster

<400>  24

Met Ala Arg Leu Cys Ala Phe Leu Leu Ala Leu Val Val Met Ser Tyr
1               5                   10                  15

Trp Ser Thr Cys Cys Leu Gly
            20

<210>  25
<211>  69
<212>  DNA
<213>  Chinese hamster

<220>
<221>  CDS
<222>  (1)..(69)
<223>  CHO Signal Peptide 13 coding sequence

<400>  25

atg aag ctc tgt gtg tct gcc ctc tct ctc ctc ctg ttc gtg gct gcc          48
Met Lys Leu Cys Val Ser Ala Leu Ser Leu Leu Leu Phe Val Ala Ala
1               5                   10                  15

ttc tgt gat cca gcg ctc tca                                              69
Phe Cys Asp Pro Ala Leu Ser
            20

<210>  26
<211>  23
<212>  PRT
<213>  Chinese hamster

<400>  26

Met Lys Leu Cys Val Ser Ala Leu Ser Leu Leu Leu Phe Val Ala Ala
1               5                   10                  15

Phe Cys Asp Pro Ala Leu Ser
            20

<210>  27
<211>  1356
<212>  DNA
<213>  Mus and Homo sapiens

<220>
<221>  CDS
<222>  (1)..(1356)
<223>  Mature Retuximab Heavy Chain coding sequence_mouse/human chimera

<400>  27
cag gtg cag ctg cag cag cct ggc gcc gag ctc gtg aaa cct ggc gcc          48
Gln Val Gln Leu Gln Gln Pro Gly Ala Glu Leu Val Lys Pro Gly Ala
1               5                   10                  15

tcc gtg aag atg tcc tgc aag gcc tcc ggc tac acc ttc acc agc tac          96
Ser Val Lys Met Ser Cys Lys Ala Ser Gly Tyr Thr Phe Thr Ser Tyr
            20                  25                  30

aac atg cac tgg gtc aag cag acc ccc ggc aga ggc ctg gaa tgg atc         144
Asn Met His Trp Val Lys Gln Thr Pro Gly Arg Gly Leu Glu Trp Ile
        35                  40                  45

ggc gct atc tac ccc ggc aac ggc gac acc tcc tac aac cag aag ttc         192
Gly Ala Ile Tyr Pro Gly Asn Gly Asp Thr Ser Tyr Asn Gln Lys Phe
    50                  55                  60

aag ggc aag gcc acc ctg acc gcc gac aag tcc tct tcc acc gcc tac         240
Lys Gly Lys Ala Thr Leu Thr Ala Asp Lys Ser Ser Ser Thr Ala Tyr
65                  70                  75                  80

atg cag ctg tcc tcc ctg acc tcc gag gac tcc gcc gtg tac tac tgc         288
Met Gln Leu Ser Ser Leu Thr Ser Glu Asp Ser Ala Val Tyr Tyr Cys
                85                  90                  95

gcc cgg tct acc tac tac ggc ggc gac tgg tac ttc aac gtg tgg ggc         336
Ala Arg Ser Thr Tyr Tyr Gly Gly Asp Trp Tyr Phe Asn Val Trp Gly
            100                 105                 110

gct ggc acc acc gtg acc gtg tct gct gct tct acc aag ggc ccc tcc 384
Ala Gly Thr Thr Val Thr Val Ser Ala Ala Ser Thr Lys Gly Pro Ser
        115                 120                 125

gtg ttc cct ctg gcc cct tcc agc aag tct acc tct ggc ggc aca gcc 432
Val Phe Pro Leu Ala Pro Ser Ser Lys Ser Thr Ser Gly Gly Thr Ala
    130                 135                 140

gct ctg ggc tgc ctc gtg aag gac tac ttc ccc gag ccc gtg aca gtg 480
Ala Leu Gly Cys Leu Val Lys Asp Tyr Phe Pro Glu Pro Val Thr Val
145                 150                 155                 160

tcc tgg aac tct ggc gct ctg acc agc gga gtg cac acc ttc cct gct 528
Ser Trp Asn Ser Gly Ala Leu Thr Ser Gly Val His Thr Phe Pro Ala
                165                 170                 175

gtg ctg cag tcc tcc ggc ctg tac tcc ctg tcc agc gtc gtg act gtg 576
Val Leu Gln Ser Ser Gly Leu Tyr Ser Leu Ser Ser Val Val Thr Val
            180                 185                 190

ccc tcc agc tct ctg ggc acc cag acc tac atc tgc aac gtg aac cac 624
Pro Ser Ser Ser Leu Gly Thr Gln Thr Tyr Ile Cys Asn Val Asn His
        195                 200                 205

aag ccc tcc aac acc aag gtg gac aag aag gtg gaa ccc aag tcc tgc 672
Lys Pro Ser Asn Thr Lys Val Asp Lys Lys Val Glu Pro Lys Ser Cys
    210                 215                 220

gac aag acc cac acc tgt ccc cct tgt cct gcc cct gaa ctg ctg ggc 720
Asp Lys Thr His Thr Cys Pro Pro Cys Pro Ala Pro Glu Leu Leu Gly
225                 230                 235                 240

gga ccc agc gtg ttc ctg ttc ccc cca aag ccc aag gat acc ctg atg 768
Gly Pro Ser Val Phe Leu Phe Pro Pro Lys Pro Lys Asp Thr Leu Met
                245                 250                 255

atc tcc cgg acc ccc gaa gtg acc tgc gtg gtg gtg gat gtg tcc cac 816
Ile Ser Arg Thr Pro Glu Val Thr Cys Val Val Val Asp Val Ser His
            260                 265                 270

gag gac cct gaa gtg aag ttc aat tgg tac gtg gac ggc gtg gaa gtg 864
Glu Asp Pro Glu Val Lys Phe Asn Trp Tyr Val Asp Gly Val Glu Val
        275                 280                 285

cac aac gcc aag acc aag cct aga gag gaa cag tac aac tcc acc tac 912
His Asn Ala Lys Thr Lys Pro Arg Glu Glu Gln Tyr Asn Ser Thr Tyr
    290                 295                 300

cgg gtg gtg tcc gtg ctg acc gtg ctg cac cag gat tgg ctg aac ggc 960
Arg Val Val Ser Val Leu Thr Val Leu His Gln Asp Trp Leu Asn Gly
305                 310                 315                 320

aaa gag tac aag tgc aag gtg tcc aac aag gcc ctg cct gcc ccc atc 1008
Lys Glu Tyr Lys Cys Lys Val Ser Asn Lys Ala Leu Pro Ala Pro Ile
                325                 330                 335

gaa aag acc atc tcc aag gcc aag ggc cag ccc cgg gaa ccc cag gtg 1056
Glu Lys Thr Ile Ser Lys Ala Lys Gly Gln Pro Arg Glu Pro Gln Val
            340                 345                 350

tac aca ctg ccc cct agc agg gac gag ctg acc aag aac cag gtg tcc 1104
Tyr Thr Leu Pro Pro Ser Arg Asp Glu Leu Thr Lys Asn Gln Val Ser

355                 360                 365

ctg aca tgc ctc gtg aaa ggc ttc tac ccc tcc gat atc gcc gtg gaa       1152
Leu Thr Cys Leu Val Lys Gly Phe Tyr Pro Ser Asp Ile Ala Val Glu
    370                 375                 380

tgg gag tcc aac ggc cag cct gag aac aac tac aag acc acc ccc cct       1200
Trp Glu Ser Asn Gly Gln Pro Glu Asn Asn Tyr Lys Thr Thr Pro Pro
385                 390                 395                 400

gtg ctg gac tcc gac ggc tca ttc ttc ctg tac agc aag ctg aca gtg       1248
Val Leu Asp Ser Asp Gly Ser Phe Phe Leu Tyr Ser Lys Leu Thr Val
                405                 410                 415

gac aag tcc cgg tgg cag cag ggc aac gtg ttc tcc tgc tcc gtg atg       1296
Asp Lys Ser Arg Trp Gln Gln Gly Asn Val Phe Ser Cys Ser Val Met
            420                 425                 430

cac gag gcc ctg cac aac cac tat acc cag aag tcc ctg tcc ctg agc       1344
His Glu Ala Leu His Asn His Tyr Thr Gln Lys Ser Leu Ser Leu Ser
        435                 440                 445

ccc ggc aag tga                                                       1356
Pro Gly Lys
    450


<210>  28
<211>  451
<212>  PRT
<213>  Mus and Homo sapiens

<400>  28

Gln Val Gln Leu Gln Gln Pro Gly Ala Glu Leu Val Lys Pro Gly Ala
1               5                   10                  15


Ser Val Lys Met Ser Cys Lys Ala Ser Gly Tyr Thr Phe Thr Ser Tyr
            20                  25                  30


Asn Met His Trp Val Lys Gln Thr Pro Gly Arg Gly Leu Glu Trp Ile
        35                  40                  45


Gly Ala Ile Tyr Pro Gly Asn Gly Asp Thr Ser Tyr Asn Gln Lys Phe
    50                  55                  60


Lys Gly Lys Ala Thr Leu Thr Ala Asp Lys Ser Ser Ser Thr Ala Tyr
65                  70                  75                  80


Met Gln Leu Ser Ser Leu Thr Ser Glu Asp Ser Ala Val Tyr Tyr Cys
                85                  90                  95


Ala Arg Ser Thr Tyr Tyr Gly Gly Asp Trp Tyr Phe Asn Val Trp Gly
            100                 105                 110


Ala Gly Thr Thr Val Thr Val Ser Ala Ala Ser Thr Lys Gly Pro Ser

        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  29
<211>  642
<212>  DNA
<213>  Mus and Homo Sapiens

<220>
<221>  CDS
<222>  (1)..(642)
<223>  Mature Retuximab Light Chain coding sequence_mouse/human chimera

<400>  29
cag att gtg ctg tct cag tcc ccc gcc atc ctg tct gct agc cct ggc        48
Gln Ile Val Leu Ser Gln Ser Pro Ala Ile Leu Ser Ala Ser Pro Gly
1               5                   10                  15

gag aaa gtg aca atg acc tgc cgg gcc tcc tcc tcc gtg tcc tac atc        96
Glu Lys Val Thr Met Thr Cys Arg Ala Ser Ser Ser Val Ser Tyr Ile
            20                  25                  30

cac tgg ttc cag cag aag ccc ggc tcc agc ccc aag cct tgg atc tac       144
His Trp Phe Gln Gln Lys Pro Gly Ser Ser Pro Lys Pro Trp Ile Tyr
        35                  40                  45

gcc acc tcc aac ctg gcc tct ggc gtg cca gtg cgg ttt tcc ggc tct       192
Ala Thr Ser Asn Leu Ala Ser Gly Val Pro Val Arg Phe Ser Gly Ser
    50                  55                  60

ggc tct ggc acc tcc tac tcc ctg acc atc tct cgg gtg gaa gcc gag       240
Gly Ser Gly Thr Ser Tyr Ser Leu Thr Ile Ser Arg Val Glu Ala Glu
65                  70                  75                  80

gat gcc gcc acc tac tac tgc cag cag tgg acc agc aac ccc ccc aca       288
Asp Ala Ala Thr Tyr Tyr Cys Gln Gln Trp Thr Ser Asn Pro Pro Thr
                85                  90                  95

ttt ggc gga ggc acc aag ctg gaa atc aag cgg acc gtg gcc gct ccc       336
Phe Gly Gly Gly Thr Lys Leu Glu Ile Lys Arg Thr Val Ala Ala Pro
            100                 105                 110

tcc gtg ttc atc ttc cca cct tcc gac gag cag ctg aag tcc ggc acc     384
Ser Val Phe Ile Phe Pro Pro Ser Asp Glu Gln Leu Lys Ser Gly Thr
        115                 120                 125

gct tct gtc gtg tgc ctg ctg aac aac ttc tac ccc cgc gag gcc aag     432
Ala Ser Val Val Cys Leu Leu Asn Asn Phe Tyr Pro Arg Glu Ala Lys
    130                 135                 140

gtg cag tgg aag gtg gac aac gcc ctg cag tcc ggc aac tcc cag gaa     480
Val Gln Trp Lys Val Asp Asn Ala Leu Gln Ser Gly Asn Ser Gln Glu
145                 150                 155                 160

tcc gtg acc gag cag gac tcc aag gac agc acc tac agc ctg tcc tcc     528
Ser Val Thr Glu Gln Asp Ser Lys Asp Ser Thr Tyr Ser Leu Ser Ser
                165                 170                 175

acc ctg acc ctg tcc aag gcc gac tac gag aag cac aag gtg tac gcc     576
Thr Leu Thr Leu Ser Lys Ala Asp Tyr Glu Lys His Lys Val Tyr Ala
            180                 185                 190

tgc gaa gtg acc cac cag ggc ctg tct agc ccc gtg acc aag tct ttc     624
Cys Glu Val Thr His Gln Gly Leu Ser Ser Pro Val Thr Lys Ser Phe
        195                 200                 205

aac cgg ggc gag tgc tga                                             642
Asn Arg Gly Glu Cys
    210


<210>  30
<211>  213
<212>  PRT
<213>  Mus and Homo Sapiens

<400>  30

Gln Ile Val Leu Ser Gln Ser Pro Ala Ile Leu Ser Ala Ser Pro Gly
1               5                   10                  15


Glu Lys Val Thr Met Thr Cys Arg Ala Ser Ser Ser Val Ser Tyr Ile
            20                  25                  30


His Trp Phe Gln Gln Lys Pro Gly Ser Ser Pro Lys Pro Trp Ile Tyr
        35                  40                  45


Ala Thr Ser Asn Leu Ala Ser Gly Val Pro Val Arg Phe Ser Gly Ser
    50                  55                  60


Gly Ser Gly Thr Ser Tyr Ser Leu Thr Ile Ser Arg Val Glu Ala Glu
65                  70                  75                  80


Asp Ala Ala Thr Tyr Tyr Cys Gln Gln Trp Thr Ser Asn Pro Pro Thr
                85                  90                  95


Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  31
<211>  654
<212>  DNA
<213>  Cytomegalovirus

<220>
<221>  promoter
<222>  (1)..(654)
<223>  Cytomegalovirus promoter

<400>  31
cgatgtacgg gccagatata cgcgttgaca ttgattattg actagttatt aatagtaatc      60

aattacgggg tcattagttc atagcccata tatggagttc cgcgttacat aacttacggt     120

aaatggcccg cctggctgac cgcccaacga cccccgccca ttgacgtcaa taatgacgta     180

tgttcccata gtaacgccaa tagggacttt ccattgacgt caatgggtgg agtatttacg     240

gtaaactgcc cacttggcag tacatcaagt gtatcatatg ccaagtacgc cccctattga     300

cgtcaatgac ggtaaatggc ccgcctggca ttatgcccag tacatgacct tatgggactt     360

tcctacttgg cagtacatct acgtattagt catcgctatt accatggtga tgcggttttg     420

gcagtacatc aatgggcgtg gatagcggtt tgactcacgg ggatttccaa gtctccaccc     480

cattgacgtc aatgggagtt tgttttggca ccaaaatcaa cgggactttc caaaatgtcg     540

taacaactcc gccccattga cgcaaatggg cggtaggcgt gtacggtggg aggtctatat     600

aagcagagct ctctggctaa ctagagaacc cactgcttac tggcttatcg aaat           654

<210>  32
<211>  350
<212>  DNA
<213>  Simian virus 40


<220>
<221>  promoter
<222>  (1)..(350)
<223>  Simian virus 40 early promoter

<400>  32
cagctgtgga atgtgtgtca gttagggtgt ggaaagtccc caggctcccc agcaggcaga      60

agtatgcaaa gcatgcatct caattagtca gcaaccaggc tccccagcag gcagaagtat     120

gcaaagcatg catctcaatt agtcagcaac catagtcccg cccctaactc cgcccatccc     180

gcccctaact ccgcccagtt ccgcccattc tccgccccat ggctgactaa ttttttttat     240

ttatgcagag gccgaggccg cctcggcctc tgagctattc cagaagtagt gaggaggctt     300

ttttggaggc ctaggctttt gcaaaaagct ttgcaaagat ggataaagtt                350


<210>  33
<211>  225
<212>  DNA
<213>  Bovinae


<220>
<221>  polyA_signal
<222>  (1)..(225)
<223>  Nucleotides 1943-2167 in bovine growth factor gene (GenBank
       E00847.1) comprising polyadenylation signal

<400>  33
ctgtgccttc tagttgccag ccatctgttg tttgcccctc ccccgtgcct tccttgaccc      60

tggaaggtgc cactcccact gtcctttcct aataaaatga ggaaattgca tcgcattgtc     120

tgagtaggtg tcattctatt ctggggggtg gggtggggca ggacagcaag ggggaggatt     180

gggaagacaa tagcaggcat gctggggatg cggtgggctc tatgg                     225


<210>  34
<211>  131
<212>  DNA
<213>  SV40


<220>
<221>  polyA_signal
<222>  (1)..(131)
<223>  Nucleotides 5929-6181 in GenBank  A70359.1 comprising SV40
       polyadenylation signal

<400>  34
aacttgttta ttgcagctta taatggttac aaataaagca atagcatcac aaatttcaca      60

aataaagcat ttttttcact gcattctagt tgtggtttgt ccaaactcat caatgtatct 120

tatcatgtct g 131

<210> 35
<211> 63
<212> DNA
<213> Synthetic sequence

<220>
<221> CDS
<222> (1)..(63)
<223> Synthetic signal peptide (SYN1) coding sequence

<400> 35
atg tgg tgg agg ctg tgg tgg ctg ctg ctg ctg ctg ctg ctg ctg tgg 48
Met Trp Trp Arg Leu Trp Trp Leu Leu Leu Leu Leu Leu Leu Leu Trp
1               5                   10                  15

ccc atg gtg tgg gcc 63
Pro Met Val Trp Ala
            20

<210> 36
<211> 21
<212> PRT
<213> Synthetic sequence

<400> 36

Met Trp Trp Arg Leu Trp Trp Leu Leu Leu Leu Leu Leu Leu Leu Trp
1               5                   10                  15

Pro Met Val Trp Ala
            20

<210> 37
<211> 69
<212> DNA
<213> Synthetic sequence

<220>
<221> CDS
<222> (1)..(69)
<223> Synthetic signal peptide (SYN2) coding sequence

<400> 37
atg agg ccc acc tgg gcc tgg tgg ctg ttc ctg gtg ctg ctg ctg gcc 48
Met Arg Pro Thr Trp Ala Trp Trp Leu Phe Leu Val Leu Leu Leu Ala
1               5                   10                  15

ctg tgg gcc ccc gcc agg ggc 69
Leu Trp Ala Pro Ala Arg Gly
            20

<210>  38
<211>  23
<212>  PRT
<213>  Synthetic sequence

<400>  38

Met Arg Pro Thr Trp Ala Trp Trp Leu Phe Leu Val Leu Leu Leu Ala
1               5                   10                  15

Leu Trp Ala Pro Ala Arg Gly
            20

<210>  39
<211>  69
<212>  DNA
<213>  Homo sapiens

<220>
<221>  CDS
<222>  (1)..(69)
<223>  Human IFNG gene encoding Interferon gamma signal peptide

<400>  39
atg aaa tat aca agt tat atc ttg gct ttt cag ctc tgc atc gtt ttg      48
Met Lys Tyr Thr Ser Tyr Ile Leu Ala Phe Gln Leu Cys Ile Val Leu
1               5                   10                  15

ggt tct ctt ggc agc gct ggt                                          69
Gly Ser Leu Gly Ser Ala Gly
            20

<210>  40
<211>  23
<212>  PRT
<213>  Homo sapiens

<400>  40

Met Lys Tyr Thr Ser Tyr Ile Leu Ala Phe Gln Leu Cys Ile Val Leu
1               5                   10                  15

Gly Ser Leu Gly Ser Ala Gly
            20

<210>  41
<211>  29
<212>  DNA
<213>  Cytomegalovirus

<220>
<221>  primer_bind
<222>  (1)..(29)
<223>  CMV_FW primer (Cytomegalovirus)

<400>  41

acgtcgcucg atgtacgggc cagatatac                                          29

<210>  42
<211>  31
<212>  DNA
<213>  Cytomegalovirus

<220>
<221>  primer_bind
<222>  (1)..(31)
<223>  CMV_RV primer (Cytomegalovirus)

<400>  42
atcgcacuat ttcgataagc cagtaagcag t                                    31

<210>  43
<211>  30
<212>  DNA
<213>  Bovine

<220>
<221>  primer_bind
<222>  (1)..(30)
<223>  BGH pA_FW primer (Bovine growth hormone)

<400>  43
acacagtcuc tgtgccttct agttgccagc                                      30

<210>  44
<211>  27
<212>  DNA
<213>  Bovine

<220>
<221>  primer_bind
<222>  (1)..(27)
<223>  BGH pA_RV primer (Bovine growth hormone)

<400>  44
acgcaagucc atagagccca ccgcatc                                         27

<210>  45
<211>  30
<212>  DNA
<213>  Bovine

<220>
<221>  primer_bind
<222>  (1)..(30)
<223>  BGHpA (Rituximab HC)FW primer

<400>  45
attaagacuc tgtgccttct agttgccagc                                      30

<210>  46
<211>  28
<212>  DNA
<213>  Bovine

<220>
<221>  primer_bind
<222>  (1)..(28)
<223>  BGHpA (Rituximab HC)RV primer

<400>  46
aggcataguc catagagccc accgcatc                                          28

<210>  47
<211>  27
<212>  DNA
<213>  Chinese hampster

<220>
<221>  primer_bind
<222>  (1)..(27)
<223>  EF1alpa_FW primer

<400>  47
actatgccug tgaggctccg gtgcccg                                           27

<210>  48
<211>  32
<212>  DNA
<213>  Chinese Hampster

<220>
<221>  primer_bind
<222>  (1)..(32)
<223>  EF1alpa_RV primer

<400>  48
accagcgcut cacgacacct gaaatggaag aa                                     32

<210>  49
<211>  27
<212>  DNA
<213>  Mus and Homo Sapiens

<220>
<221>  primer_bind
<222>  (1)..(27)
<223>  Native SP-Rituximab HC_FW primer

<400>  49
agtgcgauat gggctggtcc tgcatca                                           27

<210>  50
<211>  27
<212>  DNA

<213>  Mus and Homo Sapies

<220>
<221>  primer_bind
<222>  (1)..(27)
<223>  Rituximab HC_RV primer

<400>  50
agtcttaaut cacttgccgg ggctcag                                          27

<210>  51
<211>  17
<212>  DNA
<213>  Mus and Homo Sapiens

<220>
<221>  primer_bind
<222>  (1)..(17)
<223>  Rituximab HC_FW primer

<400>  51
agcagccugg cgccgag                                                     17

<210>  52
<211>  39
<212>  DNA
<213>  Mus and Homo Sapiens

<220>
<221>  primer_bind
<222>  (1)..(39)
<223>  SP1-Rituximab HC-RV primer

<400>  52
aggctgcugc agctgcactg cagtagcacc tgctgtgag                             39

<210>  53
<211>  38
<212>  DNA
<213>  Mus and Homo Sapiens

<220>
<221>  primer_bind
<222>  (1)..(38)
<223>  SP2-Rituximab HC-RV primer

<400>  53
aggctgcugc agctgcactc cagctactgc tcccagca                              38

<210>  54
<211>  36
<212>  DNA
<213>  Mus and Homo Sapiens

<220>
<221>  primer_bind
<222>  (1)..(36)
<223>  SP3-Rituximab HC-RV primer

<400>  54
aggctgcugc agctgcactc ccaggaccat gccatt                                36


<210>  55
<211>  35
<212>  DNA
<213>  Mus and Homo Sapiens


<220>
<221>  primer_bind
<222>  (1)..(35)
<223>  SP4-Rituximab HC-RV primer

<400>  55
aggctgcugc agctgcactc cagccaccag gcctg                                 35


<210>  56
<211>  35
<212>  DNA
<213>  Mus and Homo Sapiens


<220>
<221>  primer_bind
<222>  (1)..(35)
<223>  SP5-Rituximab HC-RV primer

<400>  56
aggctgcugc agctgcaccg ccctgcaagg ctggc                                 35


<210>  57
<211>  40
<212>  DNA
<213>  Mus and Homo Sapiens


<220>
<221>  primer_bind
<222>  (1)..(40)
<223>  SP6-Rituximab HC-RV primer

<400>  57
aggctgcugc agctgcacgt gcaggttgac taggtgggtc                            40


<210>  58
<211>  41
<212>  DNA
<213>  Mus and Homo Sapiens


<220>
<221>  primer_bind
<222>  (1)..(41)

<223>  SP7-Rituximab HC-RV primer

<400>  58
aggctgcugc agctgcactc ctctgacaga aatggaacaa a                          41


<210>  59
<211>  35
<212>  DNA
<213>  Mus and Homo Sapiens


<220>
<221>  primer_bind
<222>  (1)..(35)
<223>  SP8-Rituximab HC-RV primer

<400>  59
aggctgcugc agctgcacgg ccaggcatcc tgcca                                 35


<210>  60
<211>  40
<212>  DNA
<213>  Mus and Homo Sapiens


<220>
<221>  primer_bind
<222>  (1)..(40)
<223>  SP9-Rituximab HC-RV primer

<400>  60
aggctgcugc agctgcactg ccaggctcat tttcaataga                            40


<210>  61
<211>  39
<212>  DNA
<213>  Mus and Homo Sapiens


<220>
<221>  primer_bind
<222>  (1)..(39)
<223>  SP10-Rituximab HC-RV primer

<400>  61
aggctgcugc agctgcacag cagagctgca gcctagaac                             39


<210>  62
<211>  36
<212>  DNA
<213>  Mus and Homo sapiens


<220>
<221>  primer_bind
<222>  (1)..(36)
<223>  SP11-Rituximab HC-RV primer

<400>  62

aggctgcugc agctgcacgg ccaaggctgt tcccaa                                36

<210>  63
<211>  39
<212>  DNA
<213>  Mus and Homo sapiens

<220>
<221>  primer_bind
<222>  (1)..(39)
<223>  SP12-Rituximab HC-RV primer

<400>  63
aggctgcugc agctgcactc ccagacagca ggtagacca                             39

<210>  64
<211>  38
<212>  DNA
<213>  Mus and Homo sapiens

<220>
<221>  primer_bind
<222>  (1)..(38)
<223>  SP13-Rituximab HC-RV primer

<400>  64
aggctgcugc agctgcactg agagcgctgg atcacaga                              38

<210>  65
<211>  35
<212>  DNA
<213>  Synthetic sequence

<220>
<221>  primer_bind
<222>  (1)..(35)
<223>  SYN1-Rituximab HC-RV primer

<400>  65
aggctgcugc agctgcacgg cccacaccat gggcc                                 35

<210>  66
<211>  35
<212>  DNA
<213>  Synthetic sequence

<220>
<221>  primer_bind
<222>  (1)..(35)
<223>  SYN2-Rituximab HC-RV primer

<400>  66
aggctgcugc agctgcacgc ccctggcggg ggccc                                 35

<210>  67
<211>  38
<212>  DNA
<213>  Homo sapiens

<220>
<221>  primer_bind
<222>  (1)..(38)
<223>  IFNy SP-Rituximab HC-RV primer

<400>  67
aggctgcugc agctgcacgc caagagaacc caaaacga                              38

<210>  68
<211>  78
<212>  DNA
<213>  Mus and Homo sapiens

<220>
<221>  primer_bind
<222>  (1)..(78)
<223>  SP1-Rituximab LC-FW primer

<400>  68
agcgctggua tgaaattcct tctgctggtc actctgctca cagcaggtgc tactgcaatt      60

gtgctgtctc agtccccc                                                    78

<210>  69
<211>  84
<212>  DNA
<213>  Mus and Homo Sapiens

<220>
<221>  primer_bind
<222>  (1)..(84)
<223>  SP2-Rituximab LC-FW primer

<400>  69
agcgctggua tgcagatgct gcagctgtgg acactcgcag tgctgctggg agcagtagct      60

ggaattgtgc tgtctcagtc cccc                                             84

<210>  70
<211>  93
<212>  DNA
<213>  Mus and Homo sapiens

<220>
<221>  primer_bind
<222>  (1)..(93)
<223>  SP3-Rituximab LC-FW primer

<400>  70
agcgctggua tgaagattct cctgttgtgc gtggggctgc tgctgacctg ggacaatggc      60

atggtcctgg gaattgtgct gtctcagtcc ccc                                    93

<210>  71
<211>  81
<212>  DNA
<213>  Mus and Homo sapiens

<220>
<221>  primer_bind
<222>  (1)..(81)
<223>  SP4-Rituximab LC-FW primer

<400>  71
agcgctggua tgtggaccct ggtaagctgg ctggccttca tggcaggcct ggtggctgga      60

attgtgctgt ctcagtcccc c                                                81

<210>  72
<211>  96
<212>  DNA
<213>  Mus and Homo sapiens

<220>
<221>  primer_bind
<222>  (1)..(96)
<223>  SP5-Rituximab LC-FW primer

<400>  72
agcgctggua tgtcctgcag attcgtgttc cagctcctgg ggctctggct gctgctgagc      60

cagccttgca gggcgattgt gctgtctcag tccccc                                96

<210>  73
<211>  102
<212>  DNA
<213>  Mus and Homo sapiens

<220>
<221>  primer_bind
<222>  (1)..(102)
<223>  SP6-Rituximab LC-FW primer

<400>  73
agcgctggua tgacctccca gattcgccag aattattcca ccgaagtgga agctgtggtg      60

acccacctag tcaacctgca cattgtgctg tctcagtccc cc                        102

<210>  74
<211>  93
<212>  DNA
<213>  Mus and Homo sapiens

<220>
<221>  primer_bind
<222>  (1)..(93)

<223>  SP7-Rituximab LC-FW primer

<400>  74
agcgctggua tggcattccg tgcaatttgt gtattggttg gagtatttat ttgttccatt      60

tctgtcagag gaattgtgct gtctcagtcc ccc                                   93


<210>  75
<211>  84
<212>  DNA
<213>  Mus and Homo sapiens


<220>
<221>  primer_bind
<222>  (1)..(84)
<223>  SP8-Rituximab LC-FW primer

<400>  75
agcgctggua tgaaggctct gtgggccgtg ctggtggtca cattgctggc aggatgcctg      60

gccattgtgc tgtctcagtc cccc                                             84


<210>  76
<211>  78
<212>  DNA
<213>  Mus and Homo sapiens


<220>
<221>  primer_bind
<222>  (1)..(78)
<223>  SP9-Rituximab LC-FW primer

<400>  76
agcgctggua tgaagggcct gatcctgttc ctgtctctat tgaaaatgag cctggcaatt      60

gtgctgtctc agtccccc                                                    78


<210>  77
<211>  87
<212>  DNA
<213>  Mus and Homo sapiens


<220>
<221>  primer_bind
<222>  (1)..(87)
<223>  SP10-Rituximab LC-FW primer

<400>  77
agcgctggua tgagaccctg gccgtccctg gtcctggcac tcctggttct aggctgcagc      60

tctgctattg tgctgtctca gtccccc                                          87


<210>  78
<211>  81
<212>  DNA
<213>  Mus and Homo sapiens

<220>
<221>  primer_bind
<222>  (1)..(81)
<223>  SP11-Rituximab LC-FW primer

<400>  78
agcgctggua tgaatctttt actcctcctg gccaccctgt gcttgggaac agccttggcc      60

attgtgctgt ctcagtcccc c                                                81


<210>  79
<211>  99
<212>  DNA
<213>  Mus and Homo sapiens


<220>
<221>  primer_bind
<222>  (1)..(99)
<223>  SP12-Rituximab LC-FW primer

<400>  79
agcgctggua tggcaaggct ctgtgctttc ctgttggccc tggtggtgat gagctactgg      60

tctacctgct gtctgggaat tgtgctgtct cagtccccc                             99


<210>  80
<211>  99
<212>  DNA
<213>  Mus and Homo sapiens


<220>
<221>  primer_bind
<222>  (1)..(99)
<223>  SP13-Rituximab LC-FW primer

<400>  80
agcgctggua tgaagctctg tgtgtctgcc ctctctctcc tcctgttcgt ggctgccttc      60

tgtgatccag cgctctcaat tgtgctgtct cagtccccc                             99


<210>  81
<211>  93
<212>  DNA
<213>  Synthetic sequence


<220>
<221>  primer_bind
<222>  (1)..(93)
<223>  SYN1--Rituximab LC_FW primer

<400>  81
agcgctggua tgtggtggag gctgtggtgg ctgctgctgc tgctgctgct gctgtggccc      60

atggtgtggg ccattgtgct gtctcagtcc ccc                                   93

<210>  82
<211>  99
<212>  DNA
<213>  Synthetic sequence

<220>
<221>  primer_bind
<222>  (1)..(99)
<223>  SYN2--Rituximab LC_FW primer

<400>  82
agcgctggua tgaggcccac ctgggcctgg tggctgttcc tggtgctgct gctggccctg      60

tgggcccccg ccaggggcat tgtgctgtct cagtccccc                             99

<210>  83
<211>  90
<212>  DNA
<213>  Homo sapiens

<220>
<221>  primer_bind
<222>  (1)..(90)
<223>  IFNy--Rituximab LC_FW primer

<400>  83
agcgctggua tgaaatatac aagttatatc ttggcttttc agctctgcat cgttttgggt      60

tctcttggca ttgtgctgtc tcagtccccc                                       90

<210>  84
<211>  27
<212>  DNA
<213>  Homo sapiens

<220>
<221>  primer_bind
<222>  (1)..(27)
<223>  NaSP-Rituximab LC_FW primer

<400>  84
agcgctggua tgggctggtc ctgcatc                                          27

<210>  85
<211>  31
<212>  DNA
<213>  Mus and Homo sapiens

<220>
<221>  primer_bind
<222>  (1)..(31)
<223>  Rituximab-LC_RV primer

<400>  85
agactgtgut cagcactcgc cccggttgaa a                                     31

<210>  86
<211>  29
<212>  DNA
<213>  Synthetic sequence

<220>
<221>  primer_bind
<222>  (1)..(29)
<223>  Vector_Mam-RV primer

<400>  86
agcgacguga gtcgaataag ggcgacacc                                    29

<210>  87
<211>  1542
<212>  DNA
<213>  Homo sapiens

<220>
<221>  CDS
<222>  (1)..(1542)
<223>  Gene encoding mature secreted alkaline phosphatase (SEAP)

<400>  87
atc atc cca gtt gag gag gag aac ccg gac ttc tgg aac cgc gag gca      48
Ile Ile Pro Val Glu Glu Glu Asn Pro Asp Phe Trp Asn Arg Glu Ala
1               5                   10                  15

gcc gag gcc ctg ggt gcc gcc aag aag ctg cag cct gca cag aca gcc      96
Ala Glu Ala Leu Gly Ala Ala Lys Lys Leu Gln Pro Ala Gln Thr Ala
            20                  25                  30

gcc aag aac ctc atc atc ttc ctg ggc gat ggg atg ggg gtg tct acg      144
Ala Lys Asn Leu Ile Ile Phe Leu Gly Asp Gly Met Gly Val Ser Thr
        35                  40                  45

gtg aca gct gcc agg atc cta aaa ggg cag aag aag gac aaa ctg ggg      192
Val Thr Ala Ala Arg Ile Leu Lys Gly Gln Lys Lys Asp Lys Leu Gly
    50                  55                  60

cct gag ata ccc ctg gcc atg gac cgc ttc cca tat gtg gct ctg tcc      240
Pro Glu Ile Pro Leu Ala Met Asp Arg Phe Pro Tyr Val Ala Leu Ser
65                  70                  75                  80

aag aca tac aat gta gac aaa cat gtg cca gac agt gga gcc aca gcc      288
Lys Thr Tyr Asn Val Asp Lys His Val Pro Asp Ser Gly Ala Thr Ala
                85                  90                  95

acg gcc tac ctg tgc ggg gtc aag ggc aac ttc cag acc att ggc ttg      336
Thr Ala Tyr Leu Cys Gly Val Lys Gly Asn Phe Gln Thr Ile Gly Leu
            100                 105                 110

agt gca gcc gcc cgc ttt aac cag tgc aac acg aca cgc ggc aac gag      384
Ser Ala Ala Ala Arg Phe Asn Gln Cys Asn Thr Thr Arg Gly Asn Glu
        115                 120                 125

gtc atc tcc gtg atg aat cgg gcc aag aaa gca ggg aag tca gtg gga      432
Val Ile Ser Val Met Asn Arg Ala Lys Lys Ala Gly Lys Ser Val Gly

130                 135                 140

gtg gta acc acc aca cga gtg cag cac gcc tcg cca gcc ggc acc tac        480
Val Val Thr Thr Thr Arg Val Gln His Ala Ser Pro Ala Gly Thr Tyr
145                 150                 155                 160

gcc cac acg gtg aac cgc aac tgg tac tcg gac gcc gac gtg cct gcc        528
Ala His Thr Val Asn Arg Asn Trp Tyr Ser Asp Ala Asp Val Pro Ala
                165                 170                 175

tcg gcc cgc cag gag ggg tgc cag gac atc gct acg cag ctc atc tcc        576
Ser Ala Arg Gln Glu Gly Cys Gln Asp Ile Ala Thr Gln Leu Ile Ser
            180                 185                 190

aac atg gac att gac gtg atc cta ggt gga ggc cga aag tac atg ttt        624
Asn Met Asp Ile Asp Val Ile Leu Gly Gly Gly Arg Lys Tyr Met Phe
        195                 200                 205

ccc atg gga acc cca gac cct gag tac cca gat gac tac agc caa ggt        672
Pro Met Gly Thr Pro Asp Pro Glu Tyr Pro Asp Asp Tyr Ser Gln Gly
    210                 215                 220

ggg acc agg ctg gac ggg aag aat ctg gtg cag gaa tgg ctg gcg aag        720
Gly Thr Arg Leu Asp Gly Lys Asn Leu Val Gln Glu Trp Leu Ala Lys
225                 230                 235                 240

cgc cag ggt gcc cgg tat gtg tgg aac cgc act gag ctc atg cag gct        768
Arg Gln Gly Ala Arg Tyr Val Trp Asn Arg Thr Glu Leu Met Gln Ala
                245                 250                 255

tcc ctg gac ccg tct gtg acc cat ctc atg ggt ctc ttt gag cct gga        816
Ser Leu Asp Pro Ser Val Thr His Leu Met Gly Leu Phe Glu Pro Gly
            260                 265                 270

gac atg aaa tac gag atc cac cga gac tcc aca ctg gac ccc tcc ctg        864
Asp Met Lys Tyr Glu Ile His Arg Asp Ser Thr Leu Asp Pro Ser Leu
        275                 280                 285

atg gag atg aca gag gct gcc ctg cgc ctg ctg agc agg aac ccc cgc        912
Met Glu Met Thr Glu Ala Ala Leu Arg Leu Leu Ser Arg Asn Pro Arg
    290                 295                 300

ggc ttc ttc ctc ttc gtg gag ggt ggt cgc atc gac cat ggt cat cat        960
Gly Phe Phe Leu Phe Val Glu Gly Gly Arg Ile Asp His Gly His His
305                 310                 315                 320

gaa agc agg gct tac cgg gca ctg act gag acg atc atg ttc gac gac       1008
Glu Ser Arg Ala Tyr Arg Ala Leu Thr Glu Thr Ile Met Phe Asp Asp
                325                 330                 335

gcc att gag agg gcg ggc cag ctc acc agc gag gag gac acg ctg agc       1056
Ala Ile Glu Arg Ala Gly Gln Leu Thr Ser Glu Glu Asp Thr Leu Ser
            340                 345                 350

ctc gtc act gcc gac cac tcc cac gtc ttc tcc ttc gga ggc tac ccc       1104
Leu Val Thr Ala Asp His Ser His Val Phe Ser Phe Gly Gly Tyr Pro
        355                 360                 365

ctg cga ggg agc tcc atc ttc ggg ctg gcc cct ggc aag gcc cgg gac       1152
Leu Arg Gly Ser Ser Ile Phe Gly Leu Ala Pro Gly Lys Ala Arg Asp
    370                 375                 380

agg aag gcc tac acg gtc ctc cta tac gga aac ggt cca ggc tat gtg       1200

Arg Lys Ala Tyr Thr Val Leu Leu Tyr Gly Asn Gly Pro Gly Tyr Val
385                 390                 395                 400

ctc aag gac ggc gcc cgg ccg gat gtt acc gag agc gag agc ggg agc      1248
Leu Lys Asp Gly Ala Arg Pro Asp Val Thr Glu Ser Glu Ser Gly Ser
                405                 410                 415

ccc gag tat cgg cag cag tca gca gtg ccc ctg gac gaa gag acc cac      1296
Pro Glu Tyr Arg Gln Gln Ser Ala Val Pro Leu Asp Glu Glu Thr His
            420                 425                 430

gca ggc gag gac gtg gcg gtg ttc gcg cgc ggc ccg cag gcg cac ctg      1344
Ala Gly Glu Asp Val Ala Val Phe Ala Arg Gly Pro Gln Ala His Leu
        435                 440                 445

gtt cac ggc gtg cag gag cag acc ttc ata gcg cac gtc atg gcc ttc      1392
Val His Gly Val Gln Glu Gln Thr Phe Ile Ala His Val Met Ala Phe
    450                 455                 460

gcc gcc tgc ctg gag ccc tac acc gcc tgc gac ctg gcg ccc ccc gcc      1440
Ala Ala Cys Leu Glu Pro Tyr Thr Ala Cys Asp Leu Ala Pro Pro Ala
465                 470                 475                 480

ggc acc acc gac gcc gcg cac ccg ggg cgg tcc gtg gtc ccc gcg ttg      1488
Gly Thr Thr Asp Ala Ala His Pro Gly Arg Ser Val Val Pro Ala Leu
                485                 490                 495

ctt cct ctg ctg gcc ggg acc ctg ctg ctg ctg gag acg gcc act gct      1536
Leu Pro Leu Leu Ala Gly Thr Leu Leu Leu Leu Glu Thr Ala Thr Ala
            500                 505                 510

ccc tga                                                              1542
Pro

<210>  88
<211>  513
<212>  PRT
<213>  Homo sapiens

<400>  88

Ile Ile Pro Val Glu Glu Glu Asn Pro Asp Phe Trp Asn Arg Glu Ala
1               5                   10                  15

Ala Glu Ala Leu Gly Ala Ala Lys Lys Leu Gln Pro Ala Gln Thr Ala
            20                  25                  30

Ala Lys Asn Leu Ile Ile Phe Leu Gly Asp Gly Met Gly Val Ser Thr
        35                  40                  45

Val Thr Ala Ala Arg Ile Leu Lys Gly Gln Lys Lys Asp Lys Leu Gly
    50                  55                  60

Pro Glu Ile Pro Leu Ala Met Asp Arg Phe Pro Tyr Val Ala Leu Ser
65                  70                  75                  80

Lys Thr Tyr Asn Val Asp Lys His Val Pro Asp Ser Gly Ala Thr Ala
                85                  90                  95

Thr Ala Tyr Leu Cys Gly Val Lys Gly Asn Phe Gln Thr Ile Gly Leu
            100                 105                 110

Ser Ala Ala Ala Arg Phe Asn Gln Cys Asn Thr Thr Arg Gly Asn Glu
        115                 120                 125

Val Ile Ser Val Met Asn Arg Ala Lys Lys Ala Gly Lys Ser Val Gly
    130                 135                 140

Val Val Thr Thr Thr Arg Val Gln His Ala Ser Pro Ala Gly Thr Tyr
145                 150                 155                 160

Ala His Thr Val Asn Arg Asn Trp Tyr Ser Asp Ala Asp Val Pro Ala
                165                 170                 175

Ser Ala Arg Gln Glu Gly Cys Gln Asp Ile Ala Thr Gln Leu Ile Ser
            180                 185                 190

Asn Met Asp Ile Asp Val Ile Leu Gly Gly Gly Arg Lys Tyr Met Phe
        195                 200                 205

Pro Met Gly Thr Pro Asp Pro Glu Tyr Pro Asp Asp Tyr Ser Gln Gly
    210                 215                 220

Gly Thr Arg Leu Asp Gly Lys Asn Leu Val Gln Glu Trp Leu Ala Lys
225                 230                 235                 240

Arg Gln Gly Ala Arg Tyr Val Trp Asn Arg Thr Glu Leu Met Gln Ala
                245                 250                 255

Ser Leu Asp Pro Ser Val Thr His Leu Met Gly Leu Phe Glu Pro Gly
            260                 265                 270

Asp Met Lys Tyr Glu Ile His Arg Asp Ser Thr Leu Asp Pro Ser Leu
        275                 280                 285

Met Glu Met Thr Glu Ala Ala Leu Arg Leu Leu Ser Arg Asn Pro Arg
    290                 295                 300

Gly Phe Phe Leu Phe Val Glu Gly Gly Arg Ile Asp His Gly His His
305                 310                 315                 320

Glu Ser Arg Ala Tyr Arg Ala Leu Thr Glu Thr Ile Met Phe Asp Asp
                325                 330                 335

Ala Ile Glu Arg Ala Gly Gln Leu Thr Ser Glu Glu Asp Thr Leu Ser
            340                 345                 350

Leu Val Thr Ala Asp His Ser His Val Phe Ser Phe Gly Gly Tyr Pro
        355                 360                 365

Leu Arg Gly Ser Ser Ile Phe Gly Leu Ala Pro Gly Lys Ala Arg Asp
    370                 375                 380

Arg Lys Ala Tyr Thr Val Leu Leu Tyr Gly Asn Gly Pro Gly Tyr Val
385                 390                 395                 400

Leu Lys Asp Gly Ala Arg Pro Asp Val Thr Glu Ser Glu Ser Gly Ser
                405                 410                 415

Pro Glu Tyr Arg Gln Gln Ser Ala Val Pro Leu Asp Glu Glu Thr His
            420                 425                 430

Ala Gly Glu Asp Val Ala Val Phe Ala Arg Gly Pro Gln Ala His Leu
        435                 440                 445

Val His Gly Val Gln Glu Gln Thr Phe Ile Ala His Val Met Ala Phe
    450                 455                 460

Ala Ala Cys Leu Glu Pro Tyr Thr Ala Cys Asp Leu Ala Pro Pro Ala
465                 470                 475                 480

Gly Thr Thr Asp Ala Ala His Pro Gly Arg Ser Val Val Pro Ala Leu
                485                 490                 495

Leu Pro Leu Leu Ala Gly Thr Leu Leu Leu Leu Glu Thr Ala Thr Ala
            500                 505                 510

Pro

<210>  89
<211>  1608
<212>  DNA
<213>  Homo sapiens

<220>
<221>  CDS
<222>  (1)..(1608)
<223>  Gene encoding precursor secreted alkaline phosphatase polypeptide
       (SEAP)

<400>  89
atg ctg ggg ccc tgc atg ctg ctg ctg ctg ctg ctg ctg ggc ctg agg        48

Met Leu Gly Pro Cys Met Leu Leu Leu Leu Leu Leu Gly Leu Arg
1               5                   10                  15

cta cag ctc tcc ctg ggc atc atc cca gtt gag gag gag aac ccg gac      96
Leu Gln Leu Ser Leu Gly Ile Ile Pro Val Glu Glu Glu Asn Pro Asp
            20                  25                  30

ttc tgg aac cgc gag gca gcc gag gcc ctg ggt gcc gcc aag aag ctg      144
Phe Trp Asn Arg Glu Ala Ala Glu Ala Leu Gly Ala Ala Lys Lys Leu
        35                  40                  45

cag cct gca cag aca gcc gcc aag aac ctc atc atc ttc ctg ggc gat      192
Gln Pro Ala Gln Thr Ala Ala Lys Asn Leu Ile Ile Phe Leu Gly Asp
    50                  55                  60

ggg atg ggg gtg tct acg gtg aca gct gcc agg atc cta aaa ggg cag      240
Gly Met Gly Val Ser Thr Val Thr Ala Ala Arg Ile Leu Lys Gly Gln
65                  70                  75                  80

aag aag gac aaa ctg ggg cct gag ata ccc ctg gcc atg gac cgc ttc      288
Lys Lys Asp Lys Leu Gly Pro Glu Ile Pro Leu Ala Met Asp Arg Phe
                85                  90                  95

cca tat gtg gct ctg tcc aag aca tac aat gta gac aaa cat gtg cca      336
Pro Tyr Val Ala Leu Ser Lys Thr Tyr Asn Val Asp Lys His Val Pro
            100                 105                 110

gac agt gga gcc aca gcc acg gcc tac ctg tgc ggg gtc aag ggc aac      384
Asp Ser Gly Ala Thr Ala Thr Ala Tyr Leu Cys Gly Val Lys Gly Asn
        115                 120                 125

ttc cag acc att ggc ttg agt gca gcc gcc cgc ttt aac cag tgc aac      432
Phe Gln Thr Ile Gly Leu Ser Ala Ala Ala Arg Phe Asn Gln Cys Asn
    130                 135                 140

acg aca cgc ggc aac gag gtc atc tcc gtg atg aat cgg gcc aag aaa      480
Thr Thr Arg Gly Asn Glu Val Ile Ser Val Met Asn Arg Ala Lys Lys
145                 150                 155                 160

gca ggg aag tca gtg gga gtg gta acc acc aca cga gtg cag cac gcc      528
Ala Gly Lys Ser Val Gly Val Val Thr Thr Thr Arg Val Gln His Ala
                165                 170                 175

tcg cca gcc ggc acc tac gcc cac acg gtg aac cgc aac tgg tac tcg      576
Ser Pro Ala Gly Thr Tyr Ala His Thr Val Asn Arg Asn Trp Tyr Ser
            180                 185                 190

gac gcc gac gtg cct gcc tcg gcc cgc cag gag ggg tgc cag gac atc      624
Asp Ala Asp Val Pro Ala Ser Ala Arg Gln Glu Gly Cys Gln Asp Ile
        195                 200                 205

gct acg cag ctc atc tcc aac atg gac att gac gtg atc cta ggt gga      672
Ala Thr Gln Leu Ile Ser Asn Met Asp Ile Asp Val Ile Leu Gly Gly
    210                 215                 220

ggc cga aag tac atg ttt ccc atg gga acc cca gac cct gag tac cca      720
Gly Arg Lys Tyr Met Phe Pro Met Gly Thr Pro Asp Pro Glu Tyr Pro
225                 230                 235                 240

gat gac tac agc caa ggt ggg acc agg ctg gac ggg aag aat ctg gtg      768
Asp Asp Tyr Ser Gln Gly Gly Thr Arg Leu Asp Gly Lys Asn Leu Val
                245                 250                 255

cag gaa tgg ctg gcg aag cgc cag ggt gcc cgg tat gtg tgg aac cgc          816
Gln Glu Trp Leu Ala Lys Arg Gln Gly Ala Arg Tyr Val Trp Asn Arg
            260                 265                 270

act gag ctc atg cag gct tcc ctg gac ccg tct gtg acc cat ctc atg          864
Thr Glu Leu Met Gln Ala Ser Leu Asp Pro Ser Val Thr His Leu Met
        275                 280                 285

ggt ctc ttt gag cct gga gac atg aaa tac gag atc cac cga gac tcc          912
Gly Leu Phe Glu Pro Gly Asp Met Lys Tyr Glu Ile His Arg Asp Ser
    290                 295                 300

aca ctg gac ccc tcc ctg atg gag atg aca gag gct gcc ctg cgc ctg          960
Thr Leu Asp Pro Ser Leu Met Glu Met Thr Glu Ala Ala Leu Arg Leu
305                 310                 315                 320

ctg agc agg aac ccc cgc ggc ttc ttc ctc ttc gtg gag ggt ggt cgc         1008
Leu Ser Arg Asn Pro Arg Gly Phe Phe Leu Phe Val Glu Gly Gly Arg
                325                 330                 335

atc gac cat ggt cat cat gaa agc agg gct tac cgg gca ctg act gag         1056
Ile Asp His Gly His His Glu Ser Arg Ala Tyr Arg Ala Leu Thr Glu
            340                 345                 350

acg atc atg ttc gac gac gcc att gag agg gcg ggc cag ctc acc agc         1104
Thr Ile Met Phe Asp Asp Ala Ile Glu Arg Ala Gly Gln Leu Thr Ser
        355                 360                 365

gag gag gac acg ctg agc ctc gtc act gcc gac cac tcc cac gtc ttc         1152
Glu Glu Asp Thr Leu Ser Leu Val Thr Ala Asp His Ser His Val Phe
    370                 375                 380

tcc ttc gga ggc tac ccc ctg cga ggg agc tcc atc ttc ggg ctg gcc         1200
Ser Phe Gly Gly Tyr Pro Leu Arg Gly Ser Ser Ile Phe Gly Leu Ala
385                 390                 395                 400

cct ggc aag gcc cgg gac agg aag gcc tac acg gtc ctc cta tac gga         1248
Pro Gly Lys Ala Arg Asp Arg Lys Ala Tyr Thr Val Leu Leu Tyr Gly
                405                 410                 415

aac ggt cca ggc tat gtg ctc aag gac ggc gcc cgg ccg gat gtt acc         1296
Asn Gly Pro Gly Tyr Val Leu Lys Asp Gly Ala Arg Pro Asp Val Thr
            420                 425                 430

gag agc gag agc ggg agc ccc gag tat cgg cag cag tca gca gtg ccc         1344
Glu Ser Glu Ser Gly Ser Pro Glu Tyr Arg Gln Gln Ser Ala Val Pro
        435                 440                 445

ctg gac gaa gag acc cac gca ggc gag gac gtg gcg gtg ttc gcg cgc         1392
Leu Asp Glu Glu Thr His Ala Gly Glu Asp Val Ala Val Phe Ala Arg
    450                 455                 460

ggc ccg cag gcg cac ctg gtt cac ggc gtg cag gag cag acc ttc ata         1440
Gly Pro Gln Ala His Leu Val His Gly Val Gln Glu Gln Thr Phe Ile
465                 470                 475                 480

gcg cac gtc atg gcc ttc gcc gcc tgc ctg gag ccc tac acc gcc tgc         1488
Ala His Val Met Ala Phe Ala Ala Cys Leu Glu Pro Tyr Thr Ala Cys
                485                 490                 495

gac ctg gcg ccc ccc gcc ggc acc acc gac gcc gcg cac ccg ggg cgg         1536
Asp Leu Ala Pro Pro Ala Gly Thr Thr Asp Ala Ala His Pro Gly Arg
            500                 505                 510

tcc gtg gtc ccc gcg ttg ctt cct ctg ctg gcc ggg acc ctg ctg ctg     1584
Ser Val Val Pro Ala Leu Leu Pro Leu Leu Ala Gly Thr Leu Leu Leu
        515                 520                 525

ctg gag acg gcc act gct ccc tga                                     1608
Leu Glu Thr Ala Thr Ala Pro
    530                 535

<210>  90
<211>  535
<212>  PRT
<213>  Homo sapiens

<400>  90

<210>  91
<211>  501
<212>  DNA
<213>  Homo sapiens

<220>
<221>  CDS
<222>  (1)..(501)
<223>  Gene encoding mature hormone type erythropoetin polypeptide
       (EPO).

<400>  91
gct cct cct aga ctg atc tgc gac tcc cgg gtg ctg gaa aga tac ctg      48
Ala Pro Pro Arg Leu Ile Cys Asp Ser Arg Val Leu Glu Arg Tyr Leu
1               5                   10                  15

ctg gaa gcc aaa gag gcc gag aac atc acc acc ggc tgc gcc gag cac      96
Leu Glu Ala Lys Glu Ala Glu Asn Ile Thr Thr Gly Cys Ala Glu His
            20                  25                  30

tgc tcc ctg aac gag aat atc acc gtg ccc gac acc aaa gtg aac ttc      144
Cys Ser Leu Asn Glu Asn Ile Thr Val Pro Asp Thr Lys Val Asn Phe
        35                  40                  45

tac gcc tgg aag cgg atg gaa gtg ggc cag cag gct gtg gaa gtg tgg      192
Tyr Ala Trp Lys Arg Met Glu Val Gly Gln Gln Ala Val Glu Val Trp
    50                  55                  60

cag gga ctg gct ctg ctg agc gag gct gtg ctg aga gga cag gcc ctg      240
Gln Gly Leu Ala Leu Leu Ser Glu Ala Val Leu Arg Gly Gln Ala Leu
65                  70                  75                  80

ctc gtg aac tcc tcc cag cct tgg gaa ccc ctg cag ctg cac gtg gac      288
Leu Val Asn Ser Ser Gln Pro Trp Glu Pro Leu Gln Leu His Val Asp
                85                  90                  95

aag gct gtg tcc ggc ctg aga tcc ctg acc acc ctg ctg aga gca ctg     336
Lys Ala Val Ser Gly Leu Arg Ser Leu Thr Thr Leu Leu Arg Ala Leu
            100                 105                 110

gga gcc cag aaa gag gcc atc tct cca cct gac gcc gcc tct gct gct     384
Gly Ala Gln Lys Glu Ala Ile Ser Pro Pro Asp Ala Ala Ser Ala Ala
        115                 120                 125

cct ctg aga acc atc acc gcc gac acc ttc aga aag ctg ttc cgg gtg     432
Pro Leu Arg Thr Ile Thr Ala Asp Thr Phe Arg Lys Leu Phe Arg Val
    130                 135                 140

tac tcc aac ttc ctg cgg ggc aag ctg aag ctg tac acc ggc gag gct     480
Tyr Ser Asn Phe Leu Arg Gly Lys Leu Lys Leu Tyr Thr Gly Glu Ala
145                 150                 155                 160

tgc cgg acc ggc gat aga tga                                         501
Cys Arg Thr Gly Asp Arg
                165

<210>  92
<211>  166
<212>  PRT
<213>  Homo sapiens

<400>  92

Ala Pro Pro Arg Leu Ile Cys Asp Ser Arg Val Leu Glu Arg Tyr Leu
1               5                   10                  15

Leu Glu Ala Lys Glu Ala Glu Asn Ile Thr Thr Gly Cys Ala Glu His
            20                  25                  30

Cys Ser Leu Asn Glu Asn Ile Thr Val Pro Asp Thr Lys Val Asn Phe
        35                  40                  45

Tyr Ala Trp Lys Arg Met Glu Val Gly Gln Gln Ala Val Glu Val Trp
    50                  55                  60

Gln Gly Leu Ala Leu Leu Ser Glu Ala Val Leu Arg Gly Gln Ala Leu
65                  70                  75                  80

Leu Val Asn Ser Ser Gln Pro Trp Glu Pro Leu Gln Leu His Val Asp
                85                  90                  95

Lys Ala Val Ser Gly Leu Arg Ser Leu Thr Thr Leu Leu Arg Ala Leu
            100                 105                 110

Gly Ala Gln Lys Glu Ala Ile Ser Pro Pro Asp Ala Ala Ser Ala Ala
        115                 120                 125

Pro Leu Arg Thr Ile Thr Ala Asp Thr Phe Arg Lys Leu Phe Arg Val
    130                 135                 140

Tyr Ser Asn Phe Leu Arg Gly Lys Leu Lys Leu Tyr Thr Gly Glu Ala
145                 150                 155                 160

Cys Arg Thr Gly Asp Arg
                165

<210>  93
<211>  1407
<212>  DNA
<213>  Synthetic sequence

<220>
<221>  CDS
<222>  (1)..(1407)
<223>  Gene encoding mature Enbrel polypeptide.

<400>  93
ttg ccc gcc cag gtg gca ttt aca ccc tac gcc ccg gag ccc ggg agc      48
Leu Pro Ala Gln Val Ala Phe Thr Pro Tyr Ala Pro Glu Pro Gly Ser
1               5                   10                  15

aca tgc cgg ctc aga gaa tac tat gac cag aca gct cag atg tgc tgc      96
Thr Cys Arg Leu Arg Glu Tyr Tyr Asp Gln Thr Ala Gln Met Cys Cys
            20                  25                  30

agc aaa tgc tcg ccg ggc caa cat gca aaa gtc ttc tgt acc aag acc      144
Ser Lys Cys Ser Pro Gly Gln His Ala Lys Val Phe Cys Thr Lys Thr
        35                  40                  45

tcg gac acc gtg tgt gac tcc tgt gag gac agc aca tac acc cag ctc      192
Ser Asp Thr Val Cys Asp Ser Cys Glu Asp Ser Thr Tyr Thr Gln Leu
    50                  55                  60

tgg aac tgg gtt ccc gag tgc ttg agc tgt ggc tcc cgc tgt agc tct      240
Trp Asn Trp Val Pro Glu Cys Leu Ser Cys Gly Ser Arg Cys Ser Ser
65                  70                  75                  80

gac cag gtg gaa act caa gcc tgc act cgg gaa cag aac cgc atc tgc      288
Asp Gln Val Glu Thr Gln Ala Cys Thr Arg Glu Gln Asn Arg Ile Cys
                85                  90                  95

acc tgc agg ccc ggc tgg tac tgc gcg ctg agc aag cag gag ggg tgc      336
Thr Cys Arg Pro Gly Trp Tyr Cys Ala Leu Ser Lys Gln Glu Gly Cys
            100                 105                 110

cgg ctg tgc gcg ccg ctg cgc aag tgc cgc ccg ggc ttc ggc gtg gcc      384
Arg Leu Cys Ala Pro Leu Arg Lys Cys Arg Pro Gly Phe Gly Val Ala
        115                 120                 125

aga cca gga act gaa aca tca gac gtg gtg tgc aag ccc tgt gcc ccg      432
Arg Pro Gly Thr Glu Thr Ser Asp Val Val Cys Lys Pro Cys Ala Pro
    130                 135                 140

ggg acg ttc tcc aac acg act tca tcc acg gat att tgc agg ccc cac      480
Gly Thr Phe Ser Asn Thr Thr Ser Ser Thr Asp Ile Cys Arg Pro His
145                 150                 155                 160

cag atc tgt aac gtg gtg gcc atc cct ggg aat gca agc atg gat gca      528

Gln Ile Cys Asn Val Val Ala Ile Pro Gly Asn Ala Ser Met Asp Ala
                165                 170                 175

gtc tgc acg tcc acg tcc ccc acc cgg agt atg gcc cca ggg gca gta      576
Val Cys Thr Ser Thr Ser Pro Thr Arg Ser Met Ala Pro Gly Ala Val
            180                 185                 190

cac tta ccc cag cca gtg tcc aca cga tcc caa cac acg cag cca act      624
His Leu Pro Gln Pro Val Ser Thr Arg Ser Gln His Thr Gln Pro Thr
        195                 200                 205

cca gaa ccc agc act gct cca agc acc tcc ttc ctg ctc cca atg ggc      672
Pro Glu Pro Ser Thr Ala Pro Ser Thr Ser Phe Leu Leu Pro Met Gly
    210                 215                 220

ccc agc ccc cca gct gaa ggg agc act ggc gac gag ccc aaa tct tgt      720
Pro Ser Pro Pro Ala Glu Gly Ser Thr Gly Asp Glu Pro Lys Ser Cys
225                 230                 235                 240

gac aaa act cac aca tgc cca ccg tgc cca gca cct gaa ctc ctg ggg      768
Asp Lys Thr His Thr Cys Pro Pro Cys Pro Ala Pro Glu Leu Leu Gly
                245                 250                 255

gga ccg tca gtc ttc ctc ttc ccc cca aaa ccc aag gac acc ctc atg      816
Gly Pro Ser Val Phe Leu Phe Pro Pro Lys Pro Lys Asp Thr Leu Met
            260                 265                 270

atc tcc cgg acc cct gag gtc aca tgc gtg gtg gtg gac gtg agc cac      864
Ile Ser Arg Thr Pro Glu Val Thr Cys Val Val Val Asp Val Ser His
        275                 280                 285

gaa gac cct gag gtc aag ttc aac tgg tac gtg gac ggc gtg gag gtg      912
Glu Asp Pro Glu Val Lys Phe Asn Trp Tyr Val Asp Gly Val Glu Val
    290                 295                 300

cat aat gcc aag aca aag ccg cgg gag gag cag tac aac agc acg tac      960
His Asn Ala Lys Thr Lys Pro Arg Glu Glu Gln Tyr Asn Ser Thr Tyr
305                 310                 315                 320

cgt gtg gtc agc gtc ctc acc gtc ctg cac cag gac tgg ctg aat ggc     1008
Arg Val Val Ser Val Leu Thr Val Leu His Gln Asp Trp Leu Asn Gly
                325                 330                 335

aag gag tac aag tgc aag gtc tcc aac aaa gcc ctc cca gcc ccc atc     1056
Lys Glu Tyr Lys Cys Lys Val Ser Asn Lys Ala Leu Pro Ala Pro Ile
            340                 345                 350

gag aaa acc atc tcc aaa gcc aaa ggg cag ccc cga gaa cca cag gtg     1104
Glu Lys Thr Ile Ser Lys Ala Lys Gly Gln Pro Arg Glu Pro Gln Val
        355                 360                 365

tac acc ctg ccc cca tcc cgg gag gag atg acc aag aac cag gtc agc     1152
Tyr Thr Leu Pro Pro Ser Arg Glu Glu Met Thr Lys Asn Gln Val Ser
    370                 375                 380

ctg acc tgc ctg gtc aaa ggc ttc tat ccc agc gac atc gcc gtg gag     1200
Leu Thr Cys Leu Val Lys Gly Phe Tyr Pro Ser Asp Ile Ala Val Glu
385                 390                 395                 400

tgg gag agc aat ggg cag ccg gag aac aac tac aag acc acg cct ccc     1248
Trp Glu Ser Asn Gly Gln Pro Glu Asn Asn Tyr Lys Thr Thr Pro Pro
                405                 410                 415

gtg ctg gac tcc gac ggc tcc ttc ttc ctc tac agc aag ctc acc gtg     1296
Val Leu Asp Ser Asp Gly Ser Phe Phe Leu Tyr Ser Lys Leu Thr Val
            420                 425                 430

gac aag agc agg tgg cag cag ggg aac gtc ttc tca tgc tcc gtg atg     1344
Asp Lys Ser Arg Trp Gln Gln Gly Asn Val Phe Ser Cys Ser Val Met
        435                 440                 445

cat gag gct ctg cac aac cac tac aca cag aag agc ctc tcc ctg tct     1392
His Glu Ala Leu His Asn His Tyr Thr Gln Lys Ser Leu Ser Leu Ser
    450                 455                 460

ccg ggt aaa tga taa                                                 1407
Pro Gly Lys
465

<210>  94
<211>  467
<212>  PRT
<213>  Synthetic sequence

<400>  94

Leu Pro Ala Gln Val Ala Phe Thr Pro Tyr Ala Pro Glu Pro Gly Ser
1               5                   10                  15

Thr Cys Arg Leu Arg Glu Tyr Tyr Asp Gln Thr Ala Gln Met Cys Cys
            20                  25                  30

Ser Lys Cys Ser Pro Gly Gln His Ala Lys Val Phe Cys Thr Lys Thr
        35                  40                  45

Ser Asp Thr Val Cys Asp Ser Cys Glu Asp Ser Thr Tyr Thr Gln Leu
    50                  55                  60

Trp Asn Trp Val Pro Glu Cys Leu Ser Cys Gly Ser Arg Cys Ser Ser
65                  70                  75                  80

Asp Gln Val Glu Thr Gln Ala Cys Thr Arg Glu Gln Asn Arg Ile Cys
                85                  90                  95

Thr Cys Arg Pro Gly Trp Tyr Cys Ala Leu Ser Lys Gln Glu Gly Cys
            100                 105                 110

Arg Leu Cys Ala Pro Leu Arg Lys Cys Arg Pro Gly Phe Gly Val Ala
        115                 120                 125

Arg Pro Gly Thr Glu Thr Ser Asp Val Val Cys Lys Pro Cys Ala Pro
    130                 135                 140

Gly Thr Phe Ser Asn Thr Thr Ser Ser Thr Asp Ile Cys Arg Pro His
145                 150                 155                 160

Gln Ile Cys Asn Val Val Ala Ile Pro Gly Asn Ala Ser Met Asp Ala
                165                 170                 175

Val Cys Thr Ser Thr Ser Pro Thr Arg Ser Met Ala Pro Gly Ala Val
            180                 185                 190

His Leu Pro Gln Pro Val Ser Thr Arg Ser Gln His Thr Gln Pro Thr
        195                 200                 205

Pro Glu Pro Ser Thr Ala Pro Ser Thr Ser Phe Leu Leu Pro Met Gly
    210                 215                 220

Pro Ser Pro Pro Ala Glu Gly Ser Thr Gly Asp Glu Pro Lys Ser Cys
225                 230                 235                 240

Asp Lys Thr His Thr Cys Pro Pro Cys Pro Ala Pro Glu Leu Leu Gly
                245                 250                 255

Gly Pro Ser Val Phe Leu Phe Pro Pro Lys Pro Lys Asp Thr Leu Met
            260                 265                 270

Ile Ser Arg Thr Pro Glu Val Thr Cys Val Val Val Asp Val Ser His
        275                 280                 285

Glu Asp Pro Glu Val Lys Phe Asn Trp Tyr Val Asp Gly Val Glu Val
    290                 295                 300

His Asn Ala Lys Thr Lys Pro Arg Glu Glu Gln Tyr Asn Ser Thr Tyr
305                 310                 315                 320

Arg Val Val Ser Val Leu Thr Val Leu His Gln Asp Trp Leu Asn Gly
                325                 330                 335

Lys Glu Tyr Lys Cys Lys Val Ser Asn Lys Ala Leu Pro Ala Pro Ile
            340                 345                 350

Glu Lys Thr Ile Ser Lys Ala Lys Gly Gln Pro Arg Glu Pro Gln Val
        355                 360                 365

Tyr Thr Leu Pro Pro Ser Arg Glu Glu Met Thr Lys Asn Gln Val Ser
    370                 375                 380

Leu Thr Cys Leu Val Lys Gly Phe Tyr Pro Ser Asp Ile Ala Val Glu
385                 390                 395                 400

Trp Glu Ser Asn Gly Gln Pro Glu Asn Asn Tyr Lys Thr Thr Pro Pro
                405                 410                 415

Val Leu Asp Ser Asp Gly Ser Phe Phe Leu Tyr Ser Lys Leu Thr Val
            420                 425                 430

Asp Lys Ser Arg Trp Gln Gln Gly Asn Val Phe Ser Cys Ser Val Met
        435                 440                 445

His Glu Ala Leu His Asn His Tyr Thr Gln Lys Ser Leu Ser Leu Ser
    450                 455                 460

Pro Gly Lys
465

<210>  95
<211>  1470
<212>  DNA
<213>  Synthetic sequence

<220>
<221>  CDS
<222>  (1)..(1470)
<223>  Gene encoding precursor Enbrel polypeptide.

<400>  95
atg gcg ccc gtc gcc gtc tgg gcc gcg ctg gcc gtc gga ctg gag ctc      48
Met Ala Pro Val Ala Val Trp Ala Ala Leu Ala Val Gly Leu Glu Leu
1               5                   10                  15

tgg gct gcg gcg cac gcc ttg ccc gcc cag gtg gca ttt aca ccc tac      96
Trp Ala Ala Ala His Ala Leu Pro Ala Gln Val Ala Phe Thr Pro Tyr
            20                  25                  30

gcc ccg gag ccc ggg agc aca tgc cgg ctc aga gaa tac tat gac cag      144
Ala Pro Glu Pro Gly Ser Thr Cys Arg Leu Arg Glu Tyr Tyr Asp Gln
        35                  40                  45

aca gct cag atg tgc tgc agc aaa tgc tcg ccg ggc caa cat gca aaa      192
Thr Ala Gln Met Cys Cys Ser Lys Cys Ser Pro Gly Gln His Ala Lys
    50                  55                  60

gtc ttc tgt acc aag acc tcg gac acc gtg tgt gac tcc tgt gag gac      240
Val Phe Cys Thr Lys Thr Ser Asp Thr Val Cys Asp Ser Cys Glu Asp
65                  70                  75                  80

agc aca tac acc cag ctc tgg aac tgg gtt ccc gag tgc ttg agc tgt      288
Ser Thr Tyr Thr Gln Leu Trp Asn Trp Val Pro Glu Cys Leu Ser Cys
                85                  90                  95

ggc tcc cgc tgt agc tct gac cag gtg gaa act caa gcc tgc act cgg      336
Gly Ser Arg Cys Ser Ser Asp Gln Val Glu Thr Gln Ala Cys Thr Arg
            100                 105                 110

gaa cag aac cgc atc tgc acc tgc agg ccc ggc tgg tac tgc gcg ctg      384
Glu Gln Asn Arg Ile Cys Thr Cys Arg Pro Gly Trp Tyr Cys Ala Leu
        115                 120                 125

agc aag cag gag ggg tgc cgg ctg tgc gcg ccg ctg cgc aag tgc cgc      432

Ser Lys Gln Glu Gly Cys Arg Leu Cys Ala Pro Leu Arg Lys Cys Arg
    130                 135                 140

ccg ggc ttc ggc gtg gcc aga cca gga act gaa aca tca gac gtg gtg      480
Pro Gly Phe Gly Val Ala Arg Pro Gly Thr Glu Thr Ser Asp Val Val
145                 150                 155                 160

tgc aag ccc tgt gcc ccg ggg acg ttc tcc aac acg act tca tcc acg      528
Cys Lys Pro Cys Ala Pro Gly Thr Phe Ser Asn Thr Thr Ser Ser Thr
                165                 170                 175

gat att tgc agg ccc cac cag atc tgt aac gtg gtg gcc atc cct ggg      576
Asp Ile Cys Arg Pro His Gln Ile Cys Asn Val Val Ala Ile Pro Gly
            180                 185                 190

aat gca agc atg gat gca gtc tgc acg tcc acg tcc ccc acc cgg agt      624
Asn Ala Ser Met Asp Ala Val Cys Thr Ser Thr Ser Pro Thr Arg Ser
        195                 200                 205

atg gcc cca ggg gca gta cac tta ccc cag cca gtg tcc aca cga tcc      672
Met Ala Pro Gly Ala Val His Leu Pro Gln Pro Val Ser Thr Arg Ser
    210                 215                 220

caa cac acg cag cca act cca gaa ccc agc act gct cca agc acc tcc      720
Gln His Thr Gln Pro Thr Pro Glu Pro Ser Thr Ala Pro Ser Thr Ser
225                 230                 235                 240

ttc ctg ctc cca atg ggc ccc agc ccc cca gct gaa ggg agc act ggc      768
Phe Leu Leu Pro Met Gly Pro Ser Pro Pro Ala Glu Gly Ser Thr Gly
                245                 250                 255

gac gag ccc aaa tct tgt gac aaa act cac aca tgc cca ccg tgc cca      816
Asp Glu Pro Lys Ser Cys Asp Lys Thr His Thr Cys Pro Pro Cys Pro
            260                 265                 270

gca cct gaa ctc ctg ggg gga ccg tca gtc ttc ctc ttc ccc cca aaa      864
Ala Pro Glu Leu Leu Gly Gly Pro Ser Val Phe Leu Phe Pro Pro Lys
        275                 280                 285

ccc aag gac acc ctc atg atc tcc cgg acc cct gag gtc aca tgc gtg      912
Pro Lys Asp Thr Leu Met Ile Ser Arg Thr Pro Glu Val Thr Cys Val
    290                 295                 300

gtg gtg gac gtg agc cac gaa gac cct gag gtc aag ttc aac tgg tac      960
Val Val Asp Val Ser His Glu Asp Pro Glu Val Lys Phe Asn Trp Tyr
305                 310                 315                 320

gtg gac ggc gtg gag gtg cat aat gcc aag aca aag ccg cgg gag gag     1008
Val Asp Gly Val Glu Val His Asn Ala Lys Thr Lys Pro Arg Glu Glu
                325                 330                 335

cag tac aac agc acg tac cgt gtg gtc agc gtc ctc acc gtc ctg cac     1056
Gln Tyr Asn Ser Thr Tyr Arg Val Val Ser Val Leu Thr Val Leu His
            340                 345                 350

cag gac tgg ctg aat ggc aag gag tac aag tgc aag gtc tcc aac aaa     1104
Gln Asp Trp Leu Asn Gly Lys Glu Tyr Lys Cys Lys Val Ser Asn Lys
        355                 360                 365

gcc ctc cca gcc ccc atc gag aaa acc atc tcc aaa gcc aaa ggg cag     1152
Ala Leu Pro Ala Pro Ile Glu Lys Thr Ile Ser Lys Ala Lys Gly Gln
    370                 375                 380

ccc cga gaa cca cag gtg tac acc ctg ccc cca tcc cgg gag gag atg 1200
Pro Arg Glu Pro Gln Val Tyr Thr Leu Pro Pro Ser Arg Glu Glu Met
385                 390                 395                 400

acc aag aac cag gtc agc ctg acc tgc ctg gtc aaa ggc ttc tat ccc 1248
Thr Lys Asn Gln Val Ser Leu Thr Cys Leu Val Lys Gly Phe Tyr Pro
                405                 410                 415

agc gac atc gcc gtg gag tgg gag agc aat ggg cag ccg gag aac aac 1296
Ser Asp Ile Ala Val Glu Trp Glu Ser Asn Gly Gln Pro Glu Asn Asn
            420                 425                 430

tac aag acc acg cct ccc gtg ctg gac tcc gac ggc tcc ttc ttc ctc 1344
Tyr Lys Thr Thr Pro Pro Val Leu Asp Ser Asp Gly Ser Phe Phe Leu
        435                 440                 445

tac agc aag ctc acc gtg gac aag agc agg tgg cag cag ggg aac gtc 1392
Tyr Ser Lys Leu Thr Val Asp Lys Ser Arg Trp Gln Gln Gly Asn Val
    450                 455                 460

ttc tca tgc tcc gtg atg cat gag gct ctg cac aac cac tac aca cag 1440
Phe Ser Cys Ser Val Met His Glu Ala Leu His Asn His Tyr Thr Gln
465                 470                 475                 480

aag agc ctc tcc ctg tct ccg ggt aaa tga 1470
Lys Ser Leu Ser Leu Ser Pro Gly Lys
                485

<210> 96
<211> 489
<212> PRT
<213> Synthetic sequence

<400> 96

Met Ala Pro Val Ala Val Trp Ala Ala Leu Ala Val Gly Leu Glu Leu
1               5                   10                  15

Trp Ala Ala Ala His Ala Leu Pro Ala Gln Val Ala Phe Thr Pro Tyr
            20                  25                  30

Ala Pro Glu Pro Gly Ser Thr Cys Arg Leu Arg Glu Tyr Tyr Asp Gln
        35                  40                  45

Thr Ala Gln Met Cys Cys Ser Lys Cys Ser Pro Gly Gln His Ala Lys
    50                  55                  60

Val Phe Cys Thr Lys Thr Ser Asp Thr Val Cys Asp Ser Cys Glu Asp
65                  70                  75                  80

Ser Thr Tyr Thr Gln Leu Trp Asn Trp Val Pro Glu Cys Leu Ser Cys
                85                  90                  95

Gly Ser Arg Cys Ser Ser Asp Gln Val Glu Thr Gln Ala Cys Thr Arg
            100                 105                 110

Glu Gln Asn Arg Ile Cys Thr Cys Arg Pro Gly Trp Tyr Cys Ala Leu
        115                 120                 125

Ser Lys Gln Glu Gly Cys Arg Leu Cys Ala Pro Leu Arg Lys Cys Arg
    130                 135                 140

Pro Gly Phe Gly Val Ala Arg Pro Gly Thr Glu Thr Ser Asp Val Val
145                 150                 155                 160

Cys Lys Pro Cys Ala Pro Gly Thr Phe Ser Asn Thr Thr Ser Ser Thr
                165                 170                 175

Asp Ile Cys Arg Pro His Gln Ile Cys Asn Val Val Ala Ile Pro Gly
            180                 185                 190

Asn Ala Ser Met Asp Ala Val Cys Thr Ser Thr Ser Pro Thr Arg Ser
        195                 200                 205

Met Ala Pro Gly Ala Val His Leu Pro Gln Pro Val Ser Thr Arg Ser
    210                 215                 220

Gln His Thr Gln Pro Thr Pro Glu Pro Ser Thr Ala Pro Ser Thr Ser
225                 230                 235                 240

Phe Leu Leu Pro Met Gly Pro Ser Pro Pro Ala Glu Gly Ser Thr Gly
                245                 250                 255

Asp Glu Pro Lys Ser Cys Asp Lys Thr His Thr Cys Pro Pro Cys Pro
            260                 265                 270

Ala Pro Glu Leu Leu Gly Gly Pro Ser Val Phe Leu Phe Pro Pro Lys
        275                 280                 285

Pro Lys Asp Thr Leu Met Ile Ser Arg Thr Pro Glu Val Thr Cys Val
    290                 295                 300

Val Val Asp Val Ser His Glu Asp Pro Glu Val Lys Phe Asn Trp Tyr
305                 310                 315                 320

Val Asp Gly Val Glu Val His Asn Ala Lys Thr Lys Pro Arg Glu Glu
                325                 330                 335

Gln Tyr Asn Ser Thr Tyr Arg Val Val Ser Val Leu Thr Val Leu His
            340                 345                 350

Gln Asp Trp Leu Asn Gly Lys Glu Tyr Lys Cys Lys Val Ser Asn Lys
        355                 360                 365

Ala Leu Pro Ala Pro Ile Glu Lys Thr Ile Ser Lys Ala Lys Gly Gln
    370                 375                 380

Pro Arg Glu Pro Gln Val Tyr Thr Leu Pro Pro Ser Arg Glu Glu Met
385                 390                 395                 400

Thr Lys Asn Gln Val Ser Leu Thr Cys Leu Val Lys Gly Phe Tyr Pro
                405                 410                 415

Ser Asp Ile Ala Val Glu Trp Glu Ser Asn Gly Gln Pro Glu Asn Asn
            420                 425                 430

Tyr Lys Thr Thr Pro Pro Val Leu Asp Ser Asp Gly Ser Phe Phe Leu
        435                 440                 445

Tyr Ser Lys Leu Thr Val Asp Lys Ser Arg Trp Gln Gln Gly Asn Val
    450                 455                 460

Phe Ser Cys Ser Val Met His Glu Ala Leu His Asn His Tyr Thr Gln
465                 470                 475                 480

Lys Ser Leu Ser Leu Ser Pro Gly Lys
                485

<210>  97
<211>  30
<212>  DNA
<213>  Synthetic sequence

<220>
<221>  primer_bind
<222>  (1)..(30)
<223>  Vector_Mam-FW primer

<400>  97
attaagcuag tgagtcgaat aagggcgaca                                        30

<210>  98
<211>  121
<212>  PRT
<213>  Homo sapiens

<220>
<221>  PEPTIDE
<222>  (1)..(121)
<223>  Adalimumab Heavy Chain Variable domain

<400>  98

<210>  99
<211>  107
<212>  PRT
<213>  Homo sapiens

<220>
<221>  PEPTIDE
<222>  (1)..(107)
<223>  Adalimumab Light Chain Variable domain

<400>  99

<210>  100
<211>  214
<212>  PRT
<213>  Homo sapiens

<220>
<221>  mat_peptide
<222>  (1)..(214)
<223>  Alemtuzumab complete Light Chain

<400>  100

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asn Ile Asp Lys Tyr
            20                  25                  30

Leu Asn Trp Tyr Gln Gln Lys Pro Gly Lys Ala Pro Lys Leu Leu Ile
        35                  40                  45

Tyr Asn Thr Asn Asn Leu Gln Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Leu Gln His Ile Ser Arg Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser

165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  101
<211>  219
<212>  PRT
<213>  Homo sapiens

<220>
<221>  mat_peptide
<222>  (1)..(219)
<223>  Alemtuzumab complete Heavy Chain

<400>  101

Gln Val Gln Leu Gln Glu Ser Gly Pro Gly Leu Val Arg Pro Ser Gln
1               5                   10                  15

Thr Leu Ser Leu Thr Cys Thr Val Ser Gly Phe Thr Phe Thr Asp Phe
            20                  25                  30

Tyr Met Asn Trp Val Arg Gln Pro Pro Gly Arg Gly Leu Glu Trp Ile
        35                  40                  45

Gly Phe Ile Arg Asp Lys Ala Lys Gly Tyr Thr Thr Glu Tyr Asn Pro
    50                  55                  60

Ser Val Lys Gly Arg Val Thr Met Leu Val Asp Thr Ser Lys Asn Gln
65                  70                  75                  80

Phe Ser Leu Arg Leu Ser Ser Val Thr Ala Ala Asp Thr Ala Val Tyr
                85                  90                  95

Tyr Cys Ala Arg Glu Gly His Thr Ala Ala Pro Phe Asp Tyr Trp Gly
            100                 105                 110

Gln Gly Ser Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val
    210                 215

<210>  102
<211>  116
<212>  PRT
<213>  Homo sapiens

<220>
<221>  PEPTIDE
<222>  (1)..(116)
<223>  Basiliximab Heavy Chain Variable domain

<400>  102

Gln Leu Gln Gln Ser Gly Thr Val Leu Ala Arg Pro Gly Ala Ser Val
1               5                   10                  15

Lys Met Ser Cys Lys Ala Ser Gly Tyr Ser Phe Thr Arg Tyr Trp Met
            20                  25                  30

His Trp Ile Lys Gln Arg Pro Gly Gln Gly Leu Glu Trp Ile Gly Ala
        35                  40                  45

Ile Tyr Pro Gly Asn Ser Asp Thr Ser Tyr Asn Gln Lys Phe Glu Gly
    50                  55                  60

Lys Ala Lys Leu Thr Ala Val Thr Ser Ala Ser Thr Ala Tyr Met Glu
65                  70                  75                  80

Leu Ser Ser Leu Thr His Glu Asp Ser Ala Val Tyr Tyr Cys Ser Arg
                85                  90                  95

Asp Tyr Gly Tyr Tyr Phe Asp Phe Trp Gly Gln Gly Thr Thr Leu Thr
            100                 105                 110

Val Ser Ser Ala

        115

<210>  103
<211>  105
<212>  PRT
<213>  Homo sapiens

<220>
<221>  PEPTIDE
<222>  (1)..(105)
<223>  Basiliximab Light Chain Variable domain

<400>  103

Gln Ile Val Ser Thr Gln Ser Pro Ala Ile Met Ser Ala Ser Pro Gly
1               5                   10                  15

Glu Lys Val Thr Met Thr Cys Ser Ala Ser Ser Ser Arg Ser Tyr Met
            20                  25                  30

Gln Trp Tyr Gln Gln Lys Pro Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys His Gln Arg Ser Ser Tyr Thr Phe Gly
                85                  90                  95

Gly Gly Thr Lys Leu Glu Ile Lys Arg
            100                 105

<210>  104
<211>  124
<212>  PRT
<213>  Homo sapiens

<220>
<221>  PEPTIDE
<222>  (1)..(124)
<223>  Belimumab Heavy Chain Variable domain

<400>  104

Gln Val Gln Leu Gln Gln Ser Gly Ala Glu Val Lys Lys Pro Gly Ser
1               5                   10                  15

Ser Val Arg Val Ser Cys Lys Ala Ser Gly Gly Thr Phe Asn Asn Asn
            20                  25                  30

Ala Ile Asn Trp Val Arg Gln Ala Pro Gly Gln Gly Leu Glu Trp Met
        35                  40                  45

Gly Gly Ile Ile Pro Met Phe Gly Thr Ala Lys Tyr Ser Gln Asn Phe
    50                  55                  60

Gln Gly Arg Val Ala Ile Thr Ala Asp Glu Ser Thr Gly Thr Ala Ser
65                  70                  75                  80

Met Glu Leu Ser Ser Leu Arg Ser Glu Asp Thr Ala Val Tyr Tyr Cys
                85                  90                  95

Ala Arg Ser Arg Asp Leu Leu Leu Phe Pro His His Ala Leu Ser Pro
            100                 105                 110

Trp Gly Arg Gly Thr Met Val Thr Val Ser Ser Gly
        115                 120

<210> 105
<211> 109
<212> PRT
<213> Homo sapiens

<220>
<221> PEPTIDE
<222> (1)..(109)
<223> Belimumab Light Chain Variable domain

<400> 105

Ser Ser Glu Leu Thr Gln Asp Pro Ala Val Ser Val Ala Leu Gly Gln
1               5                   10                  15

Thr Val Arg Val Thr Cys Gln Gly Asp Ser Leu Arg Ser Tyr Tyr Ala
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Asp Tyr Tyr Cys Ser Ser Arg Asp Ser Ser Gly Asn His
                85                  90                  95

Trp Val Phe Gly Gly Gly Thr Glu Leu Thr Val Leu Gly
            100                 105

<210>  106
<211>  231
<212>  PRT
<213>  Homo sapiens

<220>
<221>  mat_peptide
<222>  (1)..(231)
<223>  Bevacizumab complete Heavy Chain

<400>  106

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr
225                 230

<210>  107
<211>  214
<212>  PRT
<213>  Homo sapiens

<220>
<221>  mat_peptide
<222>  (1)..(214)
<223>  Bevacizumab complete Light Chain

<400>  107

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  108
<211>  118
<212>  PRT
<213>  Homo sapiens

<220>
<221>  PEPTIDE
<222>  (1)..(118)
<223>  Canakinumab Heavy Chain Variable domain

<400>  108

<210>  109
<211>  107
<212>  PRT
<213>  Homo sapiens

<220>
<221>  PEPTIDE
<222>  (1)..(107)
<223>  Canakinumab Light Chain Variable domain

<400>  109

<210>  110
<211>  108
<212>  PRT
<213>  Homo sapiens

<220>
<221>  PEPTIDE
<222>  (1)..(108)
<223>  Cetuximab Heavy Chain Variable domain

<400>  110

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys Arg
            100                 105

<210>  111
<211>  120
<212>  PRT
<213>  Homo sapiens

<220>
<221>  PEPTIDE
<222>  (1)..(120)
<223>  Cetuximab Light Chain Variable domain

<400>  111

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ala Ala

115                 120

<210>  112
<211>  215
<212>  PRT
<213>  Homo sapiens

<220>
<221>  mat_peptide
<222>  (1)..(215)
<223>  Clivatuzumab complete Heavy Chain

<400>  112

Asp Ile Gln Leu Thr Gln Ser Pro Ser Ser Leu Ser Ala Ser Val Gly
1               5                   10                  15

Asp Arg Val Thr Met Thr Cys Ser Ala Ser Ser Ser Val Ser Ser Ser
            20                  25                  30

Tyr Leu Tyr Trp Tyr Gln Gln Lys Pro Gly Lys Ala Pro Lys Leu Trp
        35                  40                  45

Ile Tyr Ser Thr Ser Asn Leu Ala Ser Gly Val Pro Ala Arg Phe Ser
    50                  55                  60

Gly Ser Gly Ser Gly Thr Asp Phe Thr Leu Thr Ile Ser Ser Leu Gln
65                  70                  75                  80

Pro Glu Asp Ser Ala Ser Tyr Phe Cys His Gln Trp Asn Arg Tyr Pro
                85                  90                  95

Tyr Thr Phe Gly Gly Gly Thr Arg Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  113
<211>  449
<212>  PRT
<213>  Homo sapiens

<220>
<221>  mat_peptide
<222>  (1)..(449)
<223>  Clivatuzumab complete Light Chain

<400>  113

Gln Val Gln Leu Gln Gln Ser Gly Ala Glu Val Lys Lys Pro Gly Ala
1               5                   10                  15

Ser Val Lys Val Ser Cys Glu Ala Ser Gly Tyr Thr Phe Pro Ser Tyr
            20                  25                  30

Val Leu His Trp Val Lys Gln Ala Pro Gly Gln Gly Leu Glu Trp Ile
        35                  40                  45

Gly Tyr Ile Asn Pro Tyr Asn Asp Gly Thr Gln Tyr Asn Glu Lys Phe
    50                  55                  60

Lys Gly Lys Ala Thr Leu Thr Arg Asp Thr Ser Ile Asn Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Phe Gly Gly Ser Tyr Gly Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu

165                 170                 175

Gln Ser Ser Gly Leu Tyr Ser Leu Ser Ser Val Val Thr Val Pro Ser
        180                 185                 190

Ser Ser Leu Gly Thr Gln Thr Tyr Ile Cys Asn Val Asn His Lys Pro
    195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
        260                 265                 270

Pro Glu Val Lys Phe Asn Trp Tyr Val Asp Gly Val Glu Val His Asn
    275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
        340                 345                 350

Leu Pro Pro Ser Arg Glu Glu Met Thr Lys Asn Gln Val Ser Leu Thr
    355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210> 114
<211> 213
<212> PRT
<213> Homo sapiens

<220>
<221> mat_peptide
<222> (1)..(213)
<223> Daclizumab complete Light Chain

<400> 114

Asp Ile Gln Met Thr Gln Ser Pro Ser Thr Leu Ser Ala Ser Val Gly
1               5                   10                  15

Asp Arg Val Thr Ile Thr Cys Ser Ala Ser Ser Ser Ile Ser Tyr Met
            20                  25                  30

His Trp Tyr Gln Gln Lys Pro Gly Lys Ala Pro Lys Leu Leu Ile Tyr
        35                  40                  45

Thr Thr Ser Asn Leu Ala Ser Gly Val Pro Ala Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys His Gln Arg Ser Thr Tyr Pro Leu Thr
                85                  90                  95

Phe Gly Gln Gly Thr Lys Val Glu Val Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  115
<211>  446
<212>  PRT
<213>  Homo sapiens

<220>
<221>  mat_peptide
<222>  (1)..(446)
<223>  Daclizumab complete Heavy Chain

<400>  115

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Ser Tyr
            20                  25                  30

Arg Met His Trp Val Arg Gln Ala Pro Gly Gln Gly Leu Glu Trp Ile
        35                  40                  45

Gly Tyr Ile Asn Pro Ser Thr Gly Tyr Thr Glu Tyr Asn Gln Lys Phe
    50                  55                  60

Lys Asp Lys Ala Thr Ile Thr Ala Asp Glu Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gly Val Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Asp Glu Leu Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  116
<211>  122
<212>  PRT
<213>  Homo sapiens

<220>
<221>  PEPTIDE
<222>  (1)..(122)
<223>  Denosumab Heavy Chain variable domain

<400>  116

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Gly Ile Thr Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Pro Gly Thr Thr Val Ile Met Ser Trp Phe Asp Pro Trp
            100                 105                 110

Gly Gln Gly Thr Leu Val Thr Val Ser Ser
        115                 120

<210>  117

<211>  108
<212>  PRT
<213>  Homo sapiens

<220>
<221>  PEPTIDE
<222>  (1)..(108)
<223>  Denosumab Light Chain variable domain

<400>  117

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Arg Gly Arg
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Phe Tyr Cys Gln Gln Tyr Gly Ser Ser Pro
                85                  90                  95

Arg Thr Phe Gly Gln Gly Thr Lys Val Glu Ile Lys
            100                 105

<210>  118
<211>  448
<212>  PRT
<213>  Homo sapiens

<220>
<221>  mat_peptide
<222>  (1)..(448)
<223>  Eculizumab complete Heavy Chain

<400>  118

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ile Phe Ser Asn Tyr
            20                  25                  30

Trp Ile Gln Trp Val Arg Gln Ala Pro Gly Gln Gly Leu Glu Trp Met
        35                  40                  45

Gly Glu Ile Leu Pro Gly Ser Gly Ser Thr Glu Tyr Thr Glu Asn Phe
    50                  55                  60

Lys Asp Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Tyr Phe Phe Gly Ser Ser Pro Asn Trp Tyr Phe Asp Val Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Asn Phe Gly Thr Gln Thr Tyr Thr Cys Asn Val Asp
        195                 200                 205

His Lys Pro Ser Asn Thr Lys Val Asp Lys Thr Val Glu Arg Lys Cys
    210                 215                 220

Cys Val Glu Cys Pro Pro Cys Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Tyr Arg Val Val

290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Gln Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
        435                 440                 445

<210>  119
<211>  214
<212>  PRT
<213>  Homo sapiens

<220>
<221>  mat_peptide
<222>  (1)..(214)
<223>  Eculizumab complete Light Chain

<400>  119

<210>  120
<211>  108
<212>  PRT
<213>  Homo sapiens

<220>
<221>  PEPTIDE
<222>  (1)..(108)
<223>  Efalizumab Light Chain variable domain

<400>  120

Asp Ile Gln Met Thr Gln Ser Pro Ser Ser Leu Ser Ala Ser Val Gly
1               5                   10                  15

Asp Arg Val Thr Ile Thr Cys Arg Ala Ser Lys Thr Ile Ser Lys Tyr

20                  25                  30

Leu Ala Trp Tyr Gln Gln Lys Pro Gly Lys Ala Pro Lys Leu Leu Ile
        35                  40                  45

Tyr Ser Gly Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln His Asn Glu Tyr Pro Leu
                85                  90                  95

Thr Phe Gly Gln Gly Thr Lys Val Glu Ile Lys Arg
            100                 105

<210>  121
<211>  122
<212>  PRT
<213>  Homo sapiens

<220>
<221>  PEPTIDE
<222>  (1)..(122)
<223>  Efalizumab Heavy Chain variable domain

<400>  121

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly His
            20                  25                  30

Trp Met Asn Trp Val Arg Gln Ala Pro Gly Lys Gly Leu Glu Trp Val
        35                  40                  45

Gly Met Ile His Pro Ser Asp Ser Glu Thr Arg Tyr Asn Gln Lys Phe
    50                  55                  60

Lys Asp Arg Phe Thr Ile Ser Val Asp Lys Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ile Tyr Phe Tyr Gly Thr Thr Tyr Phe Asp Tyr Trp Gly
            100                 105                 110

Gln Gly Thr Leu Val Thr Val Ser Ser Ala
        115                 120

<210>  122
<211>  447
<212>  PRT
<213>  Homo sapiens

<220>
<221>  mat_peptide
<222>  (1)..(447)
<223>  Etaracizumab complete Heavy Chain

<400>  122

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ala Lys Val Ser Ser Gly Gly Gly Ser Thr Tyr Tyr Leu Asp Thr Val
    50                  55                  60

Gln Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Leu His Gly Ser Phe Ala Ser Trp Gly Gln Gly Thr Thr
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser

            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210>  123
<211>  214
<212>  PRT
<213>  Homo sapiens

<220>
<221>  mat_peptide
<222>  (1)..(214)
<223>  Etaracizumab complete Light Chain

<400>  123

Glu Ile Val Leu Thr Gln Ser Pro Ala Thr Leu Ser Leu Ser Pro Gly
1               5                   10                  15

Glu Arg Ala Thr Leu Ser Cys Gln Ala Ser Gln Ser Ile Ser Asn Phe
            20                  25                  30

Leu His Trp Tyr Gln Gln Arg Pro Gly Gln Ala Pro Arg Leu Leu Ile
        35                  40                  45

Arg Tyr Arg Ser Gln Ser Ile Ser Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Gly Ser Trp Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  124
<211>  214
<212>  PRT
<213>  Homo sapiens

<220>
<221>  mat_peptide
<222>  (1)..(214)
<223>  Fontolizumab complete Light Chain

<400>  124

Asp Ile Gln Met Thr Gln Ser Pro Ser Thr Leu Ser Ala Ser Val Gly
1               5                   10                  15

Asp Arg Val Thr Ile Thr Cys Lys Ala Ser Glu Asn Val Asp Thr Tyr
            20                  25                  30

Val Ser Trp Tyr Gln Gln Lys Pro Gly Lys Ala Pro Lys Leu Leu Ile
        35                  40                  45

Tyr Gly Ala Ser Asn Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Asp Asp Phe Ala Thr Tyr Tyr Cys Gly Gln Ser Tyr Asn Tyr Pro Phe
                85                  90                  95

Thr Phe Gly Gln Gly Thr Lys Val Glu Val Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  125
<211>  447
<212>  PRT
<213>  Homo sapiens

<220>
<221>  mat_peptide
<222>  (1)..(447)
<223>  Fontolizumab complete Heavy Chain

<400>  125

Gln Val Gln Leu Val Gln Ser Gly Ala Glu Leu Lys Lys Pro Gly Ser
1               5                   10                  15

Ser Val Lys Val Ser Cys Lys Ala Ser Gly Tyr Ile Phe Thr Ser Ser
            20                  25                  30

Trp Ile Asn Trp Val Lys Gln Ala Pro Gly Gln Gly Leu Glu Trp Ile
        35                  40                  45

Gly Arg Ile Asp Pro Ser Asp Gly Glu Val His Tyr Asn Gln Asp Phe
    50                  55                  60

Lys Asp Lys Ala Thr Leu Thr Val Asp Lys Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Phe Leu Pro Trp Phe Ala Asp Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser

385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210>  126
<211>  214
<212>  PRT
<213>  Homo sapiens

<220>
<221>  mat_peptide
<222>  (1)..(214)
<223>  Girentuximab complete Light Chain

<400>  126

Asp Ile Val Met Thr Gln Ser Gln Arg Phe Met Ser Thr Thr Val Gly
1               5                   10                  15

Asp Arg Val Ser Ile Thr Cys Lys Ala Ser Gln Asn Val Val Ser Ala
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Phe Phe Cys Gln Gln Tyr Ser Asn Tyr Pro Trp
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  127
<211>  449
<212>  PRT
<213>  Homo sapiens

<220>
<221>  mat_peptide
<222>  (1)..(449)
<223>  Girentuximab complete Heavy Chain

<400>  127

Asp Val Lys Leu Val Glu Ser Gly Gly Gly Leu Val Lys Leu Gly Gly
1               5                   10                  15

Ser Leu Lys Leu Ser Cys Ala Ala Ser Gly Phe Thr Phe Ser Asn Tyr
            20                  25                  30

Tyr Met Ser Trp Val Arg Gln Thr Pro Glu Lys Arg Leu Glu Leu Val
        35                  40                  45

Ala Ala Ile Asn Ser Asp Gly Gly Ile Thr Tyr Tyr Leu Asp Thr Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Leu Phe Tyr Cys
                85                  90                  95

Ala Arg His Arg Ser Gly Tyr Phe Ser Met Asp Tyr Trp Gly Gln Gly
            100                 105                 110

Thr Ser Val Thr Val Ser Ser Ala Ser Thr Lys Gly Pro Ser Val Phe

115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  128
<211>  126
<212>  PRT
<213>  Homo sapiens

<220>
<221>  PEPTIDE
<222>  (1)..(126)
<223>  Golimumab Heavy Chain variable domain

<400>  128

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Ile Phe Ser Ser Tyr
            20                  25                  30

Ala Met His Trp Val Arg Gln Ala Pro Gly Asn Gly Leu Glu Trp Val
        35                  40                  45

Ala Phe Met Ser Tyr Asp Gly Ser Asn Lys Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Ile Ala Ala Gly Gly Asn Tyr Tyr Tyr Tyr Gly
            100                 105                 110

Met Asp Val Trp Gly Gln Gly Thr Thr Val Thr Val Ser Ser
        115                 120                 125

<210>  129
<211>  108
<212>  PRT
<213>  Homo sapiens

<220>
<221>  PEPTIDE
<222>  (1)..(108)
<223>  Golimumab Light Chain variable domain

<400>  129

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Tyr Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Pro
                85                  90                  95

Phe Thr Phe Gly Pro Gly Thr Lys Val Asp Ile Lys
            100                 105

<210>  130
<211>  121
<212>  PRT
<213>  Homo sapiens

<220>
<221>  PEPTIDE
<222>  (1)..(121)
<223>  Ibritumomab tiuxetan Heavy Chain variable domain

<400>  130

Gln Val Gln Leu Gln Gln Pro Gly Ala Glu Leu Val Lys Ala Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser Ala
        115                 120

<210>  131
<211>  106
<212>  PRT
<213>  Homo sapiens

<220>
<221>  PEPTIDE
<222>  (1)..(106)
<223>  Ibritumomab tiuxetan Light Chain variable domain

<400>  131

<210>  132
<211>  119
<212>  PRT
<213>  Homo sapiens

<220>
<221>  PEPTIDE
<222>  (1)..(119)
<223>  Infliximab Heavy Chain variable domain

<400>  132

Glu Val Lys Leu Glu Glu Ser Gly Gly Gly Leu Val Gln Pro Gly Gly
1               5                   10                  15

Ser Met Lys Leu Ser Cys Val Ala Ser Gly Phe Ile Phe Ser Asn His
            20                  25                  30

Trp Met Asn Trp Val Arg Gln Ser Pro Glu Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ala
65                  70                  75                  80

Val Tyr Leu Gln Met Thr Asp Leu Arg Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Leu Thr Val Ser
        115

<210>  133
<211>  107
<212>  PRT
<213>  Homo sapiens

<220>
<221>  PEPTIDE
<222>  (1)..(107)
<223>  Infliximab Light Chain variable domain

<400>  133

Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Phe Val Gly Ser Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Met Ser Gly Ile Pro Ser Arg Phe Ser Gly
    50                  55                  60

Ser Gly Ser Gly Thr Asp Phe Thr Leu Ser Ile Asn Thr Val Glu Ser
65                  70                  75                  80

Glu Asp Ile Ala Asp Tyr Tyr Cys Gln Gln Ser His Ser Trp Pro Phe
                85                  90                  95

Thr Phe Gly Ser Gly Thr Asn Leu Glu Val Lys
            100                 105

<210>  134
<211>  118
<212>  PRT
<213>  Homo sapiens

<220>
<221>  PEPTIDE
<222>  (1)..(118)
<223>  Ipilimumab Heavy Chain variable domain

<400>  134

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Thr Met His Trp Val Arg Gln Ala Pro Gly Lys Gly Leu Glu Trp Val
        35                  40                  45

Thr Phe Ile Ser Tyr Asp Gly Asn Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ile Tyr Tyr Cys
                85                  90                  95

Ala Arg Thr Gly Trp Leu Gly Pro Phe Asp Tyr Trp Gly Gln Gly Thr
            100                 105                 110

Leu Val Thr Val Ser Ser
        115

<210>  135
<211>  108
<212>  PRT
<213>  Homo sapiens

<220>
<221>  PEPTIDE
<222>  (1)..(108)
<223>  Ipilimumab Light Chain variable domain

<400>  135

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Gly Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Phe Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Trp Thr Phe Gly Gln Gly Thr Lys Val Glu Ile Lys
            100                 105

<210>  136
<211>  449
<212>  PRT
<213>  Homo sapiens

<220>
<221>  mat_peptide
<222>  (1)..(449)
<223>  Labetuzumab complete Heavy Chain

<400>  136

Glu Val Gln Leu Val Glu Ser Gly Gly Gly Val Val Gln Pro Gly Arg
1               5                   10                  15

Ser Leu Arg Leu Ser Cys Ser Ala Ser Gly Phe Asp Phe Thr Thr Tyr
            20                  25                  30

Trp Met Ser Trp Val Arg Gln Ala Pro Gly Lys Gly Leu Glu Trp Ile
        35                  40                  45

Gly Glu Ile His Pro Asp Ser Ser Thr Ile Asn Tyr Ala Pro Ser Leu
    50                  55                  60

Lys Asp Arg Phe Thr Ile Ser Arg Asp Asn Ala Lys Asn Thr Leu Phe
65                  70                  75                  80

Leu Gln Met Asp Ser Leu Arg Pro Glu Asp Thr Gly Val Tyr Phe Cys
                85                  90                  95

Ala Ser Leu Tyr Phe Gly Phe Pro Trp Phe Ala Tyr Trp Gly Gln Gly
            100                 105                 110

Thr Pro Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  137
<211>  213
<212>  PRT
<213>  Homo sapiens

<220>

<221>  mat_peptide
<222>  (1)..(213)
<223>  Labetuzumab complete Light Chain

<400>  137

Asp Ile Gln Leu Thr Gln Ser Pro Ser Ser Leu Ser Ala Ser Val Gly
1               5                   10                  15

Asp Arg Val Thr Ile Thr Cys Lys Ala Ser Gln Asp Val Gly Thr Ser
            20                  25                  30

Val Ala Trp Tyr Gln Gln Lys Pro Gly Lys Ala Pro Lys Leu Leu Ile
        35                  40                  45

Tyr Trp Thr Ser Thr Arg His Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Ser Leu Tyr Arg Ser
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  138

<211>  119
<212>  PRT
<213>  Homo sapiens

<220>
<221>  PEPTIDE
<222>  (1)..(119)
<223>  Mepolizumab Heavy Chain variable domain

<400>  138

<210>  139
<211>  113
<212>  PRT
<213>  Homo sapiens

<220>
<221>  PEPTIDE
<222>  (1)..(113)
<223>  Mepolizumab Light Chain variable domain

<400>  139

<210> 140
<211> 213
<212> PRT
<213> Homo sapiens

<220>
<221> mat_peptide
<222> (1)..(213)
<223> Motavizumab complete Light Chain

<400> 140

Asp Ile Gln Met Thr Gln Ser Pro Ser Thr Leu Ser Ala Ser Val Gly
1               5                   10                  15

Asp Arg Val Thr Ile Thr Cys Ser Ala Ser Ser Arg Val Gly Tyr Met
            20                  25                  30

His Trp Tyr Gln Gln Lys Pro Gly Lys Ala Pro Lys Leu Leu Ile Tyr
        35                  40                  45

Asp Thr Ser Lys Leu Ala Ser Gly Val Pro Ser Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Phe Gln Gly Ser Gly Tyr Pro Phe Thr
                85                  90                  95

Phe Gly Gly Gly Thr Lys Val Glu Ile Lys Arg Thr Val Ala Ala Pro
                100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
                180                 185                 190

Cys Glu Val Thr His Gln Gly Leu Ser Ser Pro Val Thr Lys Ser Phe
        195                 200                 205

Asn Arg Gly Glu Cys
    210

<210>  141
<211>  450
<212>  PRT
<213>  Homo sapiens

<220>
<221>  mat_peptide
<222>  (1)..(450)
<223>  Motavizumab complete Heavy Chain

<400>  141

Gln Val Thr Leu Arg Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ala
                20                  25                  30

Gly Met Ser Val Gly Trp Ile Arg Gln Pro Pro Gly Lys Ala Leu Glu
        35                  40                  45

Trp Leu Ala Asp Ile Trp Trp Asp Asp Lys Lys His Tyr Asn Pro Ser
    50                  55                  60

Leu Lys Asp Arg Leu Thr Ile Ser Lys Asp Thr Ser Lys Asn Gln Val
65                  70                  75                  80

Val Leu Lys Val Thr Asn Met Asp Pro Ala Asp Thr Ala Thr Tyr Tyr
                85                  90                  95

Cys Ala Arg Asp Met Ile Phe Asn Phe Tyr Phe Asp Val Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu

325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  142
<211>  106
<212>  PRT
<213>  Homo sapiens

<220>
<221>  PEPTIDE
<222>  (1)..(106)
<223>  Natalizumab Light Chain variable domain

<400>  142

Ser Ile Val Met Thr Gln Thr Pro Lys Phe Leu Leu Val Ser Ala Gly
1               5                   10                  15

Asp Arg Val Thr Ile Thr Cys Lys Ala Ser Gln Ser Val Thr Asn Asp
            20                  25                  30

Val Ala Trp Tyr Gln Gln Lys Pro Gly Gln Ser Pro Lys Leu Leu Ile
        35                  40                  45

Tyr Tyr Ala Ser Asn Arg Tyr Thr Gly Val Pro Asp Arg Phe Thr Gly
    50                  55                  60

Ser Gly Tyr Gly Thr Asp Phe Thr Phe Thr Ile Ser Thr Val Gln Ala
65                  70                  75                  80

Glu Asp Leu Ala Val Tyr Phe Cys Gln Gln Asp Tyr Ser Ser Pro Tyr
                85                  90                  95

Thr Phe Gly Gly Gly Thr Lys Leu Glu Ile
            100                 105

<210>  143
<211>  120
<212>  PRT
<213>  Homo sapiens

<220>
<221>  PEPTIDE
<222>  (1)..(120)
<223>  Natalizumab Heavy Chain variable domain

<400>  143

Val Lys Leu Gln Gln Ser Gly Ala Glu Leu Val Lys Pro Gly Ala Ser
1               5                   10                  15

Val Lys Leu Phe Cys Thr Ala Ser Gly Phe Asn Ile Lys Asp Thr Tyr
            20                  25                  30

Met His Trp Val Lys Gln Arg Pro Gln Gln Gly Leu Glu Trp Ile Gly
        35                  40                  45

Arg Ile Asp Pro Ala Ser Gly Asp Thr Lys Tyr Asp Pro Lys Phe Gln
    50                  55                  60

Val Lys Ala Thr Ile Thr Ala Asp Thr Ser Ser Asn Thr Ala Trp Leu
65                  70                  75                  80

Gln Leu Ser Ser Leu Thr Ser Glu Asp Thr Ala Val Tyr Tyr Cys Ala
                85                  90                  95

Asp Gly Met Trp Val Ser Thr Gly Tyr Ala Leu Asp Phe Trp Gly Gln
            100                 105                 110

Gly Thr Thr Val Thr Val Ser Ser
        115                 120

<210>  144
<211>  113
<212>  PRT
<213>  Homo sapiens

<220>
<221>  PEPTIDE
<222>  (1)..(113)
<223>  Nimotuzumab Light Chain variable domain

<400>  144

Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Asn Ile Val His Ser
            20                  25                  30

Asn Gly Asn Thr Tyr Leu Asp Trp Tyr Gln Gln Thr Pro Gly Lys Ala
        35                  40                  45

Pro Lys Leu Leu Ile Tyr Lys Val Ser Asn Arg Phe Ser Gly Val Pro
    50                  55                  60

Ser Arg Phe Ser Gly Ser Gly Ser Gly Thr Asp Phe Thr Phe Thr Ile
65                  70                  75                  80

Ser Ser Leu Gln Pro Glu Asp Ile Ala Thr Tyr Tyr Cys Phe Gln Tyr
                85                  90                  95

Ser His Val Pro Trp Thr Phe Gly Gln Gly Thr Lys Leu Gln Ile Thr
            100                 105                 110

Arg

<210>  145
<211>  124
<212>  PRT
<213>  Homo sapiens

<220>
<221>  PEPTIDE
<222>  (1)..(124)
<223>  Nimotuzumab Heavy Chain variable domain

<400>  145

Gln Val Gln Leu Gln Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Tyr Ile Tyr Trp Val Arg Gln Ala Pro Gly Gln Gly Leu Glu Trp Ile
        35                  40                  45

Gly Gly Ile Asn Pro Thr Ser Gly Gly Ser Asn Phe Asn Glu Lys Phe
    50                  55                  60

Lys Thr Arg Val Thr Ile Thr Ala Asp Glu Ser Ser Thr Thr Ala Tyr
65                  70                  75                  80

Met Glu Leu Ser Ser Leu Arg Ser Glu Asp Thr Ala Phe Tyr Phe Cys
                85                  90                  95

Thr Arg Gln Gly Leu Trp Phe Asp Ser Asp Gly Arg Gly Phe Asp Phe
            100                 105                 110

Trp Gly Gln Gly Thr Thr Val Thr Val Ser Ser Ala
        115                 120

<210>  146
<211>  440
<212>  PRT
<213>  Homo sapiens

<220>
<221>  mat_peptide
<222>  (1)..(440)
<223>  Nivolumab complete Heavy Chain

<400>  146

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Lys
        435                 440

<210>  147
<211>  214
<212>  PRT
<213>  Homo sapiens

<220>
<221>  mat_peptide
<222>  (1)..(214)
<223>  Nivolumab complete Light Chain

<400>  147

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly

115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  148
<211>  448
<212>  PRT
<213>  Homo sapiens

<220>
<221>  mat_peptide
<222>  (1)..(448)
<223>  Obinutuzumab complete Heavy Chain

<400>  148

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

<210> 149
<211> 219
<212> PRT
<213> Homo sapiens

<220>
<221> mat_peptide
<222> (1)..(219)
<223> Obinutuzumab complete Light Chain

<400> 149

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  150
<211>  123
<212>  PRT
<213>  Homo sapiens

<220>
<221>  PEPTIDE
<222>  (1)..(123)
<223>  Ofatumumab Heavy Chain variable domain

<400>  150

Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
    50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser Ala
        115                 120

<210>  151
<211>  108
<212>  PRT
<213>  Homo sapiens

<220>
<221>  PEPTIDE
<222>  (1)..(108)
<223>  Ofatumumab Light Chain variable domain

<400>  151

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Ile
                85                  90                  95

Thr Phe Gly Gln Gly Thr Arg Leu Glu Ile Lys Arg
            100                 105

<210>  152
<211>  451
<212>  PRT
<213>  Homo sapiens

<220>
<221>  mat_peptide

<222>  (1)..(451)
<223>  Omalizumab complete Heavy Chain

<400>  152

Glu Val Gln Leu Val Glu Ser Gly Gly Gly Leu Val Gln Pro Gly Gly
1               5                   10                  15

Ser Leu Arg Leu Ser Cys Ala Val Ser Gly Tyr Ser Ile Thr Ser Gly
            20                  25                  30

Tyr Ser Trp Asn Trp Ile Arg Gln Ala Pro Gly Lys Gly Leu Glu Trp
        35                  40                  45

Val Ala Ser Ile Thr Tyr Asp Gly Ser Thr Asn Tyr Asn Pro Ser Val
    50                  55                  60

Lys Gly Arg Ile Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ser His Tyr Phe Gly His Trp His Phe Ala Val Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  153
<211>  218
<212>  PRT
<213>  Homo sapiens

<220>
<221>  mat_peptide
<222>  (1)..(218)
<223>  Omalizumab complete Light Chain

<400>  153

Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Val Asp Tyr Asp
            20                  25                  30

Gly Asp Ser Tyr Met Asn Trp Tyr Gln Gln Lys Pro Gly Lys Ala Pro
        35                  40                  45

Lys Leu Leu Ile Tyr Ala Ala Ser Tyr Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser His
                85                  90                  95

Glu Asp Pro Tyr Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  154
<211>  450
<212>  PRT
<213>  Homo sapiens

<220>
<221>  mat_peptide
<222>  (1)..(450)
<223>  Palivizumab complete Heavy Chain

<400>  154

Gln Val Thr Leu Arg Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Ser Val Gly Trp Ile Arg Gln Pro Pro Gly Lys Ala Leu Glu
        35                  40                  45

Trp Leu Ala Asp Ile Trp Trp Asp Asp Lys Lys Asp Tyr Asn Pro Ser
    50                  55                  60

Leu Lys Ser Arg Leu Thr Ile Ser Lys Asp Thr Ser Lys Asn Gln Val
65                  70                  75                  80

Val Leu Lys Val Thr Asn Met Asp Pro Ala Asp Thr Ala Thr Tyr Tyr
                85                  90                  95

Cys Ala Arg Ser Met Ile Thr Asn Trp Tyr Phe Asp Val Trp Gly Ala
            100                 105                 110

Gly Thr Thr Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys

195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450


<210>  155
<211>  213
<212>  PRT
<213>  Homo sapiens


<220>
<221>  mat_peptide
<222>  (1)..(213)
<223>  Palivizumab complete Light Chain

<400>  155

Asp Ile Gln Met Thr Gln Ser Pro Ser Thr Leu Ser Ala Ser Val Gly
1               5                   10                  15


Asp Arg Val Thr Ile Thr Cys Lys Cys Gln Leu Ser Val Gly Tyr Met
            20                  25                  30


His Trp Tyr Gln Gln Lys Pro Gly Lys Ala Pro Lys Leu Leu Ile Tyr
        35                  40                  45


Asp Thr Ser Lys Leu Ala Ser Gly Val Pro Ser Arg Phe Ser Gly Ser
    50                  55                  60


Gly Ser Gly Thr Glu Phe Thr Leu Thr Ile Ser Ser Leu Gln Pro Asp
65                  70                  75                  80


Asp Phe Ala Thr Tyr Tyr Cys Phe Gln Gly Ser Gly Tyr Pro Phe Thr
                85                  90                  95


Phe Gly Gly Gly Thr Lys Leu Glu Ile Lys Arg Thr Val Ala Ala Pro
            100                 105                 110


Ser Val Phe Ile Phe Pro Pro Ser Asp Glu Gln Leu Lys Ser Gly Thr
        115                 120                 125


Ala Ser Val Val Cys Leu Leu Asn Asn Phe Tyr Pro Arg Glu Ala Lys
    130                 135                 140


Val Gln Trp Lys Val Asp Asn Ala Leu Gln Ser Gly Asn Ser Gln Glu
145                 150                 155                 160


Ser Val Thr Glu Gln Asp Ser Lys Asp Ser Thr Tyr Ser Leu Ser Ser
                165                 170                 175


Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210> 156
<211> 445
<212> PRT
<213> Homo sapiens

<220>
<221> mat_peptide
<222> (1)..(445)
<223> Panitumumab complete Heavy Chain

<400> 156

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Asp Tyr Tyr Trp Thr Trp Ile Arg Gln Ser Pro Gly Lys Gly Leu Glu
        35                  40                  45

Trp Ile Gly His Ile Tyr Tyr Ser Gly Asn Thr Asn Tyr Asn Pro Ser
    50                  55                  60

Leu Lys Ser Arg Leu Thr Ile Ser Ile Asp Thr Ser Lys Thr Gln Phe
65                  70                  75                  80

Ser Leu Lys Leu Ser Ser Val Thr Ala Ala Asp Thr Ala Ile Tyr Tyr
                85                  90                  95

Cys Val Arg Asp Arg Val Thr Gly Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Cys Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Met Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
                420                 425                 430

His Tyr Thr Gln Lys Ser Leu Ser Leu Ser Pro Gly Lys
        435                 440                 445

<210>  157
<211>  214
<212>  PRT
<213>  Homo sapiens

<220>
<221>  mat_peptide
<222>  (1)..(214)
<223>  Panitumumab complete Light Chain

<400>  157

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Asp Ala Ser Asn Leu Glu Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Phe Cys Gln His Phe Asp His Leu Pro Leu
                85                  90                  95

Ala Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser

165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  158
<211>  120
<212>  PRT
<213>  Homo sapiens

<220>
<221>  PEPTIDE
<222>  (1)..(120)
<223>  Pertuzumab Heavy Chain variable domain

<400>  158

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

Thr Leu Val Thr Val Ser Ser Ala
        115                 120

<210>  159
<211>  108
<212>  PRT

<213>  Homo sapiens

<220>
<221>  PEPTIDE
<222>  (1)..(108)
<223>  Pertuzumab Light Chain variable domain

<400>  159

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Ile Gly
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ile Tyr Pro Tyr
                85                  90                  95

Thr Phe Gly Gln Gly Thr Lys Val Glu Ile Lys Arg
            100                 105

<210>  160
<211>  446
<212>  PRT
<213>  Homo sapiens

<220>
<221>  mat_peptide
<222>  (1)..(446)
<223>  Ramucirumab complete Heavy Chain

<400>  160

Glu Val Gln Leu Val Gln Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Val Thr Asp Ala Phe Asp Ile Trp Gly Gln Gly Thr Met Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  161
<211>  214
<212>  PRT
<213>  Homo sapiens

<220>
<221>  mat_peptide
<222>  (1)..(214)
<223>  Ramucirumab complete Light Chain

<400>  161

Asp Ile Gln Met Thr Gln Ser Pro Ser Ser Val Ser Ala Ser Ile Gly
1               5                   10                  15

Asp Arg Val Thr Ile Thr Cys Arg Ala Ser Gln Gly Ile Asp Asn Trp
            20                  25                  30

Leu Gly Trp Tyr Gln Gln Lys Pro Gly Lys Ala Pro Lys Leu Leu Ile
        35                  40                  45

Tyr Asp Ala Ser Asn Leu Asp Thr Gly Val Pro Ser Arg Phe Ser Gly

50                  55                  60

Ser Gly Ser Gly Thr Tyr Phe Thr Leu Thr Ile Ser Ser Leu Gln Ala
65                  70                  75                  80

Glu Asp Phe Ala Val Tyr Phe Cys Gln Gln Ala Lys Ala Phe Pro Pro
                85                  90                  95

Thr Phe Gly Gly Gly Thr Lys Val Asp Ile Lys Gly Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  162
<211>  124
<212>  PRT
<213>  Homo sapiens

<220>
<221>  PEPTIDE
<222>  (1)..(124)
<223>  Ranibizumab Heavy Chain variable domain

<400>  162

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Asp Phe Thr His Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro Tyr Tyr Tyr Gly Thr Ser His Trp Tyr Phe Asp Val
            100                 105                 110

Trp Gly Gln Gly Thr Leu Val Thr Val Ser Ser Ala
        115                 120

<210>  163
<211>  108
<212>  PRT
<213>  Homo sapiens

<220>
<221>  PEPTIDE
<222>  (1)..(108)
<223>  Ranibizumab Light Chain variable domain

<400>  163

Asp Ile Gln Leu Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Arg

100                 105

<210>  164
<211>  119
<212>  PRT
<213>  Homo sapiens

<220>
<221>  PEPTIDE
<222>  (1)..(119)
<223>  Ruplizumab Heavy Chain variable domain

<400>  164

Gln Val Gln Leu Val Gln Ser Gly Ala Glu Val Val Lys Pro Gly Ala
1               5                   10                  15

Ser Val Lys Leu Ser Cys Lys Ala Ser Gly Tyr Ile Phe Thr Ser Tyr
            20                  25                  30

Tyr Met Tyr Trp Val Lys Gln Ala Pro Gly Gln Gly Leu Glu Trp Ile
        35                  40                  45

Gly Glu Ile Asn Pro Ser Asn Gly Asp Thr Asn Phe Asn Glu Lys Phe
    50                  55                  60

Lys Ser Lys Ala Thr Leu Thr Val Asp Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Ser Asp Gly Arg Asn Asp Met Asp Ser Trp Gly Gln Gly Thr
            100                 105                 110

Leu Val Thr Val Ser Ser Ala
        115

<210>  165
<211>  112
<212>  PRT
<213>  Homo sapiens

<220>
<221>  PEPTIDE
<222>  (1)..(112)
<223>  Ruplizumab Light Chain variable domain

<400>  165

Asp Ile Val Leu Thr Gln Ser Pro Ala Thr Leu Ser Val Ser Pro Gly
1               5                   10                  15

Glu Arg Ala Thr Ile Ser Cys Arg Ala Ser Gln Arg Val Ser Ser Ser
            20                  25                  30

Thr Tyr Ser Tyr Met His Trp Tyr Gln Gln Lys Pro Gly Gln Pro Pro
        35                  40                  45

Lys Leu Leu Ile Lys Tyr Ala Ser Asn Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Val Glu Pro Glu Asp Phe Ala Thr Tyr Tyr Cys Gln His Ser Trp
                85                  90                  95

Glu Ile Pro Pro Thr Phe Gly Gly Gly Thr Lys Leu Glu Ile Lys Arg
            100                 105                 110

<210>  166
<211>  449
<212>  PRT
<213>  Homo sapiens

<220>
<221>  mat_peptide
<222>  (1)..(449)
<223>  Trastuzumab complete Heavy Chain

<400>  166

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
        100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210>  167
<211>  214
<212>  PRT
<213>  Homo sapiens

<220>
<221>  mat_peptide
<222>  (1)..(214)
<223>  Trastuzumab complete Heavy Chain

<400>  167

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro

85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  168
<211>  449
<212>  PRT
<213>  Homo sapiens

<220>
<221>  mat_peptide
<222>  (1)..(449)
<223>  Trastuzumab emtansine complete Heavy Chain

<400>  168

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210>  169
<211>  214
<212>  PRT
<213>  Homo sapiens

<220>
<221>  mat_peptide
<222>  (1)..(214)
<223>  Trastuzumab emtansine complete Light Chain

<400>  169

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  170
<211>  214
<212>  PRT
<213>  Homo sapiens

<220>
<221>  mat_peptide
<222>  (1)..(214)
<223>  Ustekinumab complete Light Chain

<400>  170

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ile Tyr Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 171
<211> 449
<212> PRT
<213> Homo sapiens

<220>
<221> mat_peptide
<222> (1)..(449)
<223> Ustekinumab complete Heavy Chain

<400> 171

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Thr Tyr
            20                  25                  30

Trp Leu Gly Trp Val Arg Gln Met Pro Gly Lys Gly Leu Asp Trp Ile
        35                  40                  45

Gly Ile Met Ser Pro Val Asp Ser Asp Ile Arg Tyr Ser Pro Ser Phe
    50                  55                  60

Gln Gly Gln Val Thr Met Ser Val Asp Lys Ser Ile Thr Thr Ala Tyr
65                  70                  75                  80

Leu Gln Trp Asn Ser Leu Lys Ala Ser Asp Thr Ala Met Tyr Tyr Cys
                85                  90                  95

Ala Arg Arg Arg Pro Gly Gln Gly Tyr Phe Asp Phe Trp Gly Gln Gly
            100                 105                 110

Thr Leu Val Thr Val Ser Ser Ser Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  172
<211>  214
<212>  PRT
<213>  Homo sapiens

<220>
<221>  mat_peptide
<222>  (1)..(214)
<223>  Visilizumab complete Light Chain

<400>  172

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Glu Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ile Tyr Pro Tyr
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  173
<211>  446
<212>  PRT
<213>  Homo sapiens

<220>
<221>  mat_peptide
<222>  (1)..(446)
<223>  Visilizumab complete Heavy Chain

<400>  173

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Tyr
            20                  25                  30

Thr Met His Trp Val Arg Gln Ala Pro Gly Gln Gly Leu Glu Trp Met
        35                  40                  45

Gly Tyr Ile Asn Pro Arg Ser Gly Tyr Thr His Tyr Asn Gln Lys Leu
    50                  55                  60

Lys Asp Lys Ala Thr Leu Thr Ala Asp Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Ala Tyr Tyr Asp Tyr Asp Gly Phe Ala Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Asn Phe Gly Thr Gln Thr Tyr Thr Cys Asn Val Asp His Lys
        195                 200                 205

Pro Ser Asn Thr Lys Val Asp Lys Thr Val Glu Arg Lys Cys Cys Val
    210                 215                 220

Glu Cys Pro Pro Cys Pro Ala Pro Pro Ala Ala Ala Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Phe Arg Val Val Ser Val
    290                 295                 300

Leu Thr Val Val His Gln Asp Trp Leu Asn Gly Lys Glu Tyr Lys Cys
305                 310                 315                 320

Lys Val Ser Asn Lys Gly Leu Pro Ala Pro Ile Glu Lys Thr Ile Ser
                325                 330                 335

Lys Thr Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Met Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Ser Lys
        435                 440                 445

<210>  174
<211>  107
<212>  PRT
<213>  Homo sapiens

<220>
<221>  PEPTIDE
<222>  (1)..(107)
<223>  Zalutumumab Light Chain variable domain

<400>  174

<210>  175
<211>  125
<212>  PRT
<213>  Homo sapiens

<220>
<221>  PEPTIDE
<222>  (1)..(125)
<223>  Zalutumumab Heavy Chain variable domain

<400>  175

<210>  176
<211>  107
<212>  PRT
<213>  Homo sapiens

<220>
<221>  PEPTIDE
<222>  (1)..(107)
<223>  Zanolimumab Light Chain variable domain

<400>  176

<210>  177
<211>  112
<212>  PRT
<213>  Homo sapiens

<220>
<221>  PEPTIDE
<222>  (1)..(112)
<223>  Zanolimumab Heavy Chain variable domain

<400>  177

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Val Ile Asn Trp Phe Asp Pro Trp Gly Gln Gly Thr Leu Val Thr
            100                 105                 110